# EUROPEAN PATENT SPECIFICATION

(11) **EP 3 020 412 B1**
(45) Date of publication and mention of the grant of the patent: **11.10.2017**
(21) Application number: 15201254.8
(22) Date of filing: 16.06.2010
(51) Int. Cl.: A61K 39/155

(54) **AN IMMUNOGENIC COMPOSITION COMPRISING NANOEMULSION INACTIVATED RSV**
NANOEMULSIONSIMPFSTOFFE
VACCINS EN NANOÉMULSION

(30) Priority: 16.06.2009 US 187529 P
(43) Date of publication of application: 18.05.2016
(62) Divisional of application: 10790134.0
(73) Proprietor: The Regents of the University of Michigan, Ann Arbor, MI 48109-1280 (US)
(72) Inventor: LUKACS, Nicholas W., Brighton, Michigan 48116 (US); LINDELL, Dennis M., Ypsilanti, Michigan 48197 (US); BAKER, James R., Jr., Ann Arbor, Michigan 48103 (US)
(74) Representative: Glawe, Delfs, Moll

(56) References cited:
- WO-A2-2004/030608
- WO-A2-2009/143524
- MEYER ET AL: "Human and bovine respiratory syncytial virus vaccine research and development", COMPARATIVE IMMUNOLOGY, MICROBIOLOGY AND INFECTIOUS DISEASES, PERGAMON PRESS, OXFORD, GB, vol. 31, no. 2-3, 1 March 2008 (2008-03-01), pages 191-225, XP022510802, ISSN: 0147-9571, DOI: 10.1016/J.CIMID.2007.07.008
- YECHIEL BECKER: "Respiratory syncytial virus (RSV) evades the human adaptive immune system by skewing the Th1/Th2 cytokine balance toward increased levels of Th2 cytokines and IgE, markers of allergy-a review", VIRUS GENES, KLUWER ACADEMIC PUBLISHERS, BO, vol. 33, no. 2, 1 October 2006 (2006-10-01), pages 235-252, XP019410081, ISSN: 1572-994X
- PAUL E MAKIDON ET AL: "Pre-Clinical Evaluation of a Novel Nanoemulsion-Based Hepatitis B Mucosal Vaccine", PLOS ONE, PUBLIC LIBRARY OF SCIENCE, US , vol. 3, no. 8 1 August 2008 (2008-08-01), pages E2954.1-E2954.15, XP002676516, ISSN: 1932-6203, DOI: 10.1371/JOURNAL.PONE.0002954 Retrieved from the Internet: URL:http://www.plosone.org/article/info%3A doi%2F10.1371%2Fjournal.pone.0002954 [retrieved on 2008-08-13]
- ANNA U BIELINSKA ET AL: "A Novel, Killed-Virus Nasal Vaccinia Virus Vaccine", CLINICAL AND VACCINE IMMUNOLOGY, AMERICAN SOCIETY FOR MICROBIOLOGY, WASHINGTON, DC, US, vol. 15, no. 2, 1 February 2008 (2008-02-01), pages 348-358, XP008138878, ISSN: 1556-6811, DOI: 10.1128/CVI.00440-07 [retrieved on 2007-12-05]
- DENNIS M. LINDELL ET AL: "A Novel Inactivated Intranasal Respiratory Syncytial Virus Vaccine Promotes Viral Clearance without Th2 Associated Vaccine-Enhanced Disease", PLOS ONE, vol. 6, no. 7, 15 July 2011 (2011-07-15), page e21823, XP055038673, DOI: 10.1371/journal.pone.0021823

## Description

### FIELD OF THE INVENTION

The present invention is defined in the claims and relates to methods and compositions for the stimulation of immune responses. Specifically, the present invention provides immunogenic compositions for use as defined in claims 1-11, a vaccine comprising the immunogenic composition as defined in claim 1 and its use as per claims 12-15. In other words, the invention as defined in the claims relates to methods of using the claimed immunogenic compositions to induce immune responses (e.g., immunity (e.g., protective immunity)) against a pathogenic virus of the *paramyxoviridae* family a *Pneumovirinae* virus (,namely respiratory syncytial virus)).

### BACKGROUND

Immunization is a principal feature for improving the health of people. Despite the availability of a variety of successful vaccines against many common illnesses, infectious diseases remain a leading cause of health problems and death. Significant problems inherent in existing vaccines include the need for repeated immunizations, and the ineffectiveness of the current vaccine delivery systems for a broad spectrum of diseases.

In order to develop vaccines against pathogens that have been recalcitrant to vaccine development, and/or to overcome the failings of commercially available vaccines (e.g., due to adverse results, expense, complexity, and/or underutilization), new methods of antigen presentation must be developed which allow for fewer immunizations, more efficient usage, and/or fewer side effects to the vaccine.

Compositions comprising inactivated viruses are known in the prior art. In particular Meyer et al., 2008 [MEYER ET AL: "Human and bovine respiratory syncytial virus vaccine research and development" COMPARATIVE IMMUNOLOGY, MICROBIOLOGY AND INFRCTIOUS DISEASES, PERGAMON PRESS, OXFORD, GB, vol. 31, no. 2-3, 1 March 2008, pages 191-225]] and Becker, 2006 [YECHIEL BECKER: "Respiratory syncytial virus (RSV) evades the human adaptive immune system by skewing the Th1/Th2 cytokine balance toward increased levels of Th2 cytokines and IgE, markers of allergy - a review", VIRUS GENES, KLUWER ACADEMIC PUBLISHERS, BO, vol. 33, no. 2, 1 October 2006, pages 235-252] disclose formaldehyde inactivated RSV (FI-RSV) vaccine. Aministration of compositions containing viruses inactivated with a nanoemulsion are disclosed in Makidon et al., 2008 [PAUL E MAKIDON ET AL: "Pre-Clinical Evaluation of a Novel Nanoemulsion-Based Hepatitis B Mucosal Vaccine", PLOS ONE, PUBLIC LIBRARY OF SCIENCE, US, vol. 3, no. 8, 1 August 2008, pages E2954.1-E2954.15], WO-A-2004/030608 and Bielinska et al., 2008 [ANNA U BIELINSKA EZ AL: "A Novel, Killed-Virus Nasal Vaccinia Virus Vaccine" CLINICAL AND VACCINE IMMUNOLOGY, AMERICAN SOCIETY FOR MICROBIOLOGY, WASHINGTON, DC, US, vol. 15, no. 2, 1 February 2008, pages 348-358]. As a side note, WO-A-2009/143524 and Dennis et al., 2007 [DENNIS M. LINDELL ET AL: " A Novel Inactivated Intranasal Respiratory Syncytial Virus Vaccine Promotes Viral Clearance without Th2 Associated Vaccine-Enhanced Disease", PLOS ONE, vol. 6, no. 7, 15 July 2011, page e21823], which might be seen as some background disclosure with regard to theory or principle underlying the invention, have been published after the filing date of the present application and therefore are not citable prior art under novelty and inventive step of the present invention.

The fact that the claimed nano-emulsion composition, as disclosed in the present application, is sufficient to inactivate the RSV, is unexpected. Starting from Meyer et al., 2008 Becker, 2006 as the closest prior art, the skilled person faced with the objective technical problem "provision of an improved immunogenic RSV composition suitable for use a a safe vaccine" would not anticipate that the inactivation of RSV would be achieved with the nano-emulsion itself, but would rather inactivate the virus beforehand.

### SUMMARY OF THE INVENTION

The present invention is defined in the claims. In detail: The present invention provides an immunogenic composition comprising: a) a nanoemulsion comprising a solvent (e.g. ethanol), an oil phase, and aqueous phase; in combination with b) a respiratory syncytial virus (RSV) immunogen; for use in stimulating an immune response to RSV in a subject. Said composition for use may optionally comprise a cationic halogen containing compound, wherein said cationic halogen containing compound may be selected from the group consisting of cetylpyridinium halides, cetyltrimethylammonium halides, cetyldimethylethylammonium halides, cetyldimethylbenzylammonium halides, cetyltributylphosphonium halides, dodecyltrimethylammonium halides, and tetradecyltrimethylammonium halides. The RSV immunogen in said immunogenic composition for use as mentioned above may be selected from the group consisting of one or more purified, isolated or synthetic RSV proteins or derivatives, variants, or analogues thereof; or, inactivated RSV from one or more serotypes of RSV. Said one or more serotypes of RSV may be selected from the group consisting of RSV strain A2, RSV strain B, RSV strain 9320, RSV strain 18537, RSV strain Long, and RSV strain Line 19. Said composition for use as defined in claim 1 may further comprises a surfactant, wherein said surfactant may be selected from the group consisting of polyoxyethylene(20)sorbitan monolaurate, polyoxyethylene(20)sorbitan monopalmitate, polyoxyethylene(20)sorbitan monostearate and polyoxyethylene(20)sorbitan monooleate. The nanoemulsion of the immunogenic composition for use as defined in claim 1 may comprises about 5 vol. % TWEEN 80 or TWEEN 20, about 8 vol. % ethanol, about 1 vol. % cetylpyridinium chloride (CPC), about 64 vol. % oil, preferably soybean oil, and about 22 vol. % water. The oil in the nanoemulsion of the immunogenic composition for use according to claim 1 may be an animal oil, a vegetable oil (e.g. soybean oil), a natural oil, a synthetic oil, a hydrocarbon oil, a silicone oil, or any combination thereof. The invention as defined in the claims further comprises a vaccine comprising the immunogenic composition as defined in claim 1, which may be formulated for administration to a mucosalsurface, the use of the immunogenic composition as defined in claim 1 in the manufacture of a vaccine for treatment or prevention of respiratory syncytial virus infection and an immunogenic composition as defined in claim 1 for use in the treatment or prevention of respiratory syncytial virus infection.

For the sake of completeness, some further examples are provided below.In some cases, the nanoemulsion is W₈₀5ECThe composition may comprise between 1-50% nanoemulsion solution, although greater and lesser amounts also find use. The immunogenic composition may comprise about 1.0% - 10%, about 10%-20%, about 20% - 30%, about 30%-40%, about 40%- 50%, about 50%-60% or more nanoemulsion solution. The immunogenic composition may comprises about 10% nanoemulsion solution. The immunogenic composition may comprise about 15% nanoemulsion solution. The immunogenic compositionmay comprise about 20% nanoemulsion solution. The immunogenic composition may comprise about 12% nanoemulsion solution. The immunogenic composition may comprise about 8% nanoemulsion solution. The immunogenic composition may comprise about 5% nanoemulsion solution. The immunogenic composition may comprise about 2% nanoemulsion solution. The immunogenic composition may comprise about 1% nanoemulsion solution. An immunogenic composition as defined in the claims may comprises 2x10⁶ plaque forming units (PFU) of inactivated pathogenic virus of t RSV, although greater, e.g., about 4x10⁶ PFU, 8x10⁶ PFU, 1x10⁷ PFU, 2x10⁷ PFU, 4x10⁷ PFU, 8x10⁷ PFU, 1x10⁸ PFU, 1x10⁹ PFU, or more PFU of RSV inactivated by nanoemulsion, and lesser, e.g., about 1x10⁶ PFU, 5x10⁵ PFU, 1x10⁵ PFU, 5x10⁴ PFU, 1x10⁴ PFU, 5x10³ PFU, 1x10³ PFU or fewer PFU of virus of the RSV inactivated by nanoemulsion amounts may also be utilized. The composition my be stable (e.g., at room temperature (e.g., for 12 hours, one day, two days, three days, four days, a week, two weeks, three weeks, a month, two months, three months, four months, five months, six months, 9 months, a year or more). The immunogenic composition may comprise a pharmaceutically acceptable carrier. Any suitable carrier may be utilized including but not limited to those described herein. The immunogenic composition may further comprise an adjuvant. Any one or more adjuvants described herein may find use in a composition of the invention as defined in the claims including but not limited to adjuvants that skew toward a Th1 immune response. The mucosal surface to be targeted by the vaccine as defined in claim 13 may comprise nasal mucosa. The immunogenic composition is for use in stimualting an immune response to RSV in a subject as defined in claim 1.

A variety of nanoemulsion compositions as defined in the claims are described herein. A variety of oils being part of the nanoemulsion as defined in the claims are contemplated, including, but not limited to, soybean, avocado, squalene, olive, canola, corn, rapeseed, safflower, sunflower, fish, flavor, and water insoluble vitamins. A variety of solvents may find use in the nanoemulsion of the immunogenic composition as defined in the claims, including, but not limited to, an alcohol (e.g., including, but not limited to, methanol, ethanol as defined in claim 2, propanol, and octanol), glycerol, polyethylene glycol, and an organic phosphate based solvent. Nanoemulsion components including oils, solvents and others are described in further detail below.

The emulsion may further optionally comprise a cationic halogen containing compound as defined in claim 3. A variety of cationic halogen containing compounds are contemplated including, but not limited to, cetylpyridinium halides, cetyltrimethylammonium halides, cetyldimethylethylammonium halides, cetyldimethylbenzylammonium halides, cetyltributylphosphonium halides, dodecyltrimethylammonium halides, and tetradecyltrimethylammonium halides as defined in claim 4. A variety of halides are contemplated including, but not limited to, halide selected from the group consisting of chloride, fluoride, bromide, and iodide.

### DESCRIPTION OF THE FIGURES

The following figures form part of the present specification. The invention may be better understood by reference to one or more of these figures in combination with the description of specific examples presented herein.
Figure 1 shows the killing of respiratory syncytial virus (RSV) by nanoemulsion.
Figure 2 shows induction of RSV-specific antibodies following immunization with nanoemulsion inactivated RSV (NE-RSV).
Figure 3 shows that administration of NE-RSV to subjects results in enhanced RSV-specific CD8 T cell responses.
Figure 4 shows administration of NE-RSV to subjects enhances antiviral cytokines in the BAL fluid from airways of RSV challenged mice.
Figure 5 shows that vaccination of mice with NE-RSV enhances IL-17 production in the lungs following challenge with live RSV.
Figure 6 shows that administration of NE-RSV to subjects provides improved clearance and induces a protective response upon subsequent live viral challenge.
Figure 7 shows expression of various genes in mice administered NE-RSV versus controls.
Figure 8 shows periodic acid schiff's (PAS) staining of lung histologic sections in mice administered NE-RSV versus controls
Figure 9 shows cytokine expression in in mice administered NE-RSV versus controls.
Figure 10 shows significant RSV-specific antibody responses were generated systemically in mice following vaccination with NE-RSV (A) and total Ig in the bronchoalveolar lavage fluid of vaccinated mice at day 2 post-challenge with live virus.

### GENERAL DESCRIPTION OF THE INVENTION

The invention is set out in the appended claims and the above section "SUMMARY OF THE INVENTION".The present disclosure provides compositions for the stimulation of immune responses. Specifically, the present disclosure provides immunogenic compositions to induce immune responses (e.g., immunity (e.g., protective immunity)) against respiratory syncytial virus. Compositions of the present disclosure find use in, among other things, clinical (e.g. therapeutic and preventative medicine (e.g., vaccination)) and research applications.

NE treatment probably preserves important antigenic epitopes (e.g., recognizable by a subject's immune system) of the virus (e.g., while concurrently neutralizing and/or eradicating the infectivity potential of the virus), stabilizing their hydrophobic and hydrophilic components in the oil and water interface of the emulsion (e.g., thereby providing one or more immunogens (e.g., stabilized antigens) against which a subject can mount an immune response). It seems that because NE formulations penetrate the mucosa through pores, they may carry immunogens to the submucosal location of dendritic cells (e.g., thereby initiating and/or stimulating an immune response). Combining a NE and RSV appears to stabilize viral immunogens and provides a proper immunogenic material for generation of an immune response.

Dendritic cells avidly phagocytose nanoemulsion (NE) oil droplets and this could provide a means to internalize viral immunogens (e.g., antigenic proteins or peptide fragments thereof generated post inactivation of the RSVwith a nanoemulsion for antigen presentation. While other vaccines rely on inflammatory toxins or other immune stimuli for adjuvant activity (See, e.g., Holmgren and Czerkinsky, Nature Med. 2005, 11; 45-53), NEs have not been shown to be inflammatory when placed on the skin or mucous membranes in studies on animals and in humans. Thus, a composition comprising a NE of the present invention may act as a "physical" adjuvant (e.g., that transports and/or presents immunogenic compositions (e.g., peptides and/or antigens of a *paramyxoviridae* family virus) to the immune system. Mucosal administration of a a vaccine as defined in claim 13 may generate mucosal (e.g., signs of mucosal immunity (e.g., generation of IgA antibody titers)) as well as systemic immunity.

Both cellular and humoral immunity play a role in protection against multiple pathogens. A composition of the present invention as defined in the claims (NE-inactivated RSV) is used as a vaccine as defined in claim 12.

A composition of the present invention as defined in the claims may induce (e.g., when administered to a subject) both systemic and mucosal immunity. Thus, administration of a composition of the present invention as defined in the claims to a subject may result in protection against an exposure (e.g., a lethal mucosal exposure) to RSV. Mucosal administration (e.g., vaccination) may provide protection against viral infection (e.g., that initiates at a mucosal surface). Although it has heretofore proven difficult to stimulate secretory IgA responses and protection against pathogens that invade at mucosal surfaces (See, e.g., Mestecky et al, Mucosal Immunology. 3ed edn. (Academic Press, San Diego, 2005)), the present invention provides compositions for stimulating mucosal immunity (e.g., a protective IgA response) against RSV.

### DEFINITIONS

To facilitate an understanding of the present invention, a number of terms and phrases are defined below:
As used herein, the term "microorganism" refers to any species or type of microorganism, including but not limited to, bacteria, viruses, archaea, fungi, protozoans, mycoplasma, prions, and parasitic organisms. The term microorganism encompasses both those organisms that are in and of themselves pathogenic to another organism (e.g., animals, including humans, and plants) and those organisms that produce agents that are pathogenic to another organism, while the organism itself is not directly pathogenic or infective to the other organism.

As used herein the term "pathogen," and grammatical equivalents, refers to an organism (e.g., biological agent), including microorganisms, that causes a disease state (e.g., infection, pathologic condition, disease, etc.) in another organism (e.g., animals and plants) by directly infecting the other organism, or by producing agents that causes disease in another organism (e.g., bacteria that produce pathogenic toxins and the like). "Pathogens" include, but are not limited to, viruses, bacteria, archaea, fungi, protozoans, mycoplasma, prions, and parasitic organisms.

The terms "bacteria" and "bacterium" refer to all prokaryotic organisms, including those within all of the phyla in the Kingdom Procaryotae. It is intended that the term encompass all microorganisms considered to be bacteria including *Mycoplasma, Chlamydia, Actinomyces, Streptomyces,* and *Rickettsia.* All forms of bacteria are included within this definition including cocci, bacilli, spirochetes, spheroplasts, protoplasts, etc.

As used herein, the term "fungi" is used in reference to eukaryotic organisms such as molds and yeasts, including dimorphic fungi.

As used herein the terms "disease" and "pathologic condition" are used interchangeably, unless indicated otherwise herein, to describe a deviation from the condition regarded as normal or average for members of a species or group (e.g., humans), and which is detrimental to an affected individual under conditions that are not inimical to the majority of individuals of that species or group. Such a deviation can manifest as a state, signs, and/or symptoms (e.g., diarrhea, nausea, fever, pain, blisters, boils, rash, immune suppression, inflammation, etc.) that are associated with any impairment of the normal state of a subject or of any of its organs or tissues that interrupts or modifies the performance of normal functions. A disease or pathological condition may be caused by or result from contact with a microorganism (e.g., a pathogen or other infective agent (e.g., a virus or bacteria)), may be responsive to environmental factors (e.g., malnutrition, industrial hazards, and/or climate), may be responsive to an inherent defect of the organism (e.g., genetic anomalies) or to combinations of these and other factors.

The terms "host" or "subject," as used herein, refer to an individual to be treated by (e.g., administered) the compositions as defined in the claims. Subjects include, but are not limited to, mammals (*e.g.*, murines, simians, equines, bovines, porcines, canines, felines, and the like), and most preferably includes humans. In the context of the invention, the term "subject" generally refers to an individual who will be administered or who has been administered one or more compositions of the present invention (e.g., a composition for inducing an immune response).

As used herein, the terms "inactivating," "inactivation" and grammatical equivalents, when used in reference to a microorganism (e.g., a pathogen (e.g., a virus)), refer to the killing, elimination, neutralization and/or reducing of the capacity of the mircroorganism (e.g., a pathogen (e.g., a virus)) to infect and/or cause a pathological response and/or disease in a host.

As used herein, the term "fusigenic" is intended to refer to an emulsion that is capable of fusing with the membrane of a microbial agent (e.g., a bacterium, bacterial spore or viral capsid). Specific examples of fusigenic emulsions are described herein.

As used herein, the term "lysogenic" refers to an emulsion (e.g., a nanoemulsion) that is capable of disrupting the membrane of a microbial agent (e.g., a virus (e.g., viral envelope) or a bacterium or bacterial spore). The presence of a lysogenic and a fusigenic agent in the same composition may produce an enhanced inactivating effect compared to either agent alone. Compositions (e.g., for inducing an immune response (e.g., used as a vaccine) using this improved antimicrobial composition are described in detail herein.

The term "emulsion," as used herein, includes classic oil-in-water or water in oil dispersions or droplets, as well as other lipid structures that can form as a result of hydrophobic forces that drive apolar residues (e.g., long hydrocarbon chains) away from water and drive polar head groups toward water, when a water immiscible oily phase is mixed with an aqueous phase. These other lipid structures include, but are not limited to, unilamellar, paucilamellar, and multilamellar lipid vesicles, micelles, and lamellar phases. Similarly, the term "nanoemulsion," as used herein, refers to oil-in-water dispersions comprising small lipid structures. For example, the nanoemulsions may comprise an oil phase having droplets with a mean particle size of approximately 0.1 to 5 microns (e.g., 150 +/-25 nm in diameter), although smaller and larger particle sizes are contemplated. The terms "emulsion" and "nanoemulsion" are often used herein, interchangeably, to refer to the nanoemulsions of the present invention.

As used herein, the terms "contact," "contacted," "expose," and "exposed," when used in reference to a nanoemulsion and a live microorganism, refer to bringing one or more nanoemulsions into contact with a microorganism (e.g., a pathogen) such that the nanoemulsion inactivates the microorganism or pathogenic agent, if present.. A variety of nanoemulsion are described herein and elsewhere (e.g., nanoemulsions described in U.S. Pat. Apps. 20020045667 and 20040043041, and U.S. Pat. Nos. 6,015,832, 6,506,803, 6,635,676, and 6,559,189). Ratios and amounts of nanoemulsion (e.g., sufficient for inactivating the microorganism (e.g., virus inactivation)) and microorganisms (e.g., sufficient to provide an antigenic composition (e.g., a composition capable of inducing an immune response)) are described herein.

The term "surfactant" refers to any molecule having both a polar head group, which energetically prefers solvation by water, and a hydrophobic tail that is not well solvated by water. The term "cationic surfactant" refers to a surfactant with a cationic head group. The term "anionic surfactant" refers to a surfactant with an anionic head group.

The terms "Hydrophile-Lipophile Balance Index Number" and "HLB Index Number" refer to an index for correlating the chemical structure of surfactant molecules with their surface activity. The HLB Index Number may be calculated by a variety of empirical formulas as described, for example, by Meyers, (See, e.g., Meyers, Surfactant Science and Technology, VCH Publishers Inc., New York, pp. 231-245 (1992)). As used herein where appropriate, the HLB Index Number of a surfactant is the HLB Index Number assigned to that surfactant in McCutcheon's Volume 1: Emulsifiers and Detergents North American Edition, 1996. The HLB Index Number ranges from 0 to about 70 or more for commercial surfactants. Hydrophilic surfactants with high solubility in water and solubilizing properties are at the high end of the scale, while surfactants with low solubility in water that are good solubilizers of water in oils are at the low end of the scale.

As used herein the term "interaction enhancers" refers to compounds that act to enhance the interaction of an emulsion with a microorganism (e.g., with a cell wall of a bacteria (*e.g.,* a Gram negative bacteria) or with a viral envelope. Contemplated interaction enhancers include, but are not limited to, chelating agents (*e.g.*, ethylenediaminetetraacetic acid (EDTA), ethylenebis(oxyethylenenitrilo)tetraacetic acid (EGTA), and the like) and certain biological agents (*e.g.*, bovine serum abulmin (BSA) and the like).

The terms "buffer" or "buffering agents" refer to materials, that when added to a solution, cause the solution to resist changes in pH.

The terms "reducing agent" and "electron donor" refer to a material that donates electrons to a second material to reduce the oxidation state of one or more of the second material's atoms.

The term "monovalent salt" refers to any salt in which the metal (*e.g.,* Na, K, or Li) has a net 1+ charge in solution (i.e., one more proton than electron).

The term "divalent salt" refers to any salt in which a metal (e.g., Mg, Ca, or Sr) has a net 2+ charge in solution.

The terms "chelator" or "chelating agent" refer to any materials having more than one atom with a lone pair of electrons that are available to bond to a metal ion.

The term "solution" refers to an aqueous or non-aqueous mixture.

As used herein, the terms "a composition for inducing an immune response," "immunogenic composition" or grammatical equivalents refer to a composition that, once administered to a subject (e.g., once, twice, three times or more (e.g., separated by weeks, months or years)), stimulates, generates and/or elicits an immune response in the subject (e.g., resulting in total or partial immunity to a microorganism (e.g., pathogen) capable of causing disease). The composition comprises a nanoemulsion and an immunogen as defined in claim 1, and may furthermore comprise one or more other compounds or agents including, but not limited to, therapeutic agents, physiologically tolerable liquids, gels, carriers, diluents, adjuvants, excipients, salicylates, steroids, immunosuppressants, immunostimulants, antibodies, cytokines, antibiotics, binders, fillers, preservatives, stabilizing agents, emulsifiers, and/or buffers. An immune response may be an innate (e.g., a non-specific) immune response or a learned (e.g., acquired) immune response (e.g. that decreases the infectivity, morbidity, or onset of mortality in a subject (e.g., caused by exposure to a pathogenic microorganism) or that prevents infectivity, morbidity, or onset of mortality in a subject (e.g., caused by exposure to a pathogenic microorganism)). As used herein, the term "adjuvant" refers to any substance that can stimulate an immune response (e.g., a mucosal immune response). Some adjuvants can cause activation of a cell of the immune system (e.g., an adjuvant can cause an immune cell to produce and secrete a cytokine). Examples of adjuvants that can cause activation of a cell of the immune system include, but are not limited to, saponins purified from the bark of the Q. saponaria tree, such as QS21 (a glycolipid that elutes in the 21st peak with HPLC fractionation; Aquila Biopharmaceuticals, Inc., Worcester, Mass.); poly(di(carboxylatophenoxy)phosphazene (PCPP polymer; Virus Research Institute, USA); derivatives of lipopolysaccharides such as monophosphoryl lipid A (MPL; Ribi ImmunoChem Research, Inc., Hamilton, Mont.), muramyl dipeptide (MDP; Ribi) and threonyl-muramyl dipeptide (t-MDP; Ribi); OM-174 (a glucosamine disaccharide related to lipid A; OM Pharma SA, Meyrin, Switzerland); and Leishmania elongation factor (a purified Leishmania protein; Corixa Corporation, Seattle, Wash.). Traditional adjuvants are well known in the art and include, for example, aluminum phosphate or hydroxide salts ("alum").

As used herein, the term "an amount effective to induce an immune response" (e.g., of a composition for inducing an immune response), refers to the dosage level required (e.g., when administered to a subject) to stimulate, generate and/or elicit an immune response in the subject. An effective amount can be administered in one or more administrations (e.g., via the same or different route), applications or dosages and is not intended to be limited to a particular formulation or administration route.

As used herein, the term "under conditions such that said subject generates an immune response" refers to any qualitative or quantitative induction, generation, and/or stimulation of an immune response (e.g., innate or acquired).

A used herein, the term "immune response" refers to a response by the immune system of a subject. For example, immune responses include, but are not limited to, a detectable alteration (e.g., increase) in Toll receptor activation, lymphokine (e.g., cytokine (e.g., Th1 or Th2 type cytokines) or chemokine) expression and/or secretion, macrophage activation, dendritic cell activation, T cell activation (e.g., CD4+ or CD8+ T cells), NK cell activation, and/or B cell activation (e.g., antibody generation and/or secretion). Additional examples of immune responses include binding of an immunogen (e.g., antigen (e.g., immunogenic polypeptide)) to an MHC molecule and inducing a cytotoxic T lymphocyte ("CTL") response, inducing a B cell response (e.g., antibody production), and/or T-helper lymphocyte response, and/or a delayed type hypersensitivity (DTH) response against the antigen from which the immunogenic polypeptide is derived, expansion (e.g., growth of a population of cells) of cells of the immune system (e.g., T cells, B cells (e.g., of any stage of development (e.g., plasma cells), and increased processing and presentation of antigen by antigen presenting cells. An immune response may be to immunogens that the subject's immune system recognizes as foreign (e.g., non-self antigens from microorganisms (e.g., pathogens), or self-antigens recognized as foreign). Thus, it is to be understood that, as used herein, "immune response" refers to any type of immune response, including, but not limited to, innate immune responses (e.g., activation of Toll receptor signaling cascade) cell-mediated immune responses (e.g., responses mediated by T cells (e.g., antigen-specific T cells) and non-specific cells of the immune system) and humoral immune responses (e.g., responses mediated by B cells (e.g., via generation and secretion of antibodies into the plasma, lymph, and/or tissue fluids). The term "immune response" is meant to encompass all aspects of the capability of a subject's immune system to respond to antigens and/or immunogens (e.g., both the initial response to an immunogen (e.g., a pathogen) as well as acquired (e.g., memory) responses that are a result of an adaptive immune response).

As used herein, the term "immunity" refers to protection from disease (e.g., preventing or attenuating (e.g., suppression) of a sign, symptom or condition of the disease) upon exposure to a microorganism (e.g., pathogen) capable of causing the disease. Immunity can be innate (e.g., non-adaptive (e.g., non-acquired) immune responses that exist in the absence of a previous exposure to an antigen) and/or acquired (e.g., immune responses that are mediated by B and T cells following a previous exposure to antigen (e.g., that exhibit increased specificity and reactivity to the antigen)).

As used herein, the term "immunogen" refers to an agent (e.g., a microorganism (e.g., bacterium, virus or fungus) and/or portion or component thereof (e.g., a protein antigen)) that is capable of eliciting an immune response in a subject. Immunogens may elicit immunity against the immunogen (e.g., microorganism (e.g., pathogen or a pathogen product)) when administered in combination with a nanoemulsion.

As used herein, the term "pathogen product" refers to any component or product derived from a pathogen including, but not limited to, polypeptides, peptides, proteins, nucleic acids, membrane fractions, and polysaccharides.

As used herein, the term "enhanced immunity" refers to an increase in the level of adaptive and/or acquired immunity in a subject to a given immunogen (e.g., microorganism (e.g., pathogen)) following administration of a composition relative to the level of adaptive and/or acquired immunity in a subject that has not been administered the composition.

As used herein, the terms "purified" or "to purify" refer to the removal of contaminants or undesired compounds from a sample or composition. As used herein, the term "substantially purified" refers to the removal of from about 70 to 90 %, up to 100%, of the contaminants or undesired compounds from a sample or composition.

As used herein, the terms "administration" and "administering" refer to the act of giving a composition to a subject. Exemplary routes of administration to the human body include, but are not limited to, through the eyes (ophthalmic), mouth (oral), skin (transdermal), nose (nasal), lungs (inhalant), oral mucosa (buccal), ear, rectal, by injection (e.g., intravenously, subcutaneously, intraperitoneally, etc.), topically, and the like.

As used herein, the terms "co-administration" and "co-administering" refer to the administration of at least two agent(s) or therapies to a subject. The co-administration of two or more agents or therapies may be concurrent, or a first agent/therapy is administered prior to a second agent/therapy. The co-administration can be via the same or different route of administration. Those of skill in the art understand that the formulations and/or routes of administration of the various agents or therapies used may vary. The appropriate dosage for co-administration can be readily determined by one skilled in the art. When agents or therapies are co-administered, the respective agents or therapies can be administered at lower dosages than appropriate for their administration alone. Thus, co-administration is especially desirable where the co-administration of the agents or therapies lowers the requisite dosage of a potentially harmful (e.g., toxic) agent(s), and/or when co-administration of two or more agents results in sensitization of a subject to beneficial effects of one of the agents via co-administration of the other agent. The co-administration may be preferable to elicit an immune response in a subject to two or more different immunogens (e.g., microorganisms (e.g., pathogens)) at or near the same time (e.g., when a subject is unlikely to be available for subsequent administration of a second, third, or more composition for inducing an immune response).

As used herein, the term "topically" refers to application of a composition to the surface of the skin and/or mucosal cells and tissues (e.g., alveolar, buccal, lingual, masticatory, vaginal or nasal mucosa, and other tissues and cells which line hollow organs or body cavities).

The compositions for use as defined in the claims are for administration in the form of topical emulsions, injectable compositions, ingestible solutions, and the like. For the sake of completeness it has to be noted that said administration is not covered by the invention as defined in the claims. When the route is topical, the form may be, for example, a spray (e.g., a nasal spray), a cream, or other viscous solution (e.g., a composition comprising a nanoemulsion and an immunogen in polyethylene glycol).

The terms "pharmaceutically acceptable" or "pharmacologically acceptable," as used herein, refer to compositions that do not substantially produce adverse reactions (e.g., toxic, allergic or immunological reactions) when administered to a subject.

As used herein, the term "pharmaceutically acceptable carrier" refers to any of the standard pharmaceutical carriers including, but not limited to, phosphate buffered saline solution, water, and various types of wetting agents (e.g., sodium lauryl sulfate), any and all solvents, dispersion media, coatings, sodium lauryl sulfate, isotonic and absorption delaying agents, disintrigrants (e.g., potato starch or sodium starch glycolate), polyethylethe glycol, and the like. The compositions also can include stabilizers and preservatives. Examples of carriers, stabilizers and adjuvants have been described and are known in the art (See e.g., Martin, Remington's Pharmaceutical Sciences, 15th Ed., Mack Publ. Co., Easton, Pa. (1975)).

As used herein, the term "pharmaceutically acceptable salt" refers to any salt (e.g., obtained by reaction with an acid or a base) of a composition that is physiologically tolerated in the target subject. "Salts" of the compositions may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, sulfonic, naphthalene-2-sulfonic, benzenesulfonic acid, and the like. Other acids, such as oxalic, while not in themselves pharmaceutically acceptable, may be employed in the preparation of salts useful as intermediates in obtaining the compositions and their pharmaceutically acceptable acid addition salts.

Examples of bases include, but are not limited to, alkali metal (e.g., sodium) hydroxides, alkaline earth metal (e.g., magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds compounded with a suitable cation such as Na⁺, NH₄⁺, and NW₄⁺ (wherein W is a C₁₋₄ alkyl group), and the like. For therapeutic use, salts of the compounds are contemplated as being pharmaceutically acceptable. However, salts of acids and bases that are non-pharmaceutically acceptable may also find use, for example, in the preparation or purification of a pharmaceutically acceptable compound.

For therapeutic use, salts of the compositions are contemplated as being pharmaceutically acceptable. However, salts of acids and bases that are non-pharmaceutically acceptable may also find use, for example, in the preparation or purification of a pharmaceutically acceptable composition.

As used herein, the term "at risk for disease" refers to a subject that is predisposed to experiencing a particular disease. This predisposition may be genetic (e.g., a particular genetic tendency to experience the disease, such as heritable disorders), or due to other factors (e.g., age, environmental conditions, exposures to detrimental compounds present in the environment, etc.)..

"Nasal application", as used herein, means applied through the nose into the nasal or sinus passages or both. The application may, for example, be done by drops, sprays, mists, coatings or mixtures thereof applied to the nasal and sinus passages.

As used herein, the term "kit" refers to any delivery system for delivering materials. In the context of immunogenic agents (e.g., compositions comprising a nanoemulsion and an immunogen), such delivery systems include systems that allow for the storage, transport, or delivery of immunogenic agents and/or supporting materials (e.g., written instructions for using the materials, etc.) from one location to another. For example, kits include one or more enclosures (e.g., boxes) containing the relevant immunogenic agents (e.g., nanoemulsions) and/or supporting materials. As used herein, the term "fragmented kit" refers to delivery systems comprising two or more separate containers that each contain a subportion of the total kit components. The containers may be delivered to the intended recipient together or separately. For example, a first container may contain a composition comprising a nanoemulsion and an immunogen for a particular use, while a second container contains a second agent (e.g., an antibiotic or spray applicator). Indeed, any delivery system comprising two or more separate containers that each contains a subportion of the total kit components are included in the term "fragmented kit." In contrast, a "combined kit" refers to a delivery system containing all of the components of an immunogenic agent needed for a particular use in a single container (e.g., in a single box housing each of the desired components). The term "kit" includes both fragmented and combined kits.

### DETAILED DESCRIPTION OF THE INVENTION

Respiratory syncytial virus (RSV) infects nearly all infants by age 2 and is the leading cause of bronchiolitis in children worldwide. It is estimated by the CDC that up to 125,000 pediatric hospitalizations in the United States each year are due to RSV, at an annual cost of over $300,000,000 (1). Despite the generation of RSV-specific adaptive immune responses, RSV does not confer protective immunity and recurrent infections throughout life are common (2, 3). While RSV is especially detrimental in very young infants whose airways are small and easily occluded, RSV is also widely becoming recognized as an important pathogen in transplant recipients, patients with chronic obstructive pulmonary disease (COPD), the elderly, as well as other patients with chronic lung disease, especially asthma. Recent data suggest that mortality for all ages combined has been approximately 30/100,000 from 1990-2000, with an annual average mortality of over 17,000 in the US (4, 5). These numbers are likely grossly underestimated, as it has not been thoroughly examined in adults in a consistent manner. Thus, RSV not only causes significant exacerbated lung disease in young and old, but also is associated with a significant amount of mortality directly. Although anti-RSV antibodies are available and appear to alleviate severe disease, they perform only when given propholactically and few other options exist for combating the RSV infections in susceptible patient populations (6-10).

In the late 1960s, attempts to vaccinate children with an alum-precipitated formalin-inactivated RSV vaccine preparation failed and caused severe exacerbated disease upon re-infection with live RSV. The clinical manifestations appeared to be a result of an enhanced Th2 disease, mucus production and eosinophilia that was not observed in non-vaccinated children. These same symptoms can occur in subsets of severely infected infants.

Several epidemiologic studies link severe RSV responses with the later development of hyperreactive airway disease even years after the infection has resolved (3). Sigurs et. al. found infants hospitalized with RSV bronchiolitis were at higher risk of developing asthma and episodes of wheezing at ages 1, 3 and 7 compared to healthy controls (11-13). RSV has also been associated with asthma exacerbations and can cause prolonged episodes of illness (14). One interesting study suggested a causal link since treating infants who had severe RSV disease with RSV immune globulin significantly decreased their later risk in developing childhood asthma and pulmonary dysfunction (15). One clinically relevant feature of RSV disease that may predispose children and adults to chronic disease is the inability to acquire protective immunity due to an altered immune response. More recent studies have suggested that patient populations with compromised lung function (especially COPD patients) are also at risk of severe complications due to RSV infection that is nearly as prevalent as those associated with influenza infections (16-20). These complications may be compounded by the potential of RSV having the ability to persist for long periods in the lung even after acute disease has resolved. This has been hypothesized to be associated with the development of an altered immune environment that less efficiently clears the virus. Thus, an effective vaccine that can be used in children and adults has the potential for broad application across the population and may provide significant protection from initiation and exacerbation of chronic lung disease.

Accordingly, the present invention provides immunogenic nanoemulision compositions for use as defined in the claims and to induce immune responses (e.g., immunity (e.g., protective immunity)) against RSV. Compositions of the present invention as defined in the claims find use in, among other things, clinical (e.g. therapeutic and preventative medicine (e.g., vaccination)) and research applications. Exemplary immunogenic compositions (e.g., vaccine compositions) are described in more detail below.

The present invention provides compositions for inducing immune responses comprising a nanoemulsion and respiratory syncytial virus immunogen for use as defined in claim 1. Claim 9 specifies a particular nanoemulsion. Indeed, a variety of nanoemulsions may find use, e.g. those described herein and those described elsewhere (e.g., nanoemulsions described in U.S. Pat. Apps. 20020045667 and 20040043041, and U.S. Pat. Nos. 6,015,832, 6,506,803, 6,635,676, and 6,559,189).

Immunogens, inactivated respiratory syncytial virus, and nanoemulsions as defined in the claims may be combined in any suitable amount. Any suitable pharmaceutical formulation may be utilized, including, but not limited to, those disclosed herein. Suitable formulations may be tested for immunogenicity using any suitable method. For example, immunogenicity may be investigated by quantitating both antibody titer and specific T-cell responses. Nanoemulsion compositions may also be tested in animal models of infectious disease states. For the sake of completeness it as to be noted that this aspect is not covered by the present invention.Suitable animal models, pathogens, and assays for immunogenicity include, but are not limited to, those described below.

Mucosal administration of a composition comprising NE and RSV (e.g., NE-killed RSV) to a subject resulted in high-titer antibody responses and specific Th1 cellular immunity (See, e.g., Examples 1-4). Further, animals were protected against subsequent challenge with RSV (See, e.g., Example 4). Moreover, in sharp contrast to an alum-precipitated formalin-inactivated RSV vaccine preparation (e.g., described above), the NE inactivated RSV led to a robust Th1 immune (e.g., as documented by enhanced expression of IFN-γ and IL-17 (See, e.g., Example 4) response and did not enhance and/or elevate expression of Th2 cytokines (e.g., IL-4, IL-5 or IL-13) associated with a Th2 type response. Mice administered even a single dose of a composition comprising NE-killed RSV developed serum concentrations of anti-RSV IgG 4 weeks after administration that continued to increase at 8 weeks post administration and that was significantly elevated after a booster administration (See, e.g., Example 2-4). Thus, it has been shown that a single administration (e.g., mucosal administration) of a composition comprising NE-killed RSV is sufficient to induce a protective immune response in a subject (e.g., protective immunity (e.g., mucosal and systemic immunity)). Subsequent administration (e.g., one or more boost administrations subsequent to a primary administration) to a subject may provide the induction of an enhanced immune response to RSV in the subject. Thus, it has been demonstrated that administration of a composition comprising NE-killed RSV to a subject provides protective immunity against RSV infection.

Both cellular and humoral immunity are likely to play a role in protection against RSV, and both were induced with the NE formulations (See, e.g., Examples 1-4). RSV-specific antibody titers are considered important for the estimate of protective immunity in human subjects and in animal models of vaccination.

Data document that NE efficiently kills RSV and generates a non-infectious immunization composition suitable for use in inducing an immune response against RSV in a subject (e.g., for use as a vaccine). The immunogenic compositions induce specific anti-RSV serum antibody titers and initiate important anti-viral cellular immune responses (e.g., including increased anti-virus cytokine production and development of RSV-specific CD8+ cytotoxic T cells) post administration to a subject. The immunogenic composition of the invention also provides improved viral clearance upon live RSV challenge. Accordingly, compositions of the present invention as defined in the claims may provide the ability to generate appropriate innate immune responses (e.g., resulting from exposure to antigens maintained in a recognizable form in NE that simulate antigens provided by an active infection) thereby providing a more appropriate vaccine strategy (e.g., compared to formalin killed RSV).

Thus, administration (e.g., mucosal administration) of a composition of the present invention as defined in the claims to a subject may provide the induction of both humoral (e.g., development of specific antibodies) and cellular (e.g., cytotoxic T lymphocyte) immune responses against RSV. A composition for use as defined in claims 1-9 may be used as a vaccine according to claim 12.

### Nanoemulsions

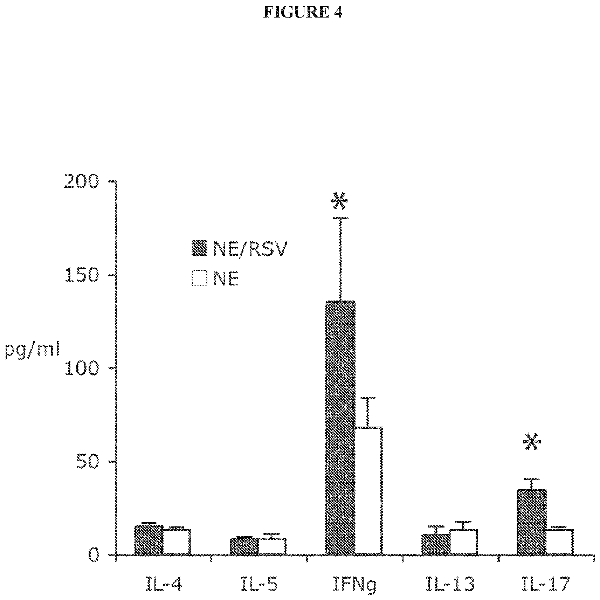

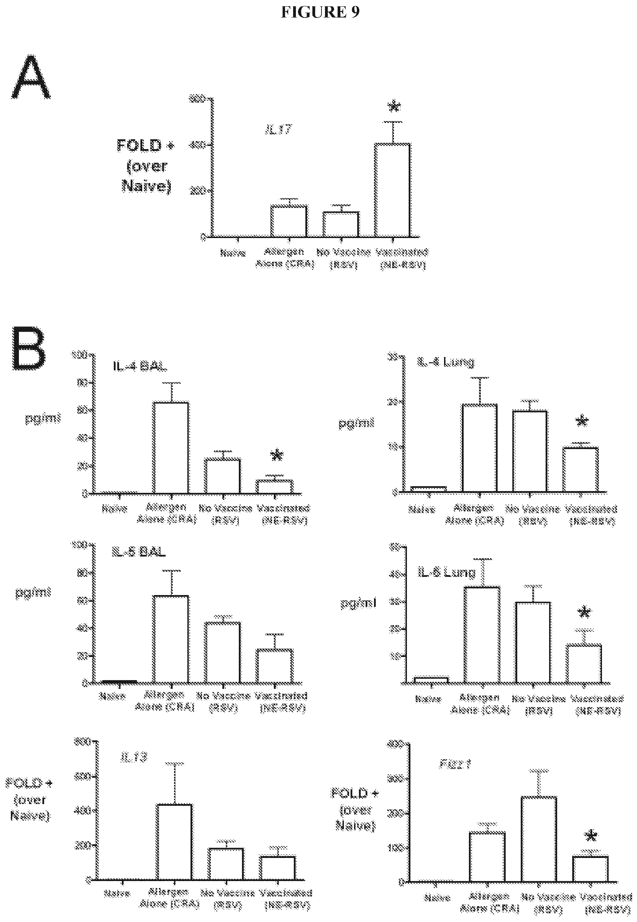

The nanoemulsion comprising vaccine compositions of the present invention are defined in the claims. Nanoemulsion compositions which may be utilized in vaccine compositions are disclosed in Hamouda et al., J. Infect Dis., 180:1939 (1999); Hamouda and Baker, J. Appl. Microbiol., 89:397 (2000); and Donovan et al., Antivir. Chem. Chemother., 11:41 (2000), as well as those shown in Tables 1 and 2 and Figures 4 and 9. Preferred nanoemulsions of the present invention are those that are disclosed in the dependent claims. Accordingly, preferred emulsion formulations utilize non-toxic solvents, such as ethanol, and achieve more effective killing at lower concentrations of emulsion. One of the nanoemulsions described in the Tables below may be utilized.

| Table 1 | | |
|---|---|---|
| Nanoemulsion Formulations | | |
| Name | Oil Phase Formula | Water to Oil Phase Ratio (Vol/Vol) |
| X8P | 1 vol. Tri(N-butyl)phosphate | 4:1 |
| | 1 vol. TRITON X-100 | |
| | 8 vol. Soybean oil | |
| NN | 86.5 g Glycerol monooleate | 3:1 |
| | 60.1 ml Nonoxynol-9 | |
| | 24.2 g GENEROL 122 | |
| | 3.27 g Cetylpyridinium chloride | |
| | 554 g Soybean oil | |
| W₈₀8P | 86.5 g Glycerol monooleate | 3.2:1 |
| | 21.2 g Polysorbate 60 | |
| | 24.2 g GENEROL 122 | |
| | 3.27 g Cetylpyddinium chloride | |
| | 4 ml Peppermint oil | |
| | 554 g Soybean oil | |
| SS | 86.5 g Glycerol monooleate | 3.2:1 |
| | 21.2 g Polysorbate 60 | (1% bismuth in water) |
| | 24.2 g GENEROL 122 | |
| | 3.27 g Cetylpyridinium chloride | |
| | 554 g Soybean oil | |

| **Table 2** | | |
|---|---|---|
| **Nanoemulsion Formulations** | | |
| **Nanoemulsion** | **Composition** | |
| X8P | 8% TRITON X-100; 8% Tributyl phosphate; 64% Soybean oil; 20% Water | |
| W₂₀5EC | 5% TWEEN 20; 8% Ethanol; 1% Cetylpyridinium Chloride; 64% Soybean oil; 22% Water | |
| EC | 1% Cetylpyridinium Chloride; 8% Ethanol; 64% Soybean oil; 27% Water | |
| Y3EC | 3% TYLOXAPOL; 1% Cetylpyridinium Chloride; 8% Ethanol; 64% Soybean oil; 24% Water | |
| X4E | 4% TRITON X-100; 8% Ethanol; 64% Soybean oil; 24% Water | |

The emulsions may contain (i) an aqueous phase and (ii) an oil phase containing ethanol as the organic solvent and optionally a germination enhancer, and (iii) TYLOXAPOL as the surfactant (preferably 2-5%, more preferably 3%). This formulation is highly efficacious against microbes and is also non-irritating and non-toxic to mammalian users (and can thus be contacted with mucosal membranes).

The emulsions may comprise a first emulsion emulsified within a second emulsion, wherein (a) the first emulsion comprises (i) an aqueous phase; and (ii) an oil phase comprising an oil and an organic solvent; and (iii) a surfactant; and (b) the second emulsion comprises (i) an aqueous phase; and (ii) an oil phase comprising an oil and a cationic containing compound; and (iii) a surfactant.

The following description provides a number of exemplary emulsions including formulations for compositions X8P and X₈W₆₀PC. X8P comprises a water-in oil nanoemulsion, in which the oil phase was made from soybean oil, tri-n-butyl phosphate, and TRITON X-100 in 80% water. X₈W₆₀PC comprises a mixture of equal volumes of X8P with W₈₀8P. W₈₀8P is a liposome-like compound made of glycerol monostearate, refined soya sterols (e.g., GENEROL sterols), TWEEN 60, soybean oil, a cationic ion halogen-containing CPC and peppermint oil. The GENEROL family are a group of a polyethoxylated soya sterols (Henkel Corporation, Ambler, Pennsylvania). Certain emulsion formulations are given in Table 1. These particular formulations may be found in U.S. Pat. Nos. 5,700,679 (NN); 5,618,840; 5,549,901 (W₈₀8P); and 5,547,677.

The X8W₆₀PC emulsion is manufactured by first making the W₈₀8P emulsion and X8P emulsions separately. A mixture of these two emulsions is then re-emulsified to produce a fresh emulsion composition termed X8W₆₀PC. Methods of producing such emulsions are described in U.S. Pat. Nos. 5,103,497 and 4,895,452. These compounds have broad-spectrum antimicrobial activity, and are able to inactivate vegetative bacteria through membrane disruption.

The compositions listed above are only exemplary and those of skill in the art will be able to alter the amounts of the components. Those skilled in the art will understand that the ratio of oil phase to water as well as the individual oil carrier, surfactant CPC and organic phosphate buffer, components of each composition may vary.

Although certain compositions comprising X8P have a water to oil ratio of 4:1, it is understood that the X8P may be formulated to have more or less of a water phase. For example, there may be 3, 4, 5, 6, 7, 8, 9, 10, or more parts of the water phase to each part of the oil phase. The same holds true for the W₈₀8P formulation. Similarly, the ratio of Tri(N-butyl)phosphate:TRITON X-100:soybean oil also may be varied.

Although Table 1 lists specific amounts of glycerol monooleate, polysorbate 60, GENEROL 122, cetylpyridinium chloride, and carrier oil for W₈₀8P, these are merely exemplary. An emulsion that has the properties of W₈₀8P may be formulated that has different concentrations of each of these components or indeed different components that will fulfill the same function. For example, the emulsion may have between about 80 to about 100g of glycerol monooleate in the initial oil phase. The emulsion may have between about 15 to about 30 g polysorbate 60 in the initial oil phase. The composition may comprise between about 20 to about 30 g of a GENEROL sterol, in the initial oil phase.

The nanoemulsions structure may play a role in their biocidal activity as well as contributing to the non-toxicity of these emulsions. For example, the active component in X8P, TRITON-X100 shows less biocidal activity against virus at concentrations equivalent to 11% X8P. Adding the oil phase to the detergent and solvent markedly reduces the toxicity of these agents in tissue culture at the same concentrations. It is suggested that the nanoemulsion enhances the interaction of its components with the pathogens thereby facilitating the inactivation of the pathogen and reducing the toxicity of the individual components. It should be noted that when all the components of X8P are combined in one composition but are not in a nanoemulsion structure, the mixture is not as effective as an antimicrobial as when the components are in a nanoemulsion structure.

Numerous additional examples presented in classes of formulations with like compositions are presented below. The following compositions recite various ratios and mixtures of active components. One skilled in the art will appreciate that the below recited formulation are exemplary and that additional formulations comprising similar percent ranges of the recited components are possible.

The formulation may comprise from about 3 to 8 vol. % of TYLOXAPOL, about 8 vol. % of ethanol, about 1 vol. % of cetylpyridinium chloride (CPC), about 60 to 70 vol. % oil (*e.g.,* soybean oil), about 15 to 25 vol. % of aqueous phase (*e.g.,* DiH₂O or PBS), and in some formulations less than about 1 vol. % of 1N NaOH. Some of these formulations may comprise PBS. It is contemplated that the addition of 1N NaOH and/or PBS, allows the user to advantageously control the pH of the formulations, such that pH ranges from about 4.0 to about 10.0, and more preferably from about 7.1 to 8.5 are achieved. For example, one formulation comprises about 3 vol. % of TYLOXAPOL, about 8 vol. % of ethanol, about 1 vol. % of CPC, about 64 vol. % of soybean oil, and about 24 vol. % of DiH₂O (designated herein as Y3EC). Another similar formulation comprises about 3.5 vol. % of TYLOXAPOL, about 8 vol. % of ethanol, and about 1 vol. % of CPC, about 64 vol. % of soybean oil, and about 23.5 vol. % of DiH₂O (designated herein as Y3.5EC). Yet another formulation comprises about 3 vol. % of TYLOXAPOL, about 8 vol. % of ethanol, about 1 vol. % of CPC, about 0.067 vol. % of 1N NaOH, such that the pH of the formulation is about 7.1, about 64 vol. % of soybean oil, and about 23.93 vol. % of DiH₂O (designated herein as Y3EC pH 7.1). Still another formulation comprises about 3 vol. % of TYLOXAPOL, about 8 vol. % of ethanol, about 1 vol. % of CPC, about 0.67 vol. % of 1N NaOH, such that the pH of the formulation is about 8.5, and about 64 vol. % of soybean oil, and about 23.33 vol. % of DiH₂O (designated herein as Y3EC pH 8.5). Another similar formulation comprises about 4% TYLOXAPOL, about 8 vol. % ethanol, about 1% CPC, and about 64 vol. % of soybean oil, and about 23 vol. % of DiH₂O (designated herein as Y4EC). The formulation may comprise about 8% TYLOXAPOL, about 8% ethanol, about 1 vol. % of CPC, and about 64 vol. % of soybean oil, and about 19 vol. % of DiH₂O (designated herein as Y8EC). A further formulation comprises about 8 vol. % of TYLOXAPOL, about 8 vol. % of ethanol, about 1 vol. % of CPC, about 64 vol. % of soybean oil, and about 19 vol. % of 1x PBS (designated herein as Y8EC PBS).

The formulations may comprise about 8 vol. % of ethanol, and about 1 vol. % of CPC, and about 64 vol. % of oil (*e.g.,* soybean oil), and about 27 vol. % of aqueous phase (*e.g.,* DiH₂O or PBS) (designated herein as EC).

Some formulations may comprise from about 8 vol. % of sodium dodecyl sulfate (SDS), about 8 vol. % of tributyl phosphate (TBP), and about 64 vol. % of oil (*e.g.,* soybean oil), and about 20 vol. % of aqueous phase (*e.g.,* DiH₂O or PBS) (designated herein as S8P).

The formulation may comprise from about 1 to 2 vol. % of TRITON X-100, from about 1 to 2 vol. % of TYLOXAPOL, from about 7 to 8 vol. % of ethanol, about 1 vol. % of cetylpyridinium chloride (CPC), about 64 to 57.6 vol. % of oil (*e.g.,* soybean oil), and about 23 vol. % of aqueous phase (*e.g.,* DiH₂O or PBS). Additionally, some of these formulations further comprise about 5 mM of L-alanine/Inosine, and about 10 mM ammonium chloride. Some of these formulations comprise PBS. It is contemplated that the addition of PBS in some of these formulations, allows the user to advantageously control the pH of the formulations. For example, one formulation comprises about 2 vol. % of TRITON X-100, about 2 vol. % of TYLOXAPOL, about 8 vol. % of ethanol, about 1 vol. % CPC, about 64 vol. % of soybean oil, and about 23 vol. % of aqueous phase DiH₂O. Another formulation comprises about 1.8 vol. % of TRITON X-100, about 1.8 vol. % of TYLOXAPOL, about 7.2 vol. % of ethanol, about 0.9 vol. % of CPC, about 5 mM L-alanine/Inosine, and about 10 mM ammonium chloride, about 57.6 vol. % of soybean oil, and the remainder of 1x PBS (designated herein as 90% X2Y2EC/GE).

In a preferred embodiment of the present invention, the NE formulations according to claim 9 comprise about 5 vol. % of TWEEN 80, about 8 vol. % of ethanol, about 1 vol. % of CPC, about 64 vol. % of oil (*e.g.,* soybean oil), and about 22 vol. % of DiH₂O (designated herein as W₈₀5EC).

In still other embodiments of the present invention also comprised by claim 9, the NE formulations comprise from 5 vol. % of TWEEN 20, about 8 vol. % of ethanol, about 1 vol. % of CPC, about 64 vol. % of oil (*e.g.,* soybean oil), and about 22 vol. % of DiH₂O (designated herein as W₂₀5EC).

The formulations may comprise from about 2 to 8 vol. % of TRITON X-100, about 8 vol. % of ethanol, about 1 vol. % of CPC, about 60 to 70 vol. % of oil (*e.g.,* soybean, or olive oil), and about 15 to 25 vol. % of aqueous phase (*e.g.,* DiH₂O or PBS). For example, formulations comprising about 2 vol. % of TRITON X-100, about 8 vol. % of ethanol, about 64 vol. % of soybean oil, and about 26 vol. % of DiH₂O (designated herein as X2E) are contemplated. Formulations may comprise about 3 vol. % of TRITON X-100, about 8 vol. % of ethanol, about 64 vol. % of soybean oil, and about 25 vol. % of DiH₂O (designated herein as X3E). The formulations may comprise about 4 vol. % TRITON X-100, about 8 vol. % of ethanol, about 64 vol. % of soybean oil, and about 24 vol. % of DiH₂O (designated herein as X4E). The formulations may comprise about 5 vol. % of TRITON X-100, about 8 vol. % of ethanol, about 64 vol. % of soybean oil, and about 23 vol. % of DiH₂O (designated herein as X5E). The formulations may comprise about 6 vol. % of TRITON X-100, about 8 vol. % of ethanol, about 64 vol. % of soybean oil, and about 22 vol. % of DiH₂O (designated herein as X6E). The formulations may comprise about 8 vol. % of TRITON X-100, about 8 vol. % of ethanol, about 64 vol. % of soybean oil, and about 20 vol. % of DiH₂O (designated herein as X8E). The formulations may comprise about 8 vol. % of TRITON X-100, about 8 vol. % of ethanol, about 64 vol. % of olive oil, and about 20 vol. % of DiH₂O (designated herein as X8E O). The formulations may comprise 8 vol. % of TRITON X-100, about 8 vol. % ethanol, about 1 vol. % CPC, about 64 vol. % of soybean oil, and about 19 vol. % of DiH₂O (designated herein as X8EC).

The formulations may comprise from about 1 to 2 vol. % of TRITON X-100, from about 1 to 2 vol. % of TYLOXAPOL, from about 6 to 8 vol. % TBP, from about 0.5 to 1.0 vol. % of CPC, from about 60 to 70 vol. % of oil (*e.g.,* soybean), and about 1 to 35 vol. % of aqueous phase (*e.g.,* DiH₂O or PBS). Additionally, certain of these formulations may comprise from about 1 to 5 vol. % of trypticase soy broth, from about 0.5 to 1.5 vol. % of yeast extract, about 5 mM L-alanine/Inosine, about 10 mM ammonium chloride, and from about 20-40 vol. % of liquid baby formula. In some of the formulations comprising liquid baby formula, the formula comprises a casein hydrolysate (*e.g.,* Neutramigen, or Progestimil, and the like). In some of these formulations, the formulations further comprise from about 0.1 to 1.0 vol. % of sodium thiosulfate, and from about 0.1 to 1.0 vol. % of sodium citrate. Other similar formulations comprising these basic components employ phosphate buffered saline (PBS) as the aqueous phase. For example, one formulation comprises about 2 vol. % of TRITON X-100, about 2 vol. % TYLOXAPOL, about 8 vol. % TBP, about 1 vol. % of CPC, about 64 vol. % of soybean oil, and about 23 vol. % of DiH₂O (designated herein as X2Y2EC). The formulation may comprise about 2 vol. % of TRITON X-100, about 2 vol. % TYLOXAPOL, about 8 vol. % TBP, about 1 vol. % of CPC, about 0.9 vol. % of sodium thiosulfate, about 0.1 vol. % of sodium citrate, about 64 vol. % of soybean oil, and about 22 vol. % of DiH₂O (designated herein as X2Y2PC STS1). The formulations may comprise about 1.7 vol. % TRITON X-100, about 1.7 vol. % TYLOXAPOL, about 6.8 vol. % TBP, about 0.85% CPC, about 29.2% NEUTRAMIGEN, about 54.4 vol. % of soybean oil, and about 4.9 vol. % of DiH₂O (designated herein as 85% X2Y2PC/baby). The formulations may comprise about 1.8 vol. % of TRITON X-100, about 1.8 vol. % of TYLOXAPOL, about 7.2 vol. % of TBP, about 0.9 vol. % of CPC, about 5mM L-alanine/Inosine, about 10mM ammonium chloride, about 57.6 vol. % of soybean oil, and the remainder vol. % of 0.1x PBS (designated herein as 90% X2Y2 PC/GE). The formulations may comprise about 1.8 vol. % of TRITON X-100, about 1.8 vol. % of TYLOXAPOL, about 7.2 vol. % TBP, about 0.9 vol. % of CPC, and about 3 vol. % trypticase soy broth, about 57.6 vol. % of soybean oil, and about 27.7 vol. % of DiH₂O (designated herein as 90% X2Y2PC/TSB). The formulations may comprise about 1.8 vol. % TRITON X-100, about 1.8 vol. % TYLOXAPOL, about 7.2 vol. % TBP, about 0.9 vol. % CPC, about 1 vol. % yeast extract, about 57.6 vol. % of soybean oil, and about 29.7 vol. % of DiH₂O (designated herein as 90% X2Y2PC/YE).

The formulations may comprise about 3 vol. % of TYLOXAPOL, about 8 vol. % of TBP, and about 1 vol. % of CPC, about 60 to 70 vol. % of oil (*e.g.,* soybean or olive oil), and about 15 to 30 vol. % of aqueous phase (*e.g.,* DiH₂O or PBS). The formulations may comprise about 3 vol. % of TYLOXAPOL, about 8 vol. % of TBP, and about 1 vol. % of CPC, about 64 vol. % of soybean, and about 24 vol. % of DiH₂O (designated herein as Y3PC).

The formulations may comprise from about 4 to 8 vol. % of TRITON X-100, from about 5 to 8 vol. % of TBP, about 30 to 70 vol. % of oil (*e.g.,* soybean or olive oil), and about 0 to 30 vol. % of aqueous phase (*e.g.,* DiH₂O or PBS). Additionally, certain of these formulations further comprise about 1 vol. % of CPC, about 1 vol. % of benzalkonium chloride, about 1 vol. % cetylyridinium bromide, about 1 vol. % cetyldimethyletylammonium bromide, 500 µM EDTA, about 10 mM ammonium chloride, about 5 mM Inosine, and about 5 mM L-alanine. For example, the formulations comprise about 8 vol. % of TRITON X-100, about 8 vol. % of TBP, about 64 vol. % of soybean oil, and about 20 vol. % of DiH₂O (designated herein as X8P). The formulations may comprise about 8 vol. % of TRITON X-100, about 8 vol. % of TBP, about 1% of CPC, about 64 vol. % of soybean oil, and about 19 vol. % of DiH₂O (designated herein as X8PC). The formulations may comprise about 8 vol. % TRITON X-100, about 8 vol. % of TBP, about 1 vol. % of CPC, about 50 vol. % of soybean oil, and about 33 vol. % of DiH₂O (designated herein as ATB-X1001). The formulations may comprise about 8 vol. % of TRITON X-100, about 8 vol. % of TBP, about 2 vol. % of CPC, about 50 vol. % of soybean oil, and about 32 vol. % of DiH₂O (designated herein as ATB-X002). Other formulations may comprise about 4 vol. % TRITON X-100, about 4 vol. % of TBP, about 0.5 vol. % of CPC, about 32 vol. % of soybean oil, and about 59.5 vol. % of DiH₂O (designated herein as 50% X8PC). Still another related formulations may comprise about 8 vol. % of TRITON X-100, about 8 vol. % of TBP, about 0.5 vol. % CPC, about 64 vol. % of soybean oil, and about 19.5 vol. % of DiH₂O (designated herein as X8PC_{1/2}). The formulations may comprise about 8 vol. % of TRITON X-100, about 8 vol. % of TBP, about 2 vol. % of CPC, about 64 vol. % of soybean oil, and about 18 vol. % of DiH₂O (designated herein as X8PC2). The formulations may comprise about 8 vol. % of TRITON X-100, about 8% of TBP, about 1% of benzalkonium chloride, about 50 vol. % of soybean oil, and about 33 vol. % of DiH₂O (designated herein as X8P BC). The formulation may comprise about 8 vol. % of TRITON X-100, about 8 vol. % of TBP, about 1 vol. % of cetylyridinium bromide, about 50 vol. % of soybean oil, and about 33 vol. % of DiH₂O (designated herein as X8P CPB). The formulations may comprise about 8 vol. % of TRITON X-100, about 8 vol. % of TBP, about 1 vol. % of cetyldimethyletylammonium bromide, about 50 vol. % of soybean oil, and about 33 vol. % of DiH₂O (designated herein as X8P CTAB). The formulations may comprise about 8 vol. % of TRITON X-100, about 8 vol. % of TBP, about 1 vol. % of CPC, about 500 µM EDTA, about 64 vol. % of soybean oil, and about 15.8 vol. % DiH₂O (designated herein as X8PC EDTA). Additional similar formulations may comprise 8 vol. % of TRITON X-100, about 8 vol. % of TBP, about 1 vol. % of CPC, about 10 mM ammonium chloride, about 5mM Inosine, about 5mM L-alanine, about 64 vol. % of soybean oil, and about 19 vol. % of DiH₂O or PBS (designated herein as X8PC GE₁ₓ). The formulations may further comprise about 5 vol. % of TRITON X-100, about 5% of TBP, about 1 vol. % of CPC, about 40 vol. % of soybean oil, and about 49 vol. % of DiH₂O (designated herein as X5P₅C).

The formulations may comprise about 2 vol. % TRITON X-100, about 6 vol. % TYLOXAPOL, about 8 vol. % ethanol, about 64 vol. % of soybean oil, and about 20 vol. % of DiH₂O (designated herein as X2Y6E).

The formulations may comprise about 8 vol. % of TRITON X-100, and about 8 vol. % of glycerol, about 60 to 70 vol. % of oil (*e.g.,* soybean or olive oil), and about 15 to 25 vol. % of aqueous phase (*e.g.,* DiH₂O or PBS). Certain related formulations further comprise about 1 vol. % L-ascorbic acid. For example, one particular formulation comprises about 8 vol. % of TRITON X-100, about 8 vol. % of glycerol, about 64 vol. % of soybean oil, and about 20 vol. % of DiH₂O (designated herein as X8G). The formulations may comprise about 8 vol. % of TRITON X-100, about 8 vol. % of glycerol, about 1 vol. % of L-ascorbic acid, about 64 vol. % of soybean oil, and about 19 vol. % of DiH₂O (designated herein as XBGV_{c}).

The formulations may comprise about 8 vol. % of TRITON X-100, from about 0.5 to 0.8 vol. % of TWEEN 60, from about 0.5 to 2.0 vol. % of CPC, about 8 vol. % of TBP, about 60 to 70 vol. % of oil (e.g., soybean or olive oil), and about 15 to 25 vol. % of aqueous phase (*e.g.,* DiH₂O or PBS). For example, the formulations may comprise about 8 vol. % of TRITON X-100, about 0.70 vol. % of TWEEN 60, about 1 vol. % of CPC, about 8 vol. % of TBP, about 64 vol. % of soybean oil, and about 18.3 vol. % of DiH₂O (designated herein as X8W60PC₁). Another related formulation comprises about 8 vol. % of TRITON X-100, about 0.71 vol. % of TWEEN 60, about 1 vol. % of CPC, about 8 vol. % of TBP, about 64 vol. % of soybean oil, and about 18.29 vol. % of DiH₂O (designated herein as W60_{0.7}X8PC). The formulations may comprise from about 8 vol. % of TRITON X-100, about 0.7 vol. % of TWEEN 60, about 0.5 vol. % of CPC, about 8 vol. % of TBP, about 64 to 70 vol. % of soybean oil, and about 18.8 vol. % of DiH₂O (designated herein as X8W60PC₂). The formulations may comprises about 8 vol. % of TRITON X-100, about 0.71 vol. % of TWEEN 60, about 2 vol. % of CPC, about 8 vol. % of TBP, about 64 vol. % of soybean oil, and about 17.3 vol. % of DiH₂O. The formulations may comprise about 0.71 vol. % of TWEEN 60, about 1 vol. % of CPC, about 8 vol. % of TBP, about 64 vol. % of soybean oil, and about 25.29 vol. % of DiH₂O (designated herein as W60_{0.7}PC).

The formulations may comprise about 2 vol. % of dioctyl sulfosuccinate, either about 8 vol. % of glycerol, or about 8 vol. % TBP, in addition to, about 60 to 70 vol. % of oil (*e.g.,* soybean or olive oil), and about 20 to 30 vol. % of aqueous phase (*e.g.,* DiH₂O or PBS). For example, one formulation comprises about 2 vol. % of dioctyl sulfosuccinate, about 8 vol. % of glycerol, about 64 vol. % of soybean oil, and about 26 vol. % of DiH₂O (designated herein as D2G). The formulations may comprise about 2 vol. % of dioctyl sulfosuccinate, and about 8 vol. % of TBP, about 64 vol. % of soybean oil, and about 26 vol. % of DiH₂O (designated herein as D2P).

The formulations may comprise about 8 to 10 vol. % of glycerol, and about 1 to 10 vol. % of CPC, about 50 to 70 vol. % of oil (*e.g.,* soybean or olive oil), and about 15 to 30 vol. % of aqueous phase (*e.g.,* DiH₂O or PBS). Additionally, the compositions may further comprise about 1 vol. % of L-ascorbic acid. For example, one particular formulation comprises about 8 vol. % of glycerol, about 1 vol. % of CPC, about 64 vol. % of soybean oil, and about 27 vol. % of DiH₂O (designated herein as GC). An additional related formulation comprises about 10 vol. % of glycerol, about 10 vol. % of CPC, about 60 vol. % of soybean oil, and about 20 vol. % of DiH₂O (designated herein as GC10). The formulations may comprise about 10 vol. % of glycerol, about 1 vol. % of CPC, about 1 vol. % of L-ascorbic acid, about 64 vol. % of soybean or oil, and about 24 vol. % of DiH₂O (designated herein as GCV_{c}).

The formulations may comprise about 8 to 10 vol. % of glycerol, about 8 to 10 vol. % of SDS, about 50 to 70 vol. % of oil (*e.g.,* soybean or olive oil), and about 15 to 30 vol. % of aqueous phase (*e.g.,* DiH₂O or PBS). Additionally, in certain of these formulations, the compositions further comprise about 1 vol. % of lecithin, and about 1 vol. % of p-Hydroxybenzoic acid methyl ester. Exemplary formulations may comprise about 8 vol. % SDS, 8 vol. % of glycerol, about 64 vol. % of soybean oil, and about 20 vol. % of DiH₂O (designated herein as S8G). A related formulation comprises about 8 vol. % of glycerol, about 8 vol. % of SDS, about 1 vol. % of lecithin, about 1 vol. % of p-Hydroxybenzoic acid methyl ester, about 64 vol. % of soybean oil, and about 18 vol. % of DiH₂O (designated herein as S8GL1B1).

The formulations may comprise about 4 vol. % of TWEEN 80, about 4 vol. % of TYLOXAPOL, about 1 vol. % of CPC, about 8 vol. % of ethanol, about 64 vol. % of soybean oil, and about 19 vol. % of DiH₂O (designated herein as W₈₀4Y4EC).

The formulations may comprise about 0.01 vol. % of CPC, about 0.08 vol. % of TYLOXAPOL, about 10 vol. % of ethanol, about 70 vol. % of soybean oil, and about 19.91 vol. % of DiH₂O (designated herein as Y.08EC.01).

The formulations may comprise about 8 vol. % of sodium lauryl sulfate, and about 8 vol. % of glycerol, about 64 vol. % of soybean oil, and about 20 vol. % of DiH₂O (designated herein as SLS8G).

The specific formulations described above are simply examples to illustrate the variety of compositions that may find use. Many variations of the above formulation, as well as additional nanoemulsions may be used. To determine if a candidate emulsion is suitable for use, three criteria may be analyzed. Using the methods and standards described herein, candidate emulsions can be easily tested to determine if they are suitable. First, the desired ingredients are prepared using the methods described herein, to determine if an emulsion can be formed. If an emulsion cannot be formed, the candidate is rejected. For example, a candidate composition made of 4.5% sodium thiosulfate, 0.5% sodium citrate, 10% n-butanol, 64% soybean oil, and 21% DiH₂O did not form an emulsion.

Second, the candidate emulsion should form a stable emulsion. An emulsion is stable if it remains in emulsion form for a sufficient period to allow its intended use. For example, for emulsions that are to be stored, shipped, etc., it may be desired that the composition remain in emulsion form for months to years. Typical emulsions that are relatively unstable, will lose their form within a day. For example, a candidate composition made of 8% 1-butanol, 5% TWEEN 10, 1% CPC, 64% soybean oil, and 22% DiH₂O did not form a stable emulsion. The following candidate emulsions were shown to be stable using the methods described herein: 0.08% TRITON X-100, 0.08% Glycerol, 0.01% Cetylpyridinium Chloride, 99% Butter, and 0.83% diH₂O (designated herein as 1% X8GC Butter); 0.8% TRITON X-100, 0.8% Glycerol, 0.1% Cetylpyridinium Chloride, 6.4% Soybean Oil, 1.9% diH₂O, and 90% Butter (designated herein as 10% X8GC Butter); 2% W₂₀5EC, 1% Natrosol 250L NF, and 97% diH₂O (designated herein as 2% W₂₀5EC L GEL); 1% Cetylpyridinium Chloride, 5% TWEEN 20, 8% Ethanol, 64% 70 Viscosity Mineral Oil, and 22% diH₂O (designated herein as W₂₀5EC 70 Mineral Oil); 1% Cetylpyridinium Chloride, 5% TWEEN 20, 8% Ethanol, 64% 350 Viscosity Mineral Oil, and 22% diH₂O (designated herein as W₂₀5EC 350 Mineral Oil).

Third, the candidate emulsion should have efficacy for its intended use. For example, an anti-bacterial emulsion should kill or disable pathogens to a detectable level. As shown herein, certain emulsions have efficacy against specific microorganisms, but not against others. Using the methods described herein, one is capable of determining the suitability of a particular candidate emulsion against the desired microorganism. Generally, this involves exposing the microorganism to the emulsion for one or more time periods in a side-by-side experiment with the appropriate control samples (*e.g.,* a negative control such as water) and determining if, and to what degree, the emulsion kills or disables the microorganism. For example, a candidate composition made of 1% ammonium chloride, 5% TWEEN 20, 8% ethanol, 64% soybean oil, and 22% DiH₂O was shown not to be an effective emulsion. The following candidate emulsions were shown to be effective using the methods described herein: 5% TWEEN 20, 5% Cetylpyridinium Chloride, 10% Glycerol, 60% Soybean Oil, and 20% diH₂O (designated herein as W₂₀5GC5); 1% Cetylpyridinium Chloride, 5% TWEEN 20, 10% Glycerol, 64% Soybean Oil, and 20% diH₂O (designated herein as W₂₀5GC); 1% Cetylpyridinium Chloride, 5% TWEEN 20, 8% Ethanol, 64% Olive Oil, and 22% diH₂O (designated herein as W₂₀5EC Olive Oil); 1% Cetylpyridinium Chloride, 5% TWEEN 20, 8% Ethanol, 64% Flaxseed Oil, and 22% diH₂O (designated herein as W₂₀5EC Flaxseed Oil); 1% Cetylpyridinium Chloride, 5% TWEEN 20, 8% Ethanol, 64% Corn Oil, and 22% diH₂O (designated herein as W₂₀5EC Corn Oil); 1% Cetylpyridinium Chloride, 5% TWEEN 20, 8% Ethanol, 64% Coconut Oil, and 22% diH₂O (designated herein as W₂₀5EC Coconut Oil); 1% Cetylpyridinium Chloride, 5% TWEEN 20, 8% Ethanol, 64% Cottonseed Oil, and 22% diH₂O (designated herein as W₂₀5EC Cottonseed Oil); 8% Dextrose, 5% TWEEN 10, 1% Cetylpyridinium Chloride, 64% Soybean Oil, and 22% diH₂O (designated herein as W₂₀5C Dextrose); 8% PEG 200, 5% TWEEN 10, 1% Cetylpyridinium Chloride, 64% Soybean Oil, and 22% diH₂O (designated herein as W₂₀5C PEG 200); 8% Methanol, 5% TWEEN 10, 1% Cetylpyridinium Chloride, 64% Soybean Oil, and 22% diH₂O (designated herein as W₂₀5C Methanol); 8% PEG 1000, 5% TWEEN 10, 1% Cetylpyridinium Chloride, 64% Soybean Oil, and 22% diH₂O (designated herein as W₂₀5C PEG 1000); 2% W₂₀5EC, 2% Natrosol 250H NF, and 96% diH₂O (designated herein as 2% W₂₀5EC Natrosol 2, also called 2% W₂₀5EC GEL); 2% W₂₀5EC, 1% Natrosol 250H NF, and 97% diH₂O (designated herein as 2% W₂₀5EC Natrosol 1); 2% W₂₀5EC, 3% Natrosol 250H NF, and 95% diH₂O (designated herein as 2% W₂₀5EC Natrosol 3); 2% W₂₀5EC, 0.5% Natrosol 250H NF, and 97.5% diH₂O (designated herein as 2% W₂₀5EC Natrosol 0.5); 2% W₂₀5EC, 2% Methocel A, and 96% diH₂O (designated herein as 2% W₂₀5EC Methocel A); 2% W₂₀5EC, 2% Methocel K, and 96% diH₂O (designated herein as 2% W₂₀5EC Methocel K); 2% Natrosol, 0.1% X8PC, 0.1x PBS, 5 mM L-alanine, 5 mM Inosine, 10 mM Ammonium Chloride, and diH₂O (designated herein as 0.1% X8PC/GE+2% Natrosol); 2% Natrosol, 0.8% TRITON X-100, 0.8% Tributyl Phosphate, 6.4% Soybean Oil, 0.1% Cetylpyridinium Chloride, 0.1x PBS, 5 mM L-alanine, 5 mM Inosine, 10 mM Ammonium Chloride, and diH₂O (designated herein as 10% X8PC/GE+2% Natrosol); 1% Cetylpyridinium Chloride, 5% TWEEN 20, 8% Ethanol, 64% Lard, and 22% diH₂O (designated herein as W₂₀5EC Lard); 1% Cetylpyridinium Chloride, 5% TWEEN 20, 8% Ethanol, 64% Mineral Oil, and 22% diH₂O (designated herein as W₂₀5EC Mineral Oil); 0.1% Cetylpyridinium Chloride, 2% Nerolidol, 5% TWEEN 20, 10% Ethanol, 64% Soybean Oil, and 18.9% diH₂O (designated herein as W₂₀5EC_{0.1}N); 0.1% Cetylpyridinium Chloride, 2% Farnesol, 5% TWEEN 20, 10% Ethanol, 64% Soybean Oil, and 18.9% diH₂O (designated herein as W₂₀5EC_{0.1}F); 0.1% Cetylpyridinium Chloride, 5% TWEEN 20, 10% Ethanol, 64% Soybean Oil, and 20.9% diH₂O (designated herein as W₂₀5EC_{0.}1); 10% Cetylpyridinium Chloride, 8% Tributyl Phosphate, 8% TRITON X-100, 54% Soybean Oil, and 20% diH₂O (designated herein as X8PC₁₀); 5% Cetylpyridinium Chloride, 8% TRITON X-100, 8% Tributyl Phosphate, 59% Soybean Oil, and 20% diH₂O (designated herein as X8PC₅); 0.02% Cetylpyridinium Chloride, 0.1% TWEEN 20, 10% Ethanol, 70% Soybean Oil, and 19.88% diH₂O (designated herein as W₂₀0.1EC_{0.02}); 1% Cetylpyridinium Chloride, 5% TWEEN 20, 8% Glycerol, 64% Mobil 1, and 22% diH₂O (designated herein as W₂₀5GC Mobil 1); 7.2% TRITON X-100, 7.2% Tributyl Phosphate, 0.9% Cetylpyridinium Chloride, 57.6% Soybean Oil, 0.1x PBS, 5 mM L-alanine, 5 mM Inosine, 10 mM Ammonium Chloride, and 25.87% diH₂O (designated herein as 90% X8PC/GE); 7.2% TRITON X-100, 7.2% Tributyl Phosphate, 0.9% Cetylpyridinium Chloride, 57.6% Soybean Oil, 1% EDTA, 5 mM L-alanine, 5 mM Inosine, 10 mM Ammonium Chloride, 0.1x PBS, and diH₂O (designated herein as 90% X8PC/GE EDTA); and 7.2% TRITON X-100, 7.2% Tributyl Phosphate, 0.9% Cetylpyridinium Chloride, 57.6% Soybean Oil, 1% Sodium Thiosulfate, 5 mM L-alanine, 5 mM Inosine, 10 mM Ammonium Chloride, 0.1x PBS, and diH₂O (designated herein as 90% X8PC/GE STS).

### 1. Aqueous Phase

The emulsion comprises an aqueous phase as defined in claim 1. The emulsion may comprises about 5 to 50, preferably 10 to 40, more preferably 15 to 30, vol. % aqueous phase, based on the total volume of the emulsion (although other concentrations are also contemplated). The aqueous phase may comprises water at a pH of about 4 to 10, preferably about 6 to 8. The water is preferably deionized (hereinafter "DiH₂O"). The aqueous phase may comprise phosphate buffered saline (PBS). The aqueous phase may be sterile and pyrogen free.

### 2. Oil Phase

The emulsion comprises an oil phase as defined in claim 1. The oil phase (e.g., carrier oil) of the emulsion may comprises 30-90, preferably 60-80, and more preferably 60-70, vol. % of oil, based on the total volume of the emulsion (although higher and lower concentrations also find use in emulsions described herein).

The oil in the nanoemulsion vaccine of the invention as defined in claim 12 can be any cosmetically or pharmaceutically acceptable oil. The oil can be volatile or non-volatile, and may be chosen from animal oil, vegetable oil, natural oil, synthetic oil, hydrocarbon oils, silicone oils, semi-synthetic derivatives thereof, and combinations thereof.

Suitable oils include, but are not limited to, mineral oil, squalene oil, flavor oils, silicon oil, essential oils, water insoluble vitamins, Isopropyl stearate, Butyl stearate, Octyl palmitate, Cetyl palmitate, Tridecyl behenate, Diisopropyl adipate, Dioctyl sebacate, Menthyl anthranhilate, Cetyl octanoate, Octyl salicylate, Isopropyl myristate, neopentyl glycol dicarpate cetols, Ceraphyls®, Decyl oleate, diisopropyl adipate, C₁₂₋₁₅ alkyl lactates, Cetyl lactate, Lauryl lactate, Isostearyl neopentanoate, Myristyl lactate, Isocetyl stearoyl stearate, Octyldodecyl stearoyl stearate, Hydrocarbon oils, Isoparaffin, Fluid paraffins, Isododecane, Petrolatum, Argan oil, Canola oil, Chile oil, Coconut oil, corn oil, Cottonseed oil, Flaxseed oil, Grape seed oil, Mustard oil, Olive oil, Palm oil, Palm kernel oil, Peanut oil, Pine seed oil, Poppy seed oil, Pumpkin seed oil, Rice bran oil, Safflower oil, Tea oil, Truffle oil, Vegetable oil, Apricot (kernel) oil, Jojoba oil (simmondsia chinensis seed oil), Grapeseed oil, Macadamia oil, Wheat germ oil, Almond oil, Rapeseed oil, Gourd oil, Soybean oil, Sesame oil, Hazelnut oil, Maize oil, Sunflower oil, Hemp oil, Bois oil, Kuki nut oil, Avocado oil, Walnut oil, Fish oil, berry oil, allspice oil, juniper oil, seed oil, almond seed oil, anise seed oil, celery seed oil, cumin seed oil, nutmeg seed oil, leaf oil, basil leaf oil, bay leaf oil, cinnamon leaf oil, common sage leaf oil, eucalyptus leaf oil, lemon grass leaf oil, melaleuca leaf oil, oregano leaf oil, patchouli leaf oil, peppermint leaf oil, pine needle oil, rosemary leaf oil, spearmint leaf oil, tea tree leaf oil, thyme leaf oil, wintergreen leaf oil, flower oil, chamomile oil, clary sage oil, clove oil, geranium flower oil, hyssop flower oil, jasmine flower oil, lavender flower oil, manuka flower oil, Marhoram flower oil, orange flower oil, rose flower oil, ylang-ylang flower oil, Bark oil, cassia Bark oil, cinnamon bark oil, sassafras Bark oil, Wood oil, camphor wood oil, cedar wood oil, rosewood oil, sandalwood oil), rhizome (ginger) wood oil, resin oil, frankincense oil, myrrh oil, peel oil, bergamot peel oil, grapefruit peel oil, lemon peel oil, lime peel oil, orange peel oil, tangerine peel oil, root oil, valerian oil, Oleic acid, Linoleic acid, Oleyl alcohol, Isostearyl alcohol, semi-synthetic derivatives thereof, and any combinations thereof.

The oil may further comprise a silicone component, such as a volatile silicone component, which can be the sole oil in the silicone component or can be combined with other silicone and non-silicone, volatile and non-volatile oils. Suitable silicone components include, but are not limited to, methylphenylpolysiloxane, simethicone, dimethicone, phenyltrimethicone (or an organomodified version thereof), alkylated derivatives of polymeric silicones, cetyl dimethicone, lauryl trimethicone, hydroxylated derivatives of polymeric silicones, such as dimethiconol, volatile silicone oils, cyclic and linear silicones, cyclomethicone, derivatives of cyclomethicone, hexamethylcyclotrisiloxane, octamethylcyclotetrasiloxane, decamethylcyclopentasiloxane, volatile linear dimethylpolysiloxanes, isohexadecane, isoeicosane, isotetracosane, polyisobutene, isooctane, isododecane, semi-synthetic derivatives thereof, and combinations thereof.

The volatile oil can be the organic solvent, or the volatile oil can be present in addition to an organic solvent. Suitable volatile oils include, but are not limited to, a terpene, monoterpene, sesquiterpene, carminative, azulene, menthol, camphor, thujone, thymol, nerol, linalool, limonene, geraniol, perillyl alcohol, nerolidol, farnesol, ylangene, bisabolol, farnesene, ascaridole, chenopodium oil, citronellal, citral, citronellol, chamazulene, yarrow, guaiazulene, chamomile, semi-synthetic derivatives, or combinations thereof.

The volatile oil in the silicone component may be different than the oil in the oil phase.

The oil phase may comprise 3-15, and preferably 5-10 vol. % of an organic solvent, based on the total volume of the emulsion. It is contemplated that the organic phosphate-based solvents employed in the emulsions serve to remove or disrupt the lipids in the membranes of the pathogens. Thus, any solvent that removes the sterols or phospholipids in the microbial membranes may find use. Suitable organic solvents include, but are not limited to, organic phosphate based solvents or alcohols. Non-toxic alcohols (e.g., ethanol) may be used as a solvent. The oil phase, and any additional compounds provided in the oil phase, are preferably sterile and pyrogen free.

### 3. Surfactants and Detergents

The emulsions may further comprise a surfactant or detergent as defined in claim 7. The emulsion may comprise from about 3 to 15 %, and preferably about 10 % of one or more surfactants or detergents (although other concentrations are also contemplated). It is contemplated that surfactants, when present in the emulsions, help to stabilize the emulsions. Both non-ionic (non-anionic) and ionic surfactants are contemplated. Additionally, surfactants from the BRIJ family of surfactants may find use in the claimed compositions of the present invention. The surfactant can be provided in either the aqueous or the oil phase. Surfactants suitable for use with the emulsions include a variety of anionic and nonionic surfactants, as well as other emulsifying compounds that are capable of promoting the formation of oil-in-water emulsions. In general, emulsifying compounds are relatively hydrophilic, and blends of emulsifying compounds can be used to achieve the necessary qualities. In some formulations, nonionic surfactants have advantages over ionic emulsifiers in that they are substantially more compatible with a broad pH range and often form more stable emulsions than do ionic (*e.g.*, soap-type) emulsifiers.

The surfactant which may find use in the nanoemulsion vaccine of the invention as defined in claim 12 can be a pharmaceutically acceptable ionic surfactant, a pharmaceutically acceptable nonionic surfactant, a pharmaceutically acceptable cationic surfactant, a pharmaceutically acceptable anionic surfactant, or a pharmaceutically acceptable zwitterionic surfactant.

Exemplary useful surfactants are described in Applied Surfactants: Principles and Applications. Tharwat F. Tadros, Copyright 8 2005 WILEY-VCH Verlag GmbH & Co. KGaA, Weinheim ISBN: 3-527-30629-3).
Further, the surfactant can be a pharmaceutically acceptable ionic polymeric surfactant, a pharmaceutically acceptable nonionic polymeric surfactant, a pharmaceutically acceptable cationic polymeric surfactant, a pharmaceutically acceptable anionic polymeric surfactant, or a pharmaceutically acceptable zwitterionic polymeric surfactant. Examples of polymeric surfactants include, but are not limited to, a graft copolymer of a poly(methyl methacrylate) backbone with multiple (at least one) polyethylene oxide (PEO) side chain, polyhydroxystearic acid, an alkoxylated alkyl phenol formaldehyde condensate, a polyalkylene glycol modified polyester with fatty acid hydrophobes, a polyester, semi-synthetic derivatives thereof, or combinations thereof.

Surface active agents or surfactants, are amphipathic molecules that consist of a nonpolar hydrophobic portion, usually a straight or branched hydrocarbon or fluorocarbon chain containing 8-18 carbon atoms, attached to a polar or ionic hydrophilic portion. The hydrophilic portion can be nonionic, ionic or zwitterionic. The hydrocarbon chain interacts weakly with the water molecules in an aqueous environment, whereas the polar or ionic head group interacts strongly with water molecules via dipole or ion-dipole interactions. Based on the nature of the hydrophilic group, surfactants are classified into anionic, cationic, zwitterionic, nonionic and polymeric surfactants.

Suitable surfactants include, but are not limited to, ethoxylated nonylphenol comprising 9 to 10 units of ethyleneglycol, ethoxylated undecanol comprising 8 units of ethyleneglycol, polyoxyethylene (20) sorbitan monolaurate, polyoxyethylene (20) sorbitan monopalmitate, polyoxyethylene (20) sorbitan monostearate, polyoxyethylene (20) sorbitan monooleate, sorbitan monolaurate, sorbitan monopalmitate, sorbitan monostearate, sorbitan monooleate, ethoxylated hydrogenated ricin oils, sodium laurylsulfate, a diblock copolymer of ethyleneoxyde and propyleneoxyde, Ethylene Oxide-Propylene Oxide Block Copolymers, and tetra-functional block copolymers based on ethylene oxide and propylene oxide, Glyceryl monoesters, Glyceryl caprate, Glyceryl caprylate, Glyceryl cocate, Glyceryl erucate, Glyceryl hydroxysterate, Glyceryl isostearate, Glyceryl lanolate, Glyceryl laurate, Glyceryl linolate, Glyceryl myristate, Glyceryl oleate, Glyceryl PABA, Glyceryl palmitate, Glyceryl ricinoleate, Glyceryl stearate, Glyceryl thiglycolate, Glyceryl dilaurate, Glyceryl dioleate, Glyceryl dimyristate, Glyceryl disterate, Glyceryl sesuioleate, Glyceryl stearate lactate, Polyoxyethylene cetyl/stearyl ether, Polyoxyethylene cholesterol ether, Polyoxyethylene laurate or dilaurate, Polyoxyethylene stearate or distearate, polyoxyethylene fatty ethers, Polyoxyethylene lauryl ether, Polyoxyethylene stearyl ether, polyoxyethylene myristyl ether, a steroid, Cholesterol, Betasitosterol, Bisabolol, fatty acid esters of alcohols, isopropyl myristate, Aliphati-isopropyl n-butyrate, Isopropyl n-hexanoate, Isopropyl n-decanoate, Isoproppyl palmitate, Octyldodecyl myristate, alkoxylated alcohols, alkoxylated acids, alkoxylated amides, alkoxylated sugar derivatives, alkoxylated derivatives of natural oils and waxes, polyoxyethylene polyoxypropylene block copolymers, nonoxynol-14, PEG-8 laurate, PEG-6 Cocoamide, PEG-20 methylglucose sesquistearate, PEG40 lanolin, PEG-40 castor oil, PEG-40 hydrogenated castor oil, polyoxyethylene fatty ethers, glyceryl diesters, polyoxyethylene stearyl ether, polyoxyethylene myristyl ether, and polyoxyethylene lauryl ether, glyceryl dilaurate, glyceryl dimystate, glyceryl distearate, semi-synthetic derivatives thereof, or mixtures thereof.
Additional suitable surfactants include, but are not limited to, non-ionic lipids, such as glyceryl laurate, glyceryl myristate, glyceryl dilaurate, glyceryl dimyristate, semi-synthetic derivatives thereof, and mixtures thereof.

The surfactant may be a polyoxyethylene fatty ether having a polyoxyethylene head group ranging from about 2 to about 100 groups, or an alkoxylated alcohol having the structure R₅ --(OCH₂ CH₂)_{y} -OH, wherein R₅ is a branched or unbranched alkyl group having from about 6 to about 22 carbon atoms and y is between about 4 and about 100, and preferably, between about 10 and about 100. Preferably, the alkoxylated alcohol is the species wherein R₅ is a lauryl group and y has an average value of 23. The surfactant may be an alkoxylated alcohol which is an ethoxylated derivative of lanolin alcohol. Preferably, the ethoxylated derivative of lanolin alcohol is laneth-10, which is the polyethylene glycol ether of lanolin alcohol with an average ethoxylation value of 10.

Nonionic surfactants include, but are not limited to, an ethoxylated surfactant, an alcohol ethoxylated, an alkyl phenol ethoxylated, a fatty acid ethoxylated, a monoalkaolamide ethoxylated, a sorbitan ester ethoxylated, a fatty amino ethoxylated, an ethylene oxide-propylene oxide copolymer, Bis (polyethylene glycol bis[imidazoyl carbonyl]), nonoxynol-9, Bis(polyethylene glycol bis[imidazoyl carbonyl]), Brij^{®} 35, Brij^{®} 56, Brij^{®} 72, Brij^{®} 76, Brij^{®} 92V, Brij^{®} 97, Brij^{®} 58P, Cremophor^{®} EL, Decaethylene glycol monododecyl ether, N-Decanoyl-N-methylglucamine, n-Decyl alpha-D-glucopyranoside, Decyl beta-D-maltopyranoside, n-Dodecanoyl-N-methylglucamide, n-Dodecyl alpha-D-maltoside, n-Dodecyl beta-D-maltoside, n-Dodecyl beta-D-maltoside, Heptaethylene glycol monodecyl ether, Heptaethylene glycol monododecyl ether, Heptaethylene glycol monotetradecyl ether, n-Hexadecyl beta-D-maltoside, Hexaethylene glycol monododecyl ether, Hexaethylene glycol monohexadecyl ether, Hexaethylene glycol monooctadecyl ether, Hexaethylene glycol monotetradecyl ether, Igepal CA-630, Igepal CA-630, Methyl-6-O-(N-heptylcarbamoyl)-alpha-D-glucopyranoside, Nonaethylene glycol monododecyl ether, N-Nonanoyl-N-methylglucamine, N-Nonanoyl-N-methylglucamine, Octaethylene glycol monodecyl ether, Octaethylene glycol monododecyl ether, Octaethylene glycol monohexadecyl ether, Octaethylene glycol monooctadecyl ether, Octaethylene glycol monotetradecyl ether, Octyl-beta-D-glucopyranoside, Pentaethylene glycol monodecyl ether, Pentaethylene glycol monododecyl ether, Pentaethylene glycol monohexadecyl ether, Pentaethylene glycol monohexyl ether, Pentaethylene glycol monooctadecyl ether, Pentaethylene glycol monooctyl ether, Polyethylene glycol diglycidyl ether, Polyethylene glycol ether W-1, Polyoxyethylene 10 tridecyl ether, Polyoxyethylene 100 stearate, Polyoxyethylene 20 isohexadecyl ether, Polyoxyethylene 20 oleyl ether, Polyoxyethylene 40 stearate, Polyoxyethylene 50 stearate, Polyoxyethylene 8 stearate, Polyoxyethylene bis(imidazolyl carbonyl), Polyoxyethylene 25 propylene glycol stearate, Saponin from Quillaja bark, Span^{®} 20, Span^{®} 40, Span^{®} 60, Span^{®} 65, Span^{®} 80, Span^{®} 85, Tergitol, Type 15-S-12, Tergitol, Type 15-S-30, Tergitol, Type 15-S-5, Tergitol, Type 15-S-7, Tergitol, Type 15-S-9, Tergitol, Type NP-10, Tergitol, Type NP-4, Tergitol, Type NP-40, Tergitol, Type NP-7, Tergitol, Type NP-9, Tergitol, Tergitol, Type TMN-10, Tergitol, Type TMN-6, Tetradecyl-beta-D-maltoside, Tetraethylene glycol monodecyl ether, Tetraethylene glycol monododecyl ether, Tetraethylene glycol monotetradecyl ether, Triethylene glycol monodecyl ether, Triethylene glycol monododecyl ether, Triethylene glycol monohexadecyl ether, Triethylene glycol monooctyl ether, Triethylene glycol monotetradecyl ether, Triton CF-21, Triton CF-32, Triton DF-12, Triton DF-16, Triton GR-5M, Triton QS-15, Triton QS-44, Triton X-100, Triton X-102, Triton X-15, Triton X-151, Triton X-200, Triton X-207, Triton^{©} X-100, Triton^{®} X-114, Triton^{®} X-165, Triton^{®} X-305, Triton^{®} X-405, Triton^{®} X-45, Triton^{®} X-705-70, TWEEN^{®} 20, TWEEN^{®} 21, TWEEN^{®} 40, TWEEN^{®} 60, TWEEN^{®} 61, TWEEN^{®} 65, TWEEN^{®} 80, TWEEN^{®} 81, TWEEN^{®} 85, Tyloxapol, n-Undecyl beta-D-glucopyranoside, semi-synthetic derivatives thereof, or combinations thereof.

In addition, the nonionic surfactant can be a poloxamer. Poloxamers are polymers made of a block of polyoxyethylene, followed by a block of polyoxypropylene, followed by a block of polyoxyethylene. The average number of units of polyoxyethylene and polyoxypropylene varies based on the number associated with the polymer. For example, the smallest polymer, Poloxamer 101, consists of a block with an average of 2 units of polyoxyethylene, a block with an average of 16 units of polyoxypropylene, followed by a block with an average of 2 units of polyoxyethylene. Poloxamers range from colorless liquids and pastes to white solids. In cosmetics and personal care products, Poloxamers are used in the formulation of skin cleansers, bath products, shampoos, hair conditioners, mouthwashes, eye makeup remover and other skin and hair products. Examples of Poloxamers include, but are not limited to, Poloxamer 101, Poloxamer 105, Poloxamer 108, Poloxamer 122, Poloxamer 123, Poloxamer 124, Poloxamer 181, Poloxamer 182, Poloxamer 183, Poloxamer 184, Poloxamer 185, Poloxamer 188, Poloxamer 212, Poloxamer 215, Poloxamer 217, Poloxamer 231, Poloxamer 234, Poloxamer 235, Poloxamer 237, Poloxamer 238, Poloxamer 282, Poloxamer 284, Poloxamer 288, Poloxamer 331, Poloxamer 333, Poloxamer 334, Poloxamer 335, Poloxamer 338, Poloxamer 401, Poloxamer 402, Poloxamer 403, Poloxamer 407, Poloxamer 105 Benzoate, and Poloxamer 182 Dibenzoate.

Suitable cationic surfactants include, but are not limited to, a quarternary ammonium compound, an alkyl trimethyl ammonium chloride compound, a dialkyl dimethyl ammonium chloride compound, a cationic halogen-containing compound, such as cetylpyridinium chloride, Benzalkonium chloride, Benzalkonium chloride, Benzyldimethylhexadecylammonium chloride, Benzyldimethyltetradecylammonium chloride, Benzyldodecyldimethylammonium bromide, Benzyltrimethylammonium tetrachloroiodate, Dimethyldioctadecylammonium bromide, Dodecylethyldimethylammonium bromide, Dodecyltrimethylammonium bromide, Dodecyltrimethylammonium bromide, Ethylhexadecyldimethylammonium bromide, Girard's reagent T, Hexadecyltrimethylammonium bromide, Hexadecyltrimethylammonium bromide, N,N',N'-Polyoxyethylene(10)-N-tallow-1,3-diaminopropane, Thonzonium bromide, Trimethyl(tetradecyl)ammonium bromide, 1,3,5-Triazine-1,3,5(2H,4H,6H)-triethanol, 1-Decanaminium, N-decyl-N, N-dimethyl-, chloride, Didecyl dimethyl ammonium chloride, 2-(2-(p-(Diisobutyl)cresosxy)ethoxy)ethyl dimethyl benzyl ammonium chloride, 2-(2-(p-(Diisobutyl)phenoxy)ethoxy)ethyl dimethyl benzyl ammonium chloride, Alkyl 1 or 3 benzyl-1-(2-hydroxethyl)-2-imidazolinium chloride, Alkyl bis(2-hydroxyethyl) benzyl ammonium chloride, Alkyl demethyl benzyl ammonium chloride, Alkyl dimethyl 3,4-dichlorobenzyl ammonium chloride (100% C12), Alkyl dimethyl 3,4-dichlorobenzyl ammonium chloride (50% C14, 40% C12, 10% C16), Alkyl dimethyl 3,4-dichlorobenzyl ammonium chloride (55% C14, 23% C12, 20% C16), Alkyl dimethyl benzyl ammonium chloride, Alkyl dimethyl benzyl ammonium chloride (100% C14), Alkyl dimethyl benzyl ammonium chloride (100% C16), Alkyl dimethyl benzyl ammonium chloride (41% C14, 28% C12), Alkyl dimethyl benzyl ammonium chloride (47% C12, 18% C14), Alkyl dimethyl benzyl ammonium chloride (55% C16, 20% C14), Alkyl dimethyl benzyl ammonium chloride (58% C14, 28% C16), Alkyl dimethyl benzyl ammonium chloride (60% C14, 25% C12), Alkyl dimethyl benzyl ammonium chloride (61% C11, 23% C14), Alkyl dimethyl benzyl ammonium chloride (61 % C12, 23% C14), Alkyl dimethyl benzyl ammonium chloride (65% C12, 25% C14), Alkyl dimethyl benzyl ammonium chloride (67% C12, 24% C14), Alkyl dimethyl benzyl ammonium chloride (67% C12, 25% C14), Alkyl dimethyl benzyl ammonium chloride (90% C14, 5% C12), Alkyl dimethyl benzyl ammonium chloride (93% C14, 4% C12), Alkyl dimethyl benzyl ammonium chloride (95% C16, 5% C18), Alkyl dimethyl benzyl ammonium chloride, Alkyl didecyl dimethyl ammonium chloride, Alkyl dimethyl benzyl ammonium chloride, Alkyl dimethyl benzyl ammonium chloride (C12-16), Alkyl dimethyl benzyl ammonium chloride (C12-18), Alkyl dimethyl benzyl ammonium chloride, dialkyl dimethyl benzyl ammonium chloride, Alkyl dimethyl dimethybenzyl ammonium chloride, Alkyl dimethyl ethyl ammonium bromide (90% C14, 5% C16, 5% C12), Alkyl dimethyl ethyl ammonium bromide (mixed alkyl and alkenyl groups as in the fatty acids of soybean oil), Alkyl dimethyl ethylbenzyl ammonium chloride, Alkyl dimethyl ethylbenzyl ammonium chloride (60% C14), Alkyl dimethyl isopropylbenzyl ammonium chloride (50% C12, 30% C14, 17% C16, 3% C18), Alkyl trimethyl ammonium chloride (58% C18, 40% C16, 1% C14, 1% C12), Alkyl trimethyl ammonium chloride (90% C18, 10% C16), Alkyldimethyl(ethylbenzyl) ammonium chloride (C12-18), Di-(C8-10)-alkyl dimethyl ammonium chlorides, Dialkyl dimethyl ammonium chloride, Dialkyl methyl benzyl ammonium chloride, Didecyl dimethyl ammonium chloride, Diisodecyl dimethyl ammonium chloride, Dioctyl dimethyl ammonium chloride, Dodecyl bis (2-hydroxyethyl) octyl hydrogen ammonium chloride, Dodecyl dimethyl benzyl ammonium chloride, Dodecylcarbamoyl methyl dinethyl benzyl ammonium chloride, Heptadecyl hydroxyethylimidazolinium chloride, Hexahydro-1,3,5 - tris(2-hydroxyethyl)-s-triazine, Hexahydro-1,3,5-tris(2-hydroxyethyl)-s-triazine, Myristalkonium chloride (and) Quat RNIUM 14, N,N-Dimethyl-2-hydroxypropylammonium chloride polymer, n-Tetradecyl dimethyl benzyl ammonium chloride monohydrate, Octyl decyl dimethyl ammonium chloride, Octyl dodecyl dimethyl ammonium chloride, Octyphenoxyethoxyethyl dimethyl benzyl ammonium chloride, Oxydiethylenebis(alkyl dimethyl ammonium chloride), Quaternary ammonium compounds, dicoco alkyldimethyl, chloride, Trimethoxysily propyl dimethyl octadecyl ammonium chloride, Trimethoxysilyl quats, Trimethyl dodecylbenzyl ammonium chloride, semi-synthetic derivatives thereof, and combinations thereof.

Exemplary cationic halogen-containing compounds include, but are not limited to, cetylpyridinium halides, cetyltrimethylammonium halides, cetyldimethylethylammonium halides, cetyldimethylbenzylammonium halides, cetyltributylphosphonium halides, dodecyltrimethylammonium halides, or tetradecyltrimethylammonium halides. In some particular embodiments, suitable cationic halogen containing compounds comprise, but are not limited to, cetylpyridinium chloride (CPC), cetyltrimethylammonium chloride, cetylbenzyldimethylammonium chloride, cetylpyridinium bromide (CPB), cetyltrimethylammonium bromide (CTAB), cetyidimethylethylammonium bromide, cetyltributylphosphonium bromide, dodecyltrimethylammonium bromide, and tetrad ecyltrimethylammonium bromide. The cationic halogen containing compound may be CPC.

Suitable anionic surfactants include, but are not limited to, a carboxylate, a sulphate, a sulphonate, a phosphate, chenodeoxycholic acid, chenodeoxycholic acid sodium salt, cholic acid, ox or sheep bile, Dehydrocholic acid, Deoxycholic acid, Deoxycholic acid, Deoxycholic acid methyl ester, Digitonin, Digitoxigenin, N,N-Dimethyldodecylamine N-oxide, Docusate sodium salt, Glycochenodeoxycholic acid sodium salt, Glycocholic acid hydrate, synthetic, Glycocholic acid sodium salt hydrate, synthetic, Glycodeoxycholic acid monohydrate, Glycodeoxycholic acid sodium salt, Glycodeoxycholic acid sodium salt, Glycolithocholic acid 3-sulfate disodium salt, Glycolithocholic acid ethyl ester, N-Lauroylsarcosine sodium salt, N-Lauroylsarcosine solution, N-Lauroylsarcosine solution, Lithium dodecyl sulfate, Lithium dodecyl sulfate, Lithium dodecyl sulfate, Lugol solution, Niaproof 4, Type 4, 1-Octanesulfonic acid sodium salt, Sodium 1-butanesulfonate, Sodium 1-decanesulfonate, Sodium 1-decanesulfonate, Sodium 1-dodecanesulfonate, Sodium 1-heptanesulfonate anhydrous, Sodium 1-heptanesulfonate anhydrous, Sodium 1-nonanesulfonate, Sodium 1-propanesulfonate monohydrate, Sodium 2-bromoethanesulfonate, Sodium cholate hydrate, Sodium choleate, Sodium deoxycholate, Sodium deoxycholate monohydrate, Sodium dodecyl sulfate, Sodium hexanesulfonate anhydrous, Sodium octyl sulfate, Sodium pentanesulfonate anhydrous, Sodium taurocholate, Taurochenodeoxycholic acid sodium salt, Taurodeoxycholic acid sodium salt monohydrate, Taurohyodeoxycholic acid sodium salt hydrate, Taurolithocholic acid 3-sulfate disodium salt, Tauroursodeoxycholic acid sodium salt, Trizma^{®} dodecyl sulfate, TWEEN^{®} 80, Ursodeoxycholic acid, semi-synthetic derivatives thereof, and combinations thereof.

Suitable zwitterionic surfactants include, but are not limited to, an N-alkyl betaine, lauryl amindo propyl dimethyl betaine, an alkyl dimethyl glycinate, an N-alkyl amino propionate, CHAPS, minimum 98% (TLC), CHAPS, SigmaUltra, minimum 98% (TLC), CHAPS, for electrophoresis, minimum 98% (TLC), CHAPSO, minimum 98%, CHAPSO, SigmaUltra, CHAPSO, for electrophoresis, 3-(Decyldimethylammonio)propanesulfonate inner salt, 3-Dodecyldimethylammonio)propanesulfonate inner salt, SigmaUltra, 3-(Dodecyldimethylammonio)propanesulfonate inner salt, 3-(N,N-Dimethylmyristylammonio)propanesulfonate, 3-(N,N-Dimethyloctadecylammonio)propanesulfonate, 3-(N,N-Dimethyloctylammonio)propanesulfonate inner salt, 3-(N,N-Dimethylpalmitylammonio)propanesulfonate, semi-synthetic derivatives thereof, and combinations thereof.

Additional surfactants and detergents useful in the compositions of the present invention may be ascertained from reference works (*e.g.*, including, but not limited to, McCutheon's Volume 1: Emulsions and Detergents - North American Edition, 2000) and commercial sources.

### 4. Cationic Halogens Containg Compounds

The emulsions may further comprise a cationic halogen containing compound as claimed in claim 3. The emulsion may comprise from about 0.5 to 1.0 wt. % or more of a cationic halogen containing compound, based on the total weight of the emulsion (although other concentrations are also contemplated). The cationic halogen-containing compound is preferably premixed with the oil phase; however, it should be understood that the cationic halogen-containing compound may be provided in combination with the emulsion composition in a distinct formulation. Suitable halogen containing compounds may be selected from compounds comprising chloride, fluoride, bromide and iodide ions. Suitable cationic halogen containing compounds include, but are not limited to, cetylpyridinium halides, cetyltrimethylammonium halides, cetyldimethylethylammonium halides, cetyldimethylbenzylammonium halides, cetyltributylphosphonium halides, dodecyltrimethylammonium halides, or tetradecyltrimethylammonium halides. In some particular embodiments, suitable cationic halogen containing compounds comprise, but are not limited to, cetylpyridinium chloride (CPC), cetyltrimethylammonium chloride, cetylbenzyldimethylammonium chloride, cetylpyridinium bromide (CPB), and cetyltrimethylammonium bromide (CTAB), cetyidimethylethylammonium bromide, cetyltributylphosphonium bromide, dodecyltrimethylammonium bromide, and tetrad ecyltrimethylammonium bromide. The cationic halogen-containing compoundmay be CPC (see e.g. the nanoemulsions as defined in claim 9), although the compositions are not limited to formulation with any particular cationic containing compound.

### Formulation Techniques

Nanoemulsions can be formed using classic emulsion forming techniques. In brief, the oil phase is mixed with the aqueous phase under relatively high shear forces (e.g., using high hydraulic and mechanical forces) to obtain an oil-in-water nanoemulsion. The emulsion is formed by blending the oil phase with an aqueous phase on a volume-to-volume basis ranging from about 1:9 to 5:1, preferably about 5:1 to 3:1, most preferably 4:1, oil phase to aqueous phase. The oil and aqueous phases can be blended using any apparatus capable of producing shear forces sufficient to form an emulsion such as French Presses or high shear mixers (e.g., FDA approved high shear mixers are available, for example, from Admix, Inc., Manchester, NH). Methods of producing such emulsions are described in U.S. Pat. Nos. 5,103,497 and 4,895,452.

Compositions may comprise droplets of an oily discontinuous phase dispersed in an aqueous continuous phase, such as water. Nanoemulsions may be stable, and do not decompose even after long storage periods (e.g., greater than one or more years).
Furthermore, nanoemulsions may be stable (e.g., for greater than 3 months, for greater than 6 months, for greater than 12 months, or for greater than 18 months) after combination with an immunogen (e.g., a pathogen). Nanoemulsions may be non-toxic and safe when administered (e.g., via spraying or contacting mucosal surfaces, swallowed, inhaled, etc.) to a subject.

A portion of the emulsion may be in the form of lipid structures including, but not limited to, unilamellar, multilamellar, and paucliamellar lipid vesicles, micelles, and lamellar phases.

Some emulsions may employ an oil phase containing ethanol. For example, the emulsions may contain (i) an aqueous phase and (ii) an oil phase containing ethanol as the organic solvent and optionally a germination enhancer, and (iii) TYLOXAPOL as the surfactant (preferably 2-5%, more preferably 3%). This formulation is highly efficacious for inactivation of pathogens and is also non-irritating and non-toxic to mammalian subjects (e.g., and thus can be used for administration to a mucosal surface).

The emulsions may comprise a first emulsion emulsified within a second emulsion, wherein (a) the first emulsion comprises (i) an aqueous phase; and (ii) an oil phase comprising an oil and an organic solvent; and (iii) a surfactant; and (b) the second emulsion comprises (i) an aqueous phase; and (ii) an oil phase comprising an oil and a cationic containing compound; and (iii) a surfactant.

### Exemplary Formulations

The following description provides a number of exemplary emulsions including formulations for compositions BCTP and X₈W₆₀PC. BCTP comprises a water-in oil nanoemulsion, in which the oil phase was made from soybean oil, tri-n-butyl phosphate, and TRITON X-100 in 80% water. X₈W₆₀PC comprises a mixture of equal volumes of BCTP with W₈₀8P. W₈₀8P is a liposome-like compound made of glycerol monostearate, refined oya sterols (e.g., GENEROL sterols), TWEEN 60, soybean oil, a cationic ion halogen-containing CPC and peppermint oil. The GENEROL family are a group of a polyethoxylated soya sterols (Henkel Corporation, Ambler, Pennsylvania). Exemplary emulsion formulations are provided in Table 1B. These particular formulations may be found in U.S. Pat. Nos. 5,700,679 (NN); 5,618,840; 5,549,901 (W₈₀8P); and 5,547,677. Certain other emulsion formulations are presented U.S. Pat. App. Serial No. 10/669,865.

The X₈W₆₀PC emulsion is manufactured by first making the W₈₀8P emulsion and BCTP emulsions separately. A mixture of these two emulsions is then re-emulsified to produce a fresh emulsion composition termed X₈W₆₀PC. Methods of producing such emulsions are described in U.S. Pat. Nos. 5,103,497 and 4,895,452.

**Table 1B**

| | Oil Phase Formula | Water to Oil Phase Ratio (Vol/Vol) |
|---|---|---|
| BCTP | 1 vol. Tri(N-butyl)phosphate | 4:1 |
| | 1 vol. TRITON X-100 | |
| | 8 vol. Soybean oil | |
| NN | 86.5 g Glycerol monooleate | 3:1 |
| | 60.1 ml Nonoxynol-9 | |
| | 24.2 g GENEROL 122 | |
| | 3.27 g Cetylpyridinium chloride | |
| | 554 g Soybean oil | |
| W₈₀8P | 86.5 g Glycerol monooleate | 3.2:1 |
| | 21.2 g Polysorbate 60 | |
| | 24.2 g GENEROL 122 | |
| | 3.27 g Cetylpyddinium chloride | |
| | 4 ml Peppermint oil | |
| | 554 g Soybean oil | |
| SS | 86.5 g Glycerol monooleate | 3.2:1 |
| | 21.2 g Polysorbate 60 | (1% bismuth in water) |
| | 24.2 g GENEROL 122 | |
| | 3.27 g Cetylpyridinium chloride | |
| | 554 g Soybean oil | |

The compositions listed above are only exemplary and those of skill in the art will be able to alter the amounts of the components to arrive at a sitable nanoemulsion composition. Those skilled in the art will understand that the ratio of oil phase to water as well as the individual oil carrier, surfactant CPC and organic phosphate buffer, components of each composition may vary.

Although certain compositions comprising BCTP have a water to oil ratio of 4:1, it is understood that the BCTP may be formulated to have more or less of a water phase. For example, there may be 3, 4, 5, 6, 7, 8, 9, 10, or more parts of the water phase to each part of the oil phase. The same holds true for the W₈₀8P formulation. Similarly, the ratio of Tri(N-butyl)phosphate:TRITON X-100:soybean oil also may be varied.

Although Table 1B lists specific amounts of glycerol monooleate, polysorbate 60, GENEROL 122, cetylpyridinium chloride, and carrier oil for W₈₀8P, these are merely exemplary. An emulsion that has the properties of W₈₀8P may be formulated that has different concentrations of each of these components or indeed different components that will fulfill the same function. For example, the emulsion may have between about 80 to about 100g of glycerol monooleate in the initial oil phase. The emulsion may have between about 15 to about 30 g polysorbate 60 in the initial oil phase. The composition may comprise between about 20 to about 30 g of a GENEROL sterol, in the initial oil phase.

Individual components of nanoemulsions can function both to inactivate a pathogen as well as to contribute to the non-toxicity of the emulsions. For example, the active component in BCTP, TRITON-X100, shows less ability to inactivate a virus at concentrations equivalent to 11% BCTP. Adding the oil phase to the detergent and solvent markedly reduces the toxicity of these agents in tissue culture at the same concentrations. It is suggested that the nanoemulsion enhances the interaction of its components with the pathogens thereby facilitating the inactivation of the pathogen and reducing the toxicity of the individual components. Furthermore, when all the components of BCTP are combined in one composition but are not in a nanoemulsion structure, the mixture is not as effective at inactivating a pathogen as when the components are in a nanoemulsion structure.

Numerous additional compositions are presented below. The following compositions recite various ratios and mixtures of active components. One skilled in the art will appreciate that the below recited formulation are exemplary and that additional formulations comprising similar percent ranges of the recited components are possible.

A nanoemulsion may comprise from about 3 to 8 vol. % of TYLOXAPOL, about 8 vol. % of ethanol, about 1 vol. % of cetylpyridinium chloride (CPC), about 60 to 70 vol. % oil (e.g., soybean oil), about 15 to 25 vol. % of aqueous phase (e.g., DiH₂O or PBS), and in some formulations less than about 1 vol. % of 1N NaOH. Some of these formulations comprise PBS. It is contemplated that the addition of 1N NaOH and/or PBS in some of these formulations, allows the user to advantageously control the pH of the formulations, such that pH ranges from about 7.0 to about 9.0, and more preferably from about 7.1 to 8.5 are achieved. For example, one formulation comprises about 3 vol. % of TYLOXAPOL, about 8 vol. % of ethanol, about 1 vol. % of CPC, about 64 vol. % of soybean oil, and about 24 vol. % of DiH₂O (designated herein as Y3EC). Another similar formulation comprises about 3.5 vol. % of TYLOXAPOL, about 8 vol. % of ethanol, and about 1 vol. % of CPC, about 64 vol. % of soybean oil, and about 23.5 vol. % of DiH₂O (designated herein as Y3.5EC). Yet another formulation comprises about 3 vol. % of TYLOXAPOL, about 8 vol. % of ethanol, about 1 vol. % of CPC, about 0.067 vol. % of 1N NaOH, such that the pH of the formulation is about 7.1, about 64 vol. % of soybean oil, and about 23.93 vol. % of DiH₂O (designated herein as Y3EC pH 7.1). Still another formulation comprises about 3 vol. % of TYLOXAPOL, about 8 vol. % of ethanol, about 1 vol. % of CPC, about 0.67 vol. % of 1N NaOH, such that the pH of the formulation is about 8.5, and about 64 vol. % of soybean oil, and about 23.33 vol. % of DiH₂O (designated herein as Y3EC pH 8.5). Another similar formulation comprises about 4% TYLOXAPOL, about 8 vol. % ethanol, about 1% CPC, and about 64 vol. % of soybean oil, and about 23 vol. % of DiH₂O (designated herein as Y4EC). The formulation may comprise about 8% TYLOXAPOL, about 8% ethanol, about 1 vol. % of CPC, and about 64 vol. % of soybean oil, and about 19 vol. % of DiH₂O (designated herein as Y8EC). A further formulation comprises about 8 vol. % of TYLOXAPOL, about 8 vol. % of ethanol, about 1 vol. % of CPC, about 64 vol. % of soybean oil, and about 19 vol. % of 1x PBS (designated herein as Y8EC PBS).

A nanoemulsion may comprises about 8 vol. % of ethanol, and about 1 vol. % of CPC, and about 64 vol. % of oil (e.g., soybean oil), and about 27 vol. % of aqueous phase (e.g., DiH₂O or PBS) (designated herein as EC).

A nanoemulsion may comprise from about 8 vol. % of sodium dodecyl sulfate (SDS), about 8 vol. % of tributyl phosphate (TBP), and about 64 vol. % of oil (e.g., soybean oil), and about 20 vol. % of aqueous phase (e.g., DiH₂O or PBS) (designated herein as S8P).

A nanoemulsion may comprise from about 1 to 2 vol. % of TRITON X-100, from about 1 to 2 vol. % of TYLOXAPOL, from about 7 to 8 vol. % of ethanol, about 1 vol. % of cetylpyridinium chloride (CPC), about 64 to 57.6 vol. % of oil (e.g., soybean oil), and about 23 vol. % of aqueous phase (e.g., DiH₂O or PBS). Additionally, some of these formulations further comprise about 5 mM of L-alanine/Inosine, and about 10 mM ammonium chloride. Some of these formulations comprise PBS. It is contemplated that the addition of PBS in some of these formulations, allows the user to advantageously control the pH of the formulations. For example,the formulation may comprise about 2 vol. % of TRITON X-100, about 2 vol. % of TYLOXAPOL, about 8 vol. % of ethanol, about 1 vol. % CPC, about 64 vol. % of soybean oil, and about 23 vol. % of aqueous phase DiH₂O. The formulation comprises about 1.8 vol. % of TRITON X-100, about 1.8 vol. % of TYLOXAPOL, about 7.2 vol. % of ethanol, about 0.9 vol. % of CPC, about 5 mM L-alanine/Inosine, and about 10 mM ammonium chloride, about 57.6 vol. % of soybean oil, and the remainder of 1x PBS (designated herein as 90% X2Y2EC/GE).

In some embodiments, a nanoemulsion comprises about 5 vol. % of TWEEN 80, about 8 vol. % of ethanol, about 1 vol. % of CPC, about 64 vol. % of oil (e.g., soybean oil), and about 22 vol. % of DiH₂O (designated herein as W₈₀5EC) as defined in claim 9.

In still other embodiments of the present invention, a nanoemulsion comprises about 5 vol. % of TWEEN 20, about 8 vol. % of ethanol, about 1 vol. % of CPC, about 64 vol. % of oil (e.g., soybean oil), and about 22 vol. % of DiH₂O (designated herein as W₂₀5EC) as defined in claim 9.

A nanoemulsion may comprise from about 2 to 8 vol. % of TRITON X-100, about 8 vol. % of ethanol, about 1 vol. % of CPC, about 60 to 70 vol. % of oil (e.g., soybean, or olive oil), and about 15 to 25 vol. % of aqueous phase (e.g., DiH₂O or PBS). For example, formulations comprising about 2 vol. % of TRITON X-100, about 8 vol. % of ethanol, about 64 vol. % of soybean oil, and about 26 vol. % of DiH₂O (designated herein as X2E) are contemplated. A nanoemulsion comprising about 3 vol. % of TRITON X-100, about 8 vol. % of ethanol, about 64 vol. % of soybean oil, and about 25 vol. % of DiH₂O (designated herein as X3E) is contemplated. The formulations may comprise about 4 vol. % Triton of X-100, about 8 vol. % of ethanol, about 64 vol. % of soybean oil, and about 24 vol. % of DiH₂O (designated herein as X4E). A nanoemulsion may comprise about 5 vol. % of TRITON X-100, about 8 vol. % of ethanol, about 64 vol. % of soybean oil, and about 23 vol. % of DiH₂O (designated herein as X5E). A nanoemulsion may comprise about 6 vol. % of TRITON X-100, about 8 vol. % of ethanol, about 64 vol. % of soybean oil, and about 22 vol. % of DiH₂O (designated herein as X6E). A nanoemulsion may comprise about 8 vol. % of TRITON X-100, about 8 vol. % of ethanol, about 64 vol. % of soybean oil, and about 20 vol. % of DiH₂O (designated herein as X8E). A nanoemulsion may comprise about 8 vol. % of TRITON X-100, about 8 vol. % of ethanol, about 64 vol. % of olive oil, and about 20 vol. % of DiH₂O (designated herein as X8E O). A nanoemulsion may comprise 8 vol. % of TRITON X-100, about 8 vol. % ethanol, about 1 vol. % CPC, about 64 vol. % of soybean oil, and about 19 vol. % of DiH₂O (designated herein as X8EC).

A nanoemulsion may comprise from about 1 to 2 vol. % of TRITON X-100, from about 1 to 2 vol. % of TYLOXAPOL, from about 6 to 8 vol. % TBP, from about 0.5 to 1.0 vol. % of CPC, from about 60 to 70 vol. % of oil (e.g., soybean), and about 1 to 35 vol. % of aqueous phase (e.g., DiH₂O or PBS). Additionally, certain of these nanoemulsions may comprise from about 1 to 5 vol. % of trypticase soy broth, from about 0.5 to 1.5 vol. % of yeast extract, about 5 mM L-alanine/Inosine, about 10 mM ammonium chloride, and from about 20-40 vol. % of liquid baby formula. In some formulations comprising liquid baby formula, the formula comprises a casein hydrolysate (e.g., Neutramigen, or Progestimil, and the like). A nanoemulsion may further comprises from about 0.1 to 1.0 vol. % of sodium thiosulfate, and from about 0.1 to 1.0 vol. % of sodium citrate. Other similar formulations comprising these basic components employ phosphate buffered saline (PBS) as the aqueous phase. For example, one formulation comprises about 2 vol. % of TRITON X-100, about 2 vol. % TYLOXAPOL, about 8 vol. % TBP, about 1 vol. % of CPC, about 64 vol. % of soybean oil, and about 23 vol. % of DiH₂O (designated herein as X2Y2EC). The formulation may comprises about 2 vol. % of TRITON X-100, about 2 vol. % TYLOXAPOL, about 8 vol. % TBP, about 1 vol. % of CPC, about 0.9 vol. % of sodium thiosulfate, about 0.1 vol. % of sodium citrate, about 64 vol. % of soybean oil, and about 22 vol. % of DiH₂O (designated herein as X2Y2PC STS1). A nanoemulsion may comprise about 1.7 vol. % TRITON X-100, about 1.7 vol. % TYLOXAPOL, about 6.8 vol. % TBP, about 0.85% CPC, about 29.2% NEUTRAMIGEN, about 54.4 vol. % of soybean oil, and about 4.9 vol. % of DiH₂O (designated herein as 85% X2Y2PC/baby). A nanoemulsion may comprise about 1.8 vol. % of TRITON X-100, about 1.8 vol. % of TYLOXAPOL, about 7.2 vol. % of TBP, about 0.9 vol. % of CPC, about 5mM L-alanine/Inosine, about 10mM ammonium chloride, about 57.6 vol. % of soybean oil, and the remainder vol. % of 0.1x PBS (designated herein as 90% X2Y2 PC/GE). A nanoemulsion may comprise about 1.8 vol. % of TRITON X-100, about 1.8 vol. % of TYLOXAPOL, about 7.2 vol. % TBP, about 0.9 vol. % of CPC, and about 3 vol. % trypticase soy broth, about 57.6 vol. % of soybean oil, and about 27.7 vol. % of DiH₂O (designated herein as 90% X2Y2PC/TSB). A nanoemulsion may comprise about 1.8 vol. % TRITON X-100, about 1.8 vol. % TYLOXAPOL, about 7.2 vol. % TBP, about 0.9 vol. % CPC, about 1 vol. % yeast extract, about 57.6 vol. % of soybean oil, and about 29.7 vol. % of DiH₂O (designated herein as 90% X2Y2PC/YE).

A nanoemulsion may comprise about 3 vol. % of TYLOXAPOL, about 8 vol. % of TBP, and about 1 vol. % of CPC, about 60 to 70 vol. % of oil (e.g., soybean or olive oil), and about 15 to 30 vol. % of aqueous phase (e.g., DiH₂O or PBS). A nanoemulsion may comprises about 3 vol. % of TYLOXAPOL, about 8 vol. % of TBP, and about 1 vol. % of CPC, about 64 vol. % of soybean, and about 24 vol. % of DiH₂O (designated herein as Y3PC).

A nanoemulsion may comprise from about 4 to 8 vol. % of TRITON X-100, from about 5 to 8 vol. % of TBP, about 30 to 70 vol. % of oil (e.g., soybean or olive oil), and about 0 to 30 vol. % of aqueous phase (e.g., DiH₂O or PBS). Additionally, certain of these formulations may further comprise about 1 vol. % of CPC, about 1 vol. % of benzalkonium chloride, about 1 vol. % cetylyridinium bromide, about 1 vol. % cetyldimethyletylammonium bromide, 500 µM EDTA, about 10 mM ammonium chloride, about 5 mM Inosine, and about 5 mM L-alanine. For example, a nanoemulsion may comprise about 8 vol. % of TRITON X-100, about 8 vol. % of TBP, about 64 vol. % of soybean oil, and about 20 vol. % of DiH₂O (designated herein as X8P). A nanoemulsion may comprise about 8 vol. % of TRITON X-100, about 8 vol. % of TBP, about 1% of CPC, about 64 vol. % of soybean oil, and about 19 vol. % of DiH₂O (designated herein as X8PC). A nanoemulsion may comprise about 8 vol. % TRITON X-100, about 8 vol. % of TBP, about 1 vol. % of CPC, about 50 vol. % of soybean oil, and about 33 vol. % of DiH₂O (designated herein as ATB-X1001). The formulations may comprise about 8 vol. % of TRITON X-100, about 8 vol. % of TBP, about 2 vol. % of CPC, about 50 vol. % of soybean oil, and about 32 vol. % of DiH₂O (designated herein as ATB-X002). A nanoemulsion may comprise about 4 vol. % TRITON X-100, about 4 vol. % of TBP, about 0.5 vol. % of CPC, about 32 vol. % of soybean oil, and about 59.5 vol. % of DiH₂O (designated herein as 50% X8PC). A nanoemulsion may comprise about 8 vol. % of TRITON X-100, about 8 vol. % of TBP, about 0.5 vol. % CPC, about 64 vol. % of soybean oil, and about 19.5 vol. % of DiH₂O (designated herein as X8PC_{1/2}). A nanoemulsion may comprise about 8 vol. % of TRITON X-100, about 8 vol. % of TBP, about 2 vol. % of CPC, about 64 vol. % of soybean oil, and about 18 vol. % of DiH₂O (designated herein as X8PC2). A nanoemulsion may comprise about 8 vol. % of TRITON X-100, about 8% of TBP, about 1% of benzalkonium chloride, about 50 vol. % of soybean oil, and about 33 vol. % of DiH₂O (designated herein as X8P BC). A nanoemulsion may comprise about 8 vol. % of TRITON X-100, about 8 vol. % of TBP, about 1 vol. % of cetylyridinium bromide, about 50 vol. % of soybean oil, and about 33 vol. % of DiH₂O (designated herein as X8P CPB). A nanoemulsion may comprise about 8 vol. % of TRITON X-100, about 8 vol. % of TBP, about 1 vol. % of cetyldimethyletylammonium bromide, about 50 vol. % of soybean oil, and about 33 vol. % of DiH₂O (designated herein as X8P CTAB). A nanoemulsion may comprise about 8 vol. % of TRITON X-100, about 8 vol. % of TBP, about 1 vol. % of CPC, about 500 µM EDTA, about 64 vol. % of soybean oil, and about 15.8 vol. % DiH₂O (designated herein as X8PC EDTA). A nanoemulsion may comprise 8 vol. % of TRITON X-100, about 8 vol. % of TBP, about 1 vol. % of CPC, about 10 mM ammonium chloride, about 5mM Inosine, about 5mM L-alanine, about 64 vol. % of soybean oil, and about 19 vol. % of DiH₂O or PBS (designated herein as X8PC Get₁ₓ). A nanoemulsion may comprise about 5 vol. % of TRITON X-100, about 5% of TBP, about 1 vol. % of CPC, about 40 vol. % of soybean oil, and about 49 vol. % of DiH₂O (designated herein as X5P₅C).

A nanoemulsion may comprise about 2 vol. % TRITON X-100, about 6 vol. % TYLOXAPOL, about 8 vol. % ethanol, about 64 vol. % of soybean oil, and about 20 vol. % of DiH₂O (designated herein as X2Y6E).

A nanoemulsion may comprises about 8 vol. % of TRITON X-100, and about 8 vol. % of glycerol, about 60 to 70 vol. % of oil (e.g., soybean or olive oil), and about 15 to 25 vol. % of aqueous phase (e.g., DiH₂O or PBS). Certain nanoemulsion compositions (e.g., used to generate an immune response (e.g., for use as a vaccine) comprise about 1 vol. % L-ascorbic acid. For example, one particular formulation comprises about 8 vol. % of TRITON X-100, about 8 vol. % of glycerol, about 64 vol. % of soybean oil, and about 20 vol. % of DiH₂O (designated herein as X8G). A nanoemulsion may comprise about 8 vol. % of TRITON X-100, about 8 vol. % of glycerol, about 1 vol. % of L-ascorbic acid, about 64 vol. % of soybean oil, and about 19 vol. % of DiH₂O (designated herein as X8GV_{c}).

A nanoemulsion may comprise about 8 vol. % of TRITON X-100, from about 0.5 to 0.8 vol. % of TWEEN 60, from about 0.5 to 2.0 vol. % of CPC, about 8 vol. % of TBP, about 60 to 70 vol. % of oil (e.g., soybean or olive oil), and about 15 to 25 vol. % of aqueous phase (e.g., DiH₂O or PBS). For example, a nanoemulsion may comprise about 8 vol. % of TRITON X-100, about 0.70 vol. % of TWEEN 60, about 1 vol. % of CPC, about 8 vol. % of TBP, about 64 vol. % of soybean oil, and about 18.3 vol. % of DiH₂O (designated herein as X8W60PC₁). A nanoemulsion may comprise about 8 vol. % of TRITON X-100, about 0.71 vol. % of TWEEN 60, about 1 vol. % of CPC, about 8 vol. % of TBP, about 64 vol. % of soybean oil, and about 18.29 vol. % of DiH₂O (designated herein as W60_{0.7}X8PC). A nanoemulsion may comprise from about 8 vol. % of TRITON X-100, about 0.7 vol. % of TWEEN 60, about 0.5 vol. % of CPC, about 8 vol. % of TBP, about 64 to 70 vol. % of soybean oil, and about 18.8 vol. % of DiH₂O (designated herein as X8W60PC₂). A nanoemulsion may comprise about 8 vol. % of TRITON X-100, about 0.71 vol. % of TWEEN 60, about 2 vol. % of CPC, about 8 vol. % of TBP, about 64 vol. % of soybean oil, and about 17.3 vol. % of DiH₂O. A nanoemulsion may comprise about 0.71 vol. % of TWEEN 60, about 1 vol. % of CPC, about 8 vol. % of TBP, about 64 vol. % of soybean oil, and about 25.29 vol. % of DiH₂O (designated herein as W60_{0.7}PC).

A nanoemulsion may comprise about 2 vol. % of dioctyl sulfosuccinate, either about 8 vol. % of glycerol, or about 8 vol. % TBP, in addition to, about 60 to 70 vol. % of oil (e.g., soybean or olive oil), and about 20 to 30 vol. % of aqueous phase (e.g., DiH₂O or PBS). For example, a nanoemulsion may comprise about 2 vol. % of dioctyl sulfosuccinate, about 8 vol. % of glycerol, about 64 vol. % of soybean oil, and about 26 vol. % of DiH₂O (designated herein as D2G). A nanoemulsion may comprise about 2 vol. % of dioctyl sulfosuccinate, and about 8 vol. % of TBP, about 64 vol. % of soybean oil, and about 26 vol. % of DiH₂O (designated herein as D2P).

A nanoemulsion may comprise about 8 to 10 vol. % of glycerol, and about 1 to 10 vol. % of CPC, about 50 to 70 vol. % of oil (e.g., soybean or olive oil), and about 15 to 30 vol. % of aqueous phase (e.g., DiH₂O or PBS). Additionally, a nanoemulsion may further comprise about 1 vol. % of L-ascorbic acid. For example, a nanoemulsion may comprise about 8 vol. % of glycerol, about 1 vol. % of CPC, about 64 vol. % of soybean oil, and about 27 vol. % of DiH₂O (designated herein as GC). A nanoemulsion may comprise about 10 vol. % of glycerol, about 10 vol. % of CPC, about 60 vol. % of soybean oil, and about 20 vol. % of DiH₂O (designated herein as GC10). A nanoemulsion may comprise about 10 vol. % of glycerol, about 1 vol. % of CPC, about 1 vol. % of L-ascorbic acid, about 64 vol. % of soybean or oil, and about 24 vol. % of DiH₂O (designated herein as GCV_{c}).

A nanoemulsion may comprise about 8 to 10 vol. % of glycerol, about 8 to 10 vol. % of SDS, about 50 to 70 vol. % of oil (e.g., soybean or olive oil), and about 15 to 30 vol. % of aqueous phase (e.g., DiH₂O or PBS). Additionally,a nanoemulsionmay further comprise about 1 vol. % of lecithin, and about 1 vol. % of p-Hydroxybenzoic acid methyl ester. Exemplary formulations comprise about 8 vol. % SDS, 8 vol. % of glycerol, about 64 vol. % of soybean oil, and about 20 vol. % of DiH₂O (designated herein as S8G). A related formulation comprises about 8 vol. % of glycerol, about 8 vol. % of SDS, about 1 vol. % of lecithin, about 1 vol. % of p-Hydroxybenzoic acid methyl ester, about 64 vol. % of soybean oil, and about 18 vol. % of DiH₂O (designated herein as S8GL1B1).

A nanoemulsion may comprise about 4 vol. % of TWEEN 80, about 4 vol. % of TYLOXAPOL, about 1 vol. % of CPC, about 8 vol. % of ethanol, about 64 vol. % of soybean oil, and about 19 vol. % of DiH₂O (designated herein as W₈₀4Y4EC).

A nanoemulsion may comprise about 0.01 vol. % of CPC, about 0.08 vol. % of TYLOXAPOL, about 10 vol. % of ethanol, about 70 vol. % of soybean oil, and about 19.91 vol. % of DiH₂O (designated herein as Y.08EC.01).

A nanoemulsion may comprise about 8 vol. % of sodium lauryl sulfate, and about 8 vol. % of glycerol, about 64 vol. % of soybean oil, and about 20 vol. % of DiH₂O (designated herein as SLS8G).

The specific formulations described above are simply examples to illustrate the variety of nanoemulsions that find use (e.g., to inactivate and/or neutralize a pathogen, and for generating an immune response in a subject (e.g., for use as a vaccine)). Many variations of the above formulations, as well as additional nanoemulsions are contemplated. Candidate emulsions can be easily tested to determine if they are suitable. First, the desired ingredients are prepared using the methods described herein, to determine if an emulsion can be formed. If an emulsion cannot be formed, the candidate is rejected. For example, a candidate composition made of 4.5% sodium thiosulfate, 0.5% sodium citrate, 10% n-butanol, 64% soybean oil, and 21% DiH₂O does not form an emulsion.

Second, the candidate emulsion should form a stable emulsion. An emulsion is stable if it remains in emulsion form for a sufficient period to allow its intended use (e.g., to generate an immune response in a subject). For example, for emulsions that are to be stored, shipped, etc., it may be desired that the composition remain in emulsion form for months to years. Typical emulsions that are relatively unstable, will lose their form within a day. For example, a candidate composition made of 8% 1-butanol, 5% Tween 10, 1% CPC, 64% soybean oil, and 22% DiH₂O does not form a stable emulsion. Nanoemulsions that have been shown to be stable include, but are not limited to, 8 vol. % of TRITON X-100, about 8 vol. % of TBP, about 64 vol. % of soybean oil, and about 20 vol. % of DiH₂O (designated herein as X8P); 5 vol. % of TWEEN 20, from about 8 vol. % of ethanol, from about 1 vol. % of CPC, about 64 vol. % of oil (e.g., soybean oil), and about 22 vol. % of DiH₂O (designated herein as W₂₀5EC); 0.08% Triton X-100, 0.08% Glycerol, 0.01% Cetylpyridinium Chloride, 99% Butter, and 0.83% DiH₂O (designated herein as 1% X8GC Butter); 0.8% Triton X-100, 0.8% Glycerol, 0.1% Cetylpyridinium Chloride, 6.4% Soybean Oil, 1.9% DiH₂O, and 90% Butter (designated herein as 10% X8GC Butter); 2% W₂₀5EC, 1% Natrosol 250L NF, and 97% DiH₂O (designated herein as 2% W₂₀5EC L GEL); 1% Cetylpyridinium Chloride, 5% Tween 20, 8% Ethanol, 64% 70 Viscosity Mineral Oil, and 22% DiH₂O (designated herein as W₂₀5EC 70 Mineral Oil); 1% Cetylpyridinium Chloride, 5% Tween 20, 8% Ethanol, 64% 350 Viscosity Mineral Oil, and 22% DiH₂O (designated herein as W₂₀5EC 350 Mineral Oil). Preferably said nanoemulsions are stable for over a week, over a month, or over a year.

Third, the candidate emulsion should have efficacy for its intended use. For example, a nanoemuslion should inactivate (e.g., kill or inhibit growth of) a pathogen to a desired level (e.g., 1 log, 2 log, 3 log, 4 log, ... reduction). Using the methods described herein, one is capable of determining the suitability of a particular candidate emulsion against the desired pathogen. Generally, this involves exposing the pathogen to the emulsion for one or more time periods in a side-by-side experiment with the appropriate control samples (e.g., a negative control such as water) and determining if, and to what degree, the emulsion inactivates (e.g., kills and/or neutralizes) the microorganism. For example, a candidate composition made of 1% ammonium chloride, 5% Tween 20, 8% ethanol, 64% soybean oil, and 22% DiH₂O was shown not to be an effective emulsion. The following candidate emulsions were shown to be effective using the methods described herein: 5% Tween 20, 5% Cetylpyridinium Chloride, 10% Glycerol, 60% Soybean Oil, and 20% DiH₂O (designated herein as W₂₀5GC5); 1% Cetylpyridinium Chloride, 5% Tween 20, 10% Glycerol, 64% Soybean Oil, and 20% DiH₂O (designated herein as W₂₀5GC); 1% Cetylpyridinium Chloride, 5% Tween 20, 8% Ethanol, 64% Olive Oil, and 22% DiH₂O (designated herein as W₂₀5EC Olive Oil); 1% Cetylpyridinium Chloride, 5% Tween 20, 8% Ethanol, 64% Flaxseed Oil, and 22% DiH₂O (designated herein as W₂₀5EC Flaxseed Oil); 1% Cetylpyridinium Chloride, 5% Tween 20, 8% Ethanol, 64% Corn Oil, and 22% DiH₂O (designated herein as W₂₀5EC Corn Oil); 1% Cetylpyridinium Chloride, 5% Tween 20, 8% Ethanol, 64% Coconut Oil, and 22% DiH₂O (designated herein as W₂₀5EC Coconut Oil); 1% Cetylpyridinium Chloride, 5% Tween 20, 8% Ethanol, 64% Cottonseed Oil, and 22% DiH₂O (designated herein as W₂₀5EC Cottonseed Oil); 8% Dextrose, 5% Tween 10, 1% Cetylpyridinium Chloride, 64% Soybean Oil, and 22% DiH₂O (designated herein as W₂₀5C Dextrose); 8% PEG 200, 5% Tween 10, 1% Cetylpyridinium Chloride, 64% Soybean Oil, and 22% DiH₂O (designated herein as W₂₀5C PEG 200); 8% Methanol, 5% Tween 10, 1% Cetylpyridinium Chloride, 64% Soybean Oil, and 22% DiH₂O (designated herein as W₂₀5C Methanol); 8% PEG 1000, 5% Tween 10, 1% Cetylpyridinium Chloride, 64% Soybean Oil, and 22% DiH₂O (designated herein as W₂₀5C PEG 1000); 2% W₂₀5EC, 2% Natrosol 250H NF, and 96% DiH₂O (designated herein as 2% W₂₀5EC Natrosol 2, also called 2% W₂₀5EC GEL); 2% W₂₀5EC, 1% Natrosol 250H NF, and 97% DiH₂O (designated herein as 2% W₂₀5EC Natrosol 1); 2% W₂₀5EC, 3% Natrosol 250H NF, and 95% DiH₂O (designated herein as 2% W₂₀5EC Natrosol 3); 2% W₂₀5EC, 0.5% Natrosol 250H NF, and 97.5% DiH₂O (designated herein as 2% W₂₀5EC Natrosol 0.5); 2% W₂₀5EC, 2% Methocel A, and 96% DiH₂O (designated herein as 2% W₂₀5EC Methocel A); 2% W₂₀5EC, 2% Methocel K, and 96% DiH₂O (designated herein as 2% W₂₀5EC Methocel K); 2% Natrosol, 0.1% X8PC, 0.1x PBS, 5 mM L-alanine, 5 mM Inosine, 10 mM Ammonium Chloride, and DiH₂O (designated herein as 0.1% X8PC/GE+2% Natrosol); 2% Natrosol, 0.8% Triton X-100, 0.8% Tributyl Phosphate, 6.4% Soybean Oil, 0.1% Cetylpyridinium Chloride, 0.1x PBS, 5 mM L-alanine, 5 mM Inosine, 10 mM Ammonium Chloride, and DiH₂O (designated herein as 10% X8PC/GE+2% Natrosol); 1% Cetylpyridinium Chloride, 5% Tween 20, 8% Ethanol, 64% Lard, and 22% dDiH₂O (designated herein as W₂₀5EC Lard); 1% Cetylpyridinium Chloride, 5% Tween 20, 8% Ethanol, 64% Mineral Oil, and 22% DiH₂O (designated herein as W₂₀5EC Mineral Oil); 0.1% Cetylpyridinium Chloride, 2% Nerolidol, 5% Tween 20, 10% Ethanol, 64% Soybean Oil, and 18.9% DiH₂O (designated herein as W₂₀5EC_{0.1}N); 0.1% Cetylpyridinium Chloride, 2% Farnesol, 5% Tween 20, 10% Ethanol, 64% Soybean Oil, and 18.9% DiH₂O (designated herein as W₂₀5EC_{0.1}F); 0.1% Cetylpyridinium Chloride, 5% Tween 20, 10% Ethanol, 64% Soybean Oil, and 20.9% DiH₂O (designated herein as W₂₀5EC_{0.1}); 10% Cetylpyridinium Chloride, 8% Tributyl Phosphate, 8% Triton X-100, 54% Soybean Oil, and 20% DiH₂O (designated herein as X8PC₁₀); 5% Cetylpyridinium Chloride, 8% Triton X-100, 8% Tributyl Phosphate, 59% Soybean Oil, and 20% DiH₂O (designated herein as X8PC₅); 0.02% Cetylpyridinium Chloride, 0.1% Tween 20, 10% Ethanol, 70% Soybean Oil, and 19.88% DiH₂O (designated herein as W₂₀0.1EC_{0.02}); 1% Cetylpyridinium Chloride, 5% Tween 20, 8% Glycerol, 64% Mobil 1, and 22% DiH₂O (designated herein as W₂₀5GC Mobil 1); 7.2% Triton X-100, 7.2% Tributyl Phosphate, 0.9% Cetylpyridinium Chloride, 57.6% Soybean Oil, 0.1x PBS, 5 mM L-alanine, 5 mM Inosine, 10 mM Ammonium Chloride, and 25.87% DiH₂O (designated herein as 90% X8PC/GE); 7.2% Triton X-100, 7.2% Tributyl Phosphate, 0.9% Cetylpyridinium Chloride, 57.6% Soybean Oil, 1% EDTA, 5 mM L-alanine, 5 mM Inosine, 10 mM Ammonium Chloride, 0.1x PBS, and DiH₂O (designated herein as 90% X8PC/GE EDTA); and 7.2% Triton X-100, 7.2% Tributyl Phosphate, 0.9% Cetylpyridinium Chloride, 57.6% Soybean Oil, 1% Sodium Thiosulfate, 5 mM L-alanine, 5 mM Inosine, 10 mM Ammonium Chloride, 0.1x PBS, and DiH₂O (designated herein as 90% X8PC/GE STS).

Preferably the nanoemulsions are non-toxic (e.g., to humans, plants, or animals), non-irritant (e.g., to humans, plants, or animals), and non-corrosive (e.g., to humans, plants, or animals or the environment), while possessing potency against a broad range of microorganisms including bacteria, fungi, viruses, and spores. While a number of the above described nanoemulsions meet these qualifications, the following description provides a number of preferred non-toxic, non-irritant, non-corrosive, anti-microbial nanoemulsions (hereinafter in this section referred to as "non-toxic nanoemulsions").

The non-toxic nanoemulsions may comprise surfactant lipid preparations (SLPs) for use as broad-spectrum antimicrobial agents that are effective against bacteria and their spores, enveloped viruses, and fungi. These SLPs may comprise a mixture of oils, detergents, solvents, and cationic halogen-containing compounds in addition to several ions that enhance their biocidal activities. These SLPs are characterized as stable, non-irritant, and non-toxic compounds compared to commercially available bactericidal and sporicidal agents, which are highly irritant and/or toxic.

Ingredients for use in the non-toxic nanoemulsions include, but are not limited to: detergents (e.g., TRITON X-100 (5-15%) or other members of the TRITON family, TWEEN 60 (0.5-2%) or other members of the TWEEN family, or TYLOXAPOL (1-10%)); solvents (e.g., tributyl phosphate (5-15%)); alcohols (e.g., ethanol (5-15%) or glycerol (5-15%)); oils (e.g., soybean oil (40-70%)); cationic halogen-containing compounds (e.g., cetylpyridinium chloride (0.5-2%), cetylpyridinium bromide (0.5-2%)), or cetyldimethylethyl ammonium bromide (0.5-2%)); quaternary ammonium compounds (e.g., benzalkonium chloride (0.5-2%), N-alkyldimethylbenzyl ammonium chloride (0.5-2%)); ions (calcium chloride (1mM-40mM), ammonium chloride (1mM-20mM), sodium chloride (5mM-200mM), sodium phosphate (1mM-20mM)); nucleosides (e.g., inosine (50µM-20mM)); and amino acids (e.g., L-alanine (50µM-20mM)). Emulsions are prepared, for example, by mixing in a high shear mixer for 3-10 minutes. The emulsions may or may not be heated before mixing at 82°C for 1 hour.

Quaternary ammonium compounds for use include, but are not limited to, N-alkyldimethyl benzyl ammonium saccharinate; 1,3,5-Triazine-1,3,5(2H,4H,6H)-triethanol; 1-Decanaminium, N-decyl-N, N-dimethyl-, chloride (or) Didecyl dimethyl ammonium chloride; 2-(2-(p-(Diisobuyl)cresosxy)ethoxy)ehyl dimethyl benzyl ammonium chloride; 2-(2-(p-(Diisobutyl)phenoxy)ethoxy)ethyl dimethyl benzyl ammonium chloride; alkyl 1 or 3 benzyl-1-(2-hydroxethyl)-2-imidazolinium chloride; alkyl bis(2-hydroxyethyl) benzyl ammonium chloride; alkyl demethyl benzyl ammonium chloride; alkyl dimethyl 3,4-dichlorobenzyl ammonium chloride (100% C12); alkyl dimethyl 3,4-dichlorobenzyl ammonium chloride (50% C14, 40% C12, 10% C16); alkyl dimethyl 3,4-dichlorobenzyl ammonium chloride (55% C14, 23% C12, 20% C16); alkyl dimethyl benzyl ammonium chloride; alkyl dimethyl benzyl ammonium chloride (100% C14); alkyl dimethyl benzyl ammonium chloride (100% C16); alkyl dimethyl benzyl ammonium chloride (41% C14, 28% C12); alkyl dimethyl benzyl ammonium chloride (47% C12, 18% C14); alkyl dimethyl benzyl ammonium chloride (55% C16, 20% C14); alkyl dimethyl benzyl ammonium chloride (58% C14, 28% C16); alkyl dimethyl benzyl ammonium chloride (60% C14, 25% C12); alkyl dimethyl benzyl ammonium chloride (61% C11, 23% C14); alkyl dimethyl benzyl ammonium chloride (61% C12, 23% C14); alkyl dimethyl benzyl ammonium chloride (65% C12, 25% C14); alkyl dimethyl benzyl ammonium chloride (67% C12, 24% C14); alkyl dimethyl benzyl ammonium chloride (67% C12, 25% C14); alkyl dimethyl benzyl ammonium chloride (90% C14, 5% C12); alkyl dimethyl benzyl ammonium chloride (93% C14, 4% C12); alkyl dimethyl benzyl ammonium chloride (95% C16, 5% C18); alkyl dimethyl benzyl ammonium chloride (and) didecyl dimethyl ammonium chloride; alkyl dimethyl benzyl ammonium chloride (as in fatty acids); alkyl dimethyl benzyl ammonium chloride (C12-C16); alkyl dimethyl benzyl ammonium chloride (C12-C18); alkyl dimethyl benzyl and dialkyl dimethyl ammonium chloride; alkyl dimethyl dimethybenzyl ammonium chloride; alkyl dimethyl ethyl ammonium bromide (90% C14, 5% C16, 5% C12); alkyl dimethyl ethyl ammonium bromide (mixed alkyl and alkenyl groups as in the fatty acids of soybean oil); alkyl dimethyl ethylbenzyl ammonium chloride; alkyl dimethyl ethylbenzyl ammonium chloride (60% C14); alkyl dimethyl isoproylbenzyl ammonium chloride (50% C12, 30% C14, 17% C16, 3% C18); alkyl trimethyl ammonium chloride (58% C18, 40% C16, 1% C14, 1% C12); alkyl trimethyl ammonium chloride (90% C18, 10% C16); alkyldimethyl(ethylbenzyl) ammonium chloride (C12-18); Di-(C8-10)-alkyl dimethyl ammonium chlorides; dialkyl dimethyl ammonium chloride; dialkyl dimethyl ammonium chloride; dialkyl dimethyl ammonium chloride; dialkyl methyl benzyl ammonium chloride; didecyl dimethyl ammonium chloride; diisodecyl dimethyl ammonium chloride; dioctyl dimethyl ammonium chloride; dodecyl bis (2-hydroxyethyl) octyl hydrogen ammonium chloride; dodecyl dimethyl benzyl ammonium chloride; dodecylcarbamoyl methyl dinethyl benzyl ammonium chloride; heptadecyl hydroxyethylimidazolinium chloride; hexahydro-1,3,5-thris(2-hydroxyethyl)-s-triazine; myristalkonium chloride (and) Quat RNIUM 14; N,N-Dimethyl-2-hydroxypropylammonium chloride polymer; n-alkyl dimethyl benzyl ammonium chloride; n-alkyl dimethyl ethylbenzyl ammonium chloride; n-tetradecyl dimethyl benzyl ammonium chloride monohydrate; octyl decyl dimethyl ammonium chloride; octyl dodecyl dimethyl ammonium chloride; octyphenoxyethoxyethyl dimethyl benzyl ammonium chloride; oxydiethylenebis (alkyl dimethyl ammonium chloride); quaternary ammonium compounds, dicoco alkyldimethyl, chloride; trimethoxysily propyl dimethyl octadecyl ammonium chloride; trimethoxysilyl quats, trimethyl dodecylbenzyl ammonium chloride; n-dodecyl dimethyl ethylbenzyl ammonium chloride; n-hexadecyl dimethyl benzyl ammonium chloride; n-tetradecyl dimethyl benzyl ammonium chloride; n-tetradecyl dimethyl ethyylbenzyl ammonium chloride; and n-octadecyl dimethyl benzyl ammonium chloride.

In general, the preferred non-toxic nanoemulsions are characterized by the following: they are approximately 200-800 nm in diameter, although both larger and smaller diameter nanoemulsions are contemplated; the charge depends on the ingredients; they are stable for relatively long periods of time (e.g., up to two years), with preservation of their biocidal activity; they are non-irritant and non-toxic compared to their individual components due, at least in part, to their oil contents that markedly reduce the toxicity of the detergents and the solvents; they are effective at concentrations as low as 0.1%; they have antimicrobial activity against most vegetative bacteria (including Gram-positive and Gram-negative organisms), fungi, and enveloped and nonenveloped viruses in 15 minutes (e.g., 99.99% killing); and they have sporicidal activity in 1-4 hours (e.g., 99.99% killing) when produced with germination enhancers.

### D. Animal Models

Potential nanoemulsion compositions (e.g., for generating an immune response (e.g., for use as a vaccine) may be tested in animal models of infectious diseases. The use of well-developed animal models provides a method of measuring the effectiveness and safety of a vaccine before administration to human subjects. Exemplary animal models of disease are shown in Table 3. These animals are commercially available (*e.g.,* from Jackson Laboratories Charles River; Portage, MI).

Animal models of *Bacillus cereus* (closely related to *Bacillus anthracis*) are utilized to test Anthrax vaccines of the present invention. Both bacteria are spore forming Gram positive rods and the disease syndrome produced by each bacteria is largely due to toxin production and the effects of these toxins on the infected host (Brown et al., J. Bact., 75:499 (1958); Burdon and Wende, J. Infect Dis., 107:224 (1960); Burdon et al., J. Infect. Dis., 117:307 (1967)). *Bacillus cereus* infection mimics the disease syndrome caused by *Bacillus anthracis.* Mice are reported to rapidly succumb to the effects of *B. cereus* toxin and are a useful model for acute infection. Guinea pigs develop a skin lesion subsequent to subcutaneous infection with *B. cereus* that resembles the cutaneous form of anthrax.

*Clostridium perfringens* infection in both mice and guinea pigs has been used as a model system for the *in vivo* testing of antibiotic drugs (Stevens et al., Antimicrob. Agents Chemother., 31:312 (1987); Stevens et al., J. Infect. Dis., 155:220 (1987); Alttemeier et al., Surgery, 28:621 (1950); Sandusky et al., Surgery, 28:632 (1950)). *Clostridium tetani* is well known to infect and cause disease in a variety of mammalian species. Mice, guinea pigs, and rabbits have all been used experimentally (Willis, Topley and Wilson's Principles of Bacteriology, Virology and Immunity. Wilson, G., A. Miles, and M.T. Parker, eds. pages 442-475 1983).

*Vibrio cholerae* infection has been successfully initiated in mice, guinea pigs, and rabbits. According to published reports it is preferred to alter the normal intestinal bacterial flora for the infection to be established in these experimental hosts. This is accomplished by administration of antibiotics to suppress the normal intestinal flora and, in some cases, withholding food from the animals (Butterton et al., Infect. Immun., 64:4373 (1996); Levine et al., Microbiol. Rev., 47:510 (1983); Finkelstein et al., J. Infect. Dis., 114:203 (1964); Freter, J. Exp. Med., 104:411 (1956); and Freter, J. Infect. Dis., 97:57 (1955)).

*Shigella flexnerii* infection has been successfully initiated in mice and guinea pigs. As is the case with vibrio infections, it is preferred that the normal intestinal bacterial flora be altered to aid in the establishment of infection in these experimental hosts. This is accomplished by administration of antibiotics to suppress the normal intestinal flora and, in some cases, withholding food from the animals (Levine et al., Microbiol. Rev., 47:510 (1983); Freter, J. Exp. Med., 104:411 (1956); Formal et al., J. Bact., 85:119 (1963); LaBrec et al., J. Bact. 88:1503 (1964); Takeuchi et al., Am. J. Pathol., 47:1011 (1965)).

Mice and rats have been used extensively in experimental studies with *Salmonella typhimurium* and *Salmonella enteriditis* (Naughton et al., J. Appl. Bact., 81:651 (1996); Carter and Collins, J. Exp. Med., 139:1189 (1974); Collins, Infect. Immun., 5:191 (1972); Collins and Carter, Infect. Immun., 6:451 (1972)).

Mice and rats are well established experimental models for infection with *Sendai* virus (Jacoby et al., Exp. Gerontol., 29:89 (1994); Massion et al., Am. J. Respir. Cell Mol. Biol. 9:361 (1993); Castleman et al., Am. J. Path., 129:277 (1987); Castleman, Am. J. Vet. Res., 44:1024 (1983); Mims and Murphy, Am. J. Path., 70:315 (1973)).

*Sindbis* virus infection of mice is usually accomplished by intracerebral inoculation of newborn mice. Alternatively, weanling mice are inoculated subcutaneously in the footpad (Johnson et al., J. Infect. Dis., 125:257 (1972); Johnson, Am. J. Path., 46:929 (1965)).

It is preferred that animals are housed for 3-5 days to rest from shipping and adapt to new housing environments before use in experiments. At the start of each experiment, control animals are sacrificed and tissue is harvested to establish baseline parameters. Animals are anesthetized by any suitable method (e.g., including, but not limited to, inhalation of Isofluorane for short procedures or ketamine/xylazine injection for longer procedure).

| **Table 3** | | | | | |
|---|---|---|---|---|---|
| **Animal Models of Infectious Diseases** | | | | | |
| **Microorganism** | **Experimental Animal Species** | **Experimental Animal Strains** | **Sex** | **Age** | **Route of Infection** |
| *Francisella philomiraga* | mice | BALB/C | M | 6 W | Intraperitoneal |
| *Neisseria meningitidis* | mice | BALB/C | F | 6-10 W | Intraperitoneal |
| | rats | COBS/CD | M/F | 4 D | Intranasal |
| *Streptococcus pneumoniae* | mice | BALB/C | F | 6 W | Intranasal |
| | rats | COBS/CD | M | 6-8 W | Intranasal |
| | guinea Pigs | Hartley | M/F | 4-5 W | Intranasal |
| *Yersinia pseudotuberculosis* | mice | BALB/C | F | 6 W | Intranasal |
| *Influenza virus* | mice | BALB/C | F | 6 W | Intranasal |
| *Sendai virus* | mice | CD-1 | F | 6 W | Intranasal |
| | rats | Sprague-Dawley | M | 6-8 W | Intranasal |
| *Sindbis* | mice | CD-1 | M/F | 1-2 D | Intracerebral/SC |
| *Vaccinia* | mice | BALB/C | F | 2-3 W | Intradermal |

### E. Assays For Evaluation of Vaccines (not part of the claimed invention)

Candidate nanoemulsion vaccines may be evaluated using one of several suitable model systems. For example, cell-mediated immune responses can be evaluated *in vitro.* In addition, an animal model may be used to evaluate *in vivo* immune response and immunity to pathogen challenge. Any suitable animal model may be utilized, including, but not limited to, those disclosed in Table 3.

Before testing a nanoemulsion vaccine in an animal system, the amount of exposure of the pathogen to a nanoemulsion sufficient to inactivate the pathogen is investigated. It is contemplated that pathogens such as bacterial spores require longer periods of time for inactivation by the nanoemulsion in order to be sufficiently neutralized to allow for immunization. The time period required for inactivation may be investigated using any suitable method, including, but not limited to, those described in the illustrative examples below.

In addition, the stability of emulsion-developed vaccines is evaluated, particularly over time and storage condition, to ensure that vaccines are effective long-term. The ability of other stabilizing materials (*e.g.,* dendritic polymers) to enhance the stability and immunogenicity of vaccines is also evaluated.

Once a given nanoemulsion/pathogen vaccine has been formulated to result in pathogen inactivation, the ability of the vaccine to elicit an immune response and provide immunity is optimized. Non-limiting examples of methods for assaying vaccine effectiveness are described in Examples 1-4 below. For example, the timing and dosage of the vaccine can be varied and the most effective dosage and administration schedule determined. The level of immune response is quantitated by measuring serum antibody levels. In addition, *in vitro* assays are used to monitor proliferation activity by measuring H³-thymidine uptake. In addition to proliferation, Th1 and Th2 cytokine responses (*e.g.,* including but not limited to, levels of include IL-2, TNF-γ, IFN-γ, IL-4, IL-6, IL-11, IL-12, etc.) are measured to qualitatively evaluate the immune response.

Finally, animal models are utilized to evaluate the effect of a nanoemulsion mucosal vaccine. Purified pathogens are mixed in emulsions (or emulsions are contact with a pre-infected animal), administered, and the immune response is determined. The level of protection is then evaluated by challenging the animal with the specific pathogen and subsequently evaluating the level of disease symptoms. The level of immunity is measured over time to determine the necessity and spacing of booster immunizations.

### III. Therapeutics and Prophylactics

The immunogenic compositions are for use in stimulating an immune response to RSV in a subject as defined in the enclosed claim set as per claims 1-11. Furthermore, the immunogenic compositions can be comprised by a vaccine, which in turn may be formulated for administration to a mucosal surface as defined in claims 12-13. The treatment of the human or animal body is not comprised by the present invention.

A composition of the present invention may induce (e.g., when administered to a subject) both systemic and mucosal immunity. Thus, administration of a composition of the present invention to a subject may result in protection against an exposure (e.g., a mucosal exposure) to RSV. Mucosal administration (e.g., vaccination) seems to provide protection against RSV infection (e.g., that initiates at a mucosal surface). Although it has heretofore proven difficult to stimulate secretory IgA responses and protection against pathogens that invade at mucosal surfaces (See, e.g., Mestecky et al, Mucosal Immunology. 3ed edn. (Academic Press, San Diego, 2005)), the present compositions appear to stimulae mucosal immunity (e.g., a protective IgA response) from a pathogen in a subject.

The composition (e.g., a composition comprising a NE and immunogenic protein antigens from RSV (e.g., M2 peptide, F protein, and/or other protein/peptide antigen or virulence factor) may serve as a mucosal vaccine. This material can easily be produced with NE and M2, F protein, and/or other protein/peptide (e.g., viral-derived protein, live-virus-vector-derived protein, recombinant protein, recombinant denatured protein/antigens, small peptide segments protein/antigen, and induces both mucosal and systemic immunity). The ability to produce this formulation rapidly and administer it via mucosal (e.g., nasal) instillation provides a vaccine that can be used in large-scale administrations (e.g., to a population of a town, village, city, state or country).

A composition comprising a NE and an immunogen (e.g., a purified, isolated or synthetic protein or derivative, variant, or analogue thereof; or, one or more serotypes of RSV inactivated by the nanoemulsion) may generate an immune response. When administered to a subject, a composition may stimulate an immune response against the immunogen within the subject. Generation of an immune response (e.g., resulting from administration of a composition comprising a nanoemulsion and an immunogen) may provide total or partial immunity to the subject (e.g., from signs, symptoms or conditions of a disease (e.g., RSV)). Protection and/or immunity from disease (e.g., the ability of a subject's immune system to prevent or attenuate (e.g., suppress) a sign, symptom or condition of disease) after exposure to an immunogenic composition of the present disclosure may be due to adaptive (e.g., acquired) immune responses (e.g., immune responses mediated by B and T cells following exposure to a NE comprising an immunogen of the present disclosure (e.g., immune responses that exhibit increased specificity and reactivity towards RSV). Thus, the compositions are used prophylactically or therapeutically to prevent or attenuate a sign, symptom or condition associated with RSV.

A NE comprising an immunogen (e.g., a recombinant RSV protein) may be administered alone. A composition comprising a NE and an immunogen (e.g., a recombinant RSV protein) may comprise one or more other agents (e.g., a pharmaceutically acceptable carrier, adjuvant, excipient, and the like). A composition for stimulating an immune response may be administered in a manner to induce a humoral immune response. A composition for stimulating an immune response may be administered in a manner to induce a cellular (e.g., cytotoxic T lymphocyte) immune response, rather than a humoral response. A composition comprising a NE and an immunogen may induce both a cellular and humoral immune response.

Apart from RSV, other viruses are known in the prior art, e.g. the type or strain of virus of the *paramyxoviridae* family (e.g., a *Paramyxovirinae* virus (e.g., *Paramyxovirus, Rubulavirus and*/*or Morbillivirus*) Each *paramyxoviridae* family member alone, or in combination with another family member, may be used to generate a composition comprising a NE and an immunogen (e.g., used to generate an immune response). the RSV strain A2 is available from the ATCC, Manassas, VA, ATCC accession No. VR-1540). The virus strain may be RSV strain B (B WV/14617/85, ATCC accession No. VR-1400), RSV strain 9320 (ATCC accession No. VR-955), RSV strain 18537 (ATCC accession No. VR-1580), RSV strain Long (ATCC accession No. VR-26), orRSV strain Line 19 (See, e.g., Lukacs et al., Immunopathology and Infection, 169, 977-986 (2006)). Thus, the virus (RSV) strain utilized may be a modified (e.g., genetically modified (e.g., naturally modified via natural selection or modified using recombinant genetic techniques)) strain that displays greater pathogenic capacity (e.g., causes more sever RSV-induced disease (e.g., comprising enhanced airway hyperreactivity and/or mucus overproduction)).

The immunogen may comprise one or more antigens derived from a pathogen (RSV). For example, the immunogen may be a purified, recombinant, synthetic, or otherwise isolated protein (e.g., added to the NE to generate an immunogenic composition). Similarly, the immunogenic protein may be a derivative, analogue or otherwise modified (e.g., PEGylated) form of a protein from a pathogen.

The composition comprising a NE and immunogen of the present inventionas defined in the claims may comprise one or more different agents in addition to the NE and immunogen. These agents or cofactors include, but are not limited to, adjuvants, surfactants, additives, buffers, solubilizers, chelators, oils, salts, therapeutic agents, drugs, bioactive agents, antibacterials, and antimicrobial agents (e.g., antibiotics, antivirals, etc.). A composition comprising a NE and immunogen of the present invention may comprise an agent and/or co-factor that enhance the ability of the immunogen to induce an immune response (e.g., an adjuvant). The presence of one or more co-factors or agents may reduce the amount of immunogen required for induction of an immune response (e.g., a protective immune respone (e.g., protective immunization)). The presence of one or more co-factors or agents can be used to skew the immune response towards a cellular (e.g., T cell mediated) or humoral (e.g., antibody mediated) immune response..

Adjuvants are described in general in Vaccine Design--the Subunit and Adjuvant Approach, edited by Powell and Newman, Plenum Press, New York, 1995. Suitable adjuvants include an aluminium salt such as aluminium hydroxide gel (alum) or aluminium phosphate. An adjuvant may be a salt of calcium, iron or zinc, or may be an insoluble suspension of acylated tyrosine, or acylated sugars, cationically or anionically derivatised polysaccharides, or polyphosphazenes.

It is preferred that a composition comprising a NE and immunogen comprise one or more adjuvants that induce a Th1-type response or a Th2-type response.

In general, an immune response is generated to an antigen through the interaction of the antigen with the cells of the immune system. Immune responses may be broadly categorized into two categories: humoral and cell mediated immune responses (e.g., traditionally characterized by antibody and cellular effector mechanisms of protection, respectively). These categories of response have been termed Th1-type responses (cell-mediated response), and Th2-type immune responses (humoral response).

Stimulation of an immune response can result from a direct or indirect response of a cell or component of the immune system to an intervention (e.g., exposure to an immunogen). Immune responses can be measured in many ways including activation, proliferation or differentiation of cells of the immune system (e.g., B cells, T cells, dendritic cells, APCs, macrophages, NK cells, NKT cells etc.); up-regulated or down-regulated expression of markers and cytokines; stimulation of IgA, IgM, or IgG titer; splenomegaly (including increased spleen cellularity); hyperplasia and mixed cellular infiltrates in various organs. Other responses, cells, and components of the immune system that can be assessed with respect to immune stimulation are known in the art.

Compositions of the present invention may induce expression and secretion of cytokines (e.g., by macrophages, dendritic cells and CD4+ T cells). Modulation of expression of a particular cytokine can occur locally or systemically. It is known that cytokine profiles can determine T cell regulatory and effector functions in immune responses. Th1-type cytokines can be induced, and thus, the immunostimulatory compositions of the present invention as defined in the claims can promote a Th1 type antigen-specific immune response including cytotoxic T-cells (e.g., thereby avoiding unwanted Th2 type immune responses (e.g., generation of Th2 type cytokines (e.g., IL-13) involved in enhancing the severity of disease (e.g., IL-13 induction of mucus formation))).

Cytokines play a role in directing the T cell response. Helper (CD4+) T cells orchestrate the immune response of mammals through production of soluble factors that act on other immune system cells, including B and other T cells. Most mature CD4+T helper cells express one of two cytokine profiles: Th1 or Th2. Th1-type CD4+ T cells secrete IL-2, IL-3, IFN-γ, GM-CSF and high levels of TNF-α. Th2 cells express IL-3, IL-4, IL-5, IL-6, IL-9, IL-10, IL-13, GM-CSF and low levels of TNF-α. Th1 type cytokines promote both cell-mediated immunity, and humoral immunity that is characterized by immunoglobulin class switching to IgG2a in mice and IgG1 in humans. Th1 responses may also be associated with delayed-type hypersensitivity and autoimmune disease. Th2 type cytokines induce primarily humoral immunity and induce class switching to IgG1 and IgE. The antibody isotypes associated with Th1 responses generally have neutralizing and opsonizing capabilities whereas those associated with Th2 responses are associated more with allergic responses.

Several factors have been shown to influence skewing of an immune response towards either a Th1 or Th2 type response. The best characterized regulators are cytokines. IL-12 and IFN-γ are positive Th1 and negative Th2 regulators. IL-12 promotes IFN-γ production, and IFN- γ provides positive feedback for IL-12. IL-4 and IL-10 appear important for the establishment of the Th2 cytokine profile and to down-regulate Th1 cytokine production.

Thus, stimulating a Th1-type immune response in a subject can be provided by administering to a subject a composition comprising a NE and an immunogen. Furthermore, stimulating a Th2-type immune response in a subject (e.g., if balancing of a T cell mediated response is desired) can be provided by administering to a subject a composition comprising a NE and an immunogen. Adjuvants can be used (e.g., can be co-administered with a composition) to skew an immune response toward either a Th1 or Th2 type immune response. For example, adjuvants that induce Th2 or weak Th1 responses include, but are not limited to, alum, saponins, and SB-As4. Adjuvants that induce Th1 responses include but are not limited to MPL, MDP, ISCOMS, IL-12, IFN-γ, and SB-AS2.

Several other types of Th1-type immunogens can be used (e.g., as an adjuvant). These include, but are not limited to, the following. Monophosphoryl lipid A (e.g., in particular 3-de-O-acylated monophosphoryl lipid A (3D-MPL)), can be used. 3D-MPL is a well known adjuvant manufactured by Ribi Immunochem, Montana. Chemically it is often supplied as a mixture of 3-de-O-acylated monophosphoryl lipid A with either 4, 5, or 6 acylated chains. Diphosphoryl lipid A, and 3-O-deacylated variants thereof can be used. Each of these immunogens can be purified and prepared by methods described in GB 2122204B. Other purified and synthetic lipopolysaccharides have been described (See, e.g., U.S. Pat. No. 6,005,099 and EP 0 729 473; Hilgers et al., 1986, Int.Arch.Allergy.Immunol., 79(4):392-6; Hilgers et al., 1987, Immunology, 60(1):141-6; and EP 0 549 074). 3D-MPL may be used in the form of a particulate formulation (e.g., having a small particle size less than 0.2 µm in diameter, described in EP 0 689 454).

Saponins are also known as an immunogen (e.g.,Th1-type adjuvant) in a composition. Saponins are well known adjuvants (See, e.g., Lacaille-Dubois and Wagner (1996) Phytomedicine vol 2 pp 363-386). Examples of saponins include Quil A (derived from the bark of the South American tree Quillaja Saponaria Molina), and fractions thereof (See, e.g., U.S. Pat. No. 5,057,540; Kensil, Crit Rev Ther Drug Carrier Syst, 1996, 12 (1-2):1-55; and EP 0 362 279). Also contemplated to be useful are the haemolytic saponins QS7, QS17, and QS21 (HPLC purified fractions of Quil A; See, e.g., Kensil et al. (1991). J. Immunology 146,431-437, U.S. Pat. No. 5,057,540; WO 96/33739; WO 96/11711 and EP 0 362 279). Also contemplated to be useful are combinations of QS21 and polysorbate or cyclodextrin (See, e.g., WO 99/10008.

An immunogenic oligonucleotide containing unmethylated CpG dinucleotides ("CpG") may be used as an adjuvant. CpG is an abbreviation for cytosine-guanosine dinucleotide motifs present in DNA. CpG is known in the art as being an adjuvant when administered by both systemic and mucosal routes (See, e.g., WO 96/02555, EP 468520, Davis et al., J.Immunol, 1998, 160(2):870-876; McCluskie and Davis, J.Immunol., 1998, 161(9):4463-6; and U.S. Pat. App. No. 20050238660). For example, the immunostimulatory sequence is Purine-Purine-C-G-pyrimidine-pyrimidine; wherein the CG motif is not methylated.

The presence of one or more CpG oligonucleotides seems to activate various immune subsets including natural killer cells (which produce IFN-γ) and macrophages. CpG oligonucleotides may be formulated into a composition for inducing an immune response. A free solution of CpG may be co-administered together with an antigen (e.g., present within a NE solution (See, e.g., WO 96/02555). A CpG oligonucleotide may be covalently conjugated to an antigen (See, e.g., WO 98/16247), or formulated with a carrier such as aluminium hydroxide (See, e.g., Brazolot-Millan et al., Proc.Natl.AcadSci., USA, 1998, 95(26), 15553-8).

Adjuvants such as Complete Freunds Adjuvant and Incomplete Freunds Adjuvant, cytokines (e.g., interleukins (e.g., IL-2, IFN-γ, IL-4, etc.), macrophage colony stimulating factor, tumor necrosis factor, etc.), detoxified mutants of a bacterial ADP-ribosylating toxin such as a cholera toxin (CT), a pertussis toxin (PT), or an E. Coli heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (See, e.g., WO93/13202 and WO92/19265), and other immunogenic substances (e.g., that enhance the effectiveness of a composition of the present invention) may be used with a composition as defined in the claims.

Additional examples of adjuvants that may find use include poly(di(carboxylatophenoxy)phosphazene (PCPP polymer; Virus Research Institute, USA); derivatives of lipopolysaccharides such as monophosphoryl lipid A (MPL; Ribi ImmunoChem Research, Inc., Hamilton, Mont.), muramyl dipeptide (MDP; Ribi) and threonyl-muramyl dipeptide (t-MDP; Ribi); OM-174 (a glucosamine disaccharide related to lipid A; OM Pharma SA, Meyrin, Switzerland); and Leishmania elongation factor (a purified Leishmania protein; Corixa Corporation, Seattle, Wash.).

Adjuvants may be added to a composition comprising a NE and an immunogen, or, the adjuvant may be formulated with carriers, for example liposomes, or metallic salts (e.g., aluminium salts (e.g., aluminium hydroxide)) prior to combining with or co-administration with a composition comprising a NE and an immunogen.

A composition comprising a NE and an immunogen may comprise a single adjuvant, or two or more adjuvants (See, e.g., WO 94/00153; WO 95/17210; WO 96/33739; WO 98/56414; WO 99/12565; WO 99/11241; and WO 94/00153).

A composition comprising a NE and an immunogen of the present invention may comprise one or more mucoadhesives (See, e.g., U.S. Pat. App. No. 20050281843). A variety of mucoadhesives are contemplated to be useful including, but not limited to, crosslinked derivatives of poly(acrylic acid) (e.g., carbopol and polycarbophil), polyvinyl alcohol, polyvinyl pyrollidone, polysaccharides (e.g., alginate and chitosan), hydroxypropyl methylcellulose, lectins, fimbrial proteins, and carboxymethylcellulose. Use of a mucoadhesive (e.g., in a claimed composition comprising a NE and immunogen) may enhance induction of an immune response in a subject (e.g., administered a composition of the present invention) due to an increase in duration and/or amount of exposure to an immunogen that a subject experiences when a mucoadhesive is used compared to the duration and/or amount of exposure to an immunogen in the absence of using the mucoadhesive.

A composition as defined in the claims may comprise sterile aqueous preparations. Acceptable vehicles and solvents include, but are not limited to, water, Ringer's solution, phosphate buffered saline and isotonic sodium chloride solution. In addition, sterile, fixed oils are conventionally employed as a solvent or suspending medium. For this purpose any bland fixed mineral or non-mineral oil may be employed including synthetic mono-ordi-glycerides. In addition, fatty acids such as oleic acid find use in the preparation of injectables. Carrier formulations suitable for mucosal, subcutaneous, intramuscular, intraperitoneal, intravenous, or administration via other routes may be found in Remington's Pharmaceutical Sciences, Mack Publishing Company, Easton, Pa.

A composition as defined in the claims can be used therapeutically (e.g., to enhance an immune response) or as a prophylactic (e.g., for immunization (e.g., to prevent signs or symptoms of disease)). A composition as defined in the claims can be administered to a subject via a number of different delivery routes and methods.

For example, the compositions can be administered to a subject (e.g., mucosally (e.g., nasal mucosa, vaginal mucosa, etc.)) by multiple methods, including, but not limited to: being suspended in a solution and applied to a surface; being suspended in a solution and sprayed onto a surface using a spray applicator; being mixed with a mucoadhesive and applied (e.g., sprayed or wiped) onto a surface (e.g., mucosal surface); being placed on or impregnated onto a nasal and/or vaginal applicator and applied; being applied by a controlled-release mechanism; being applied as a liposome; or being applied on a polymer.

Compositions may be administered mucosally (e.g., using standard techniques; See, e.g., Remington: The Science and Practice of Pharmacy, Mack Publishing Company, Easton, Pa., 19th edition, 1995 (e.g., for mucosal delivery techniques, including intranasal, pulmonary, vaginal and rectal techniques), as well as European Publication No. 517,565 and Illum et al., J. Controlled Rel., 1994, 29:133-141 (e.g.,for techniques of intranasal administration)). Alternatively, the compositions may be administered dermally or transdermally, using standard techniques (See, e.g., Remington: The Science arid Practice of Pharmacy, Mack Publishing Company, Easton, Pa., 19th edition, 1995).

Mucosal vaccination seems to be the preferred route of administration as it has been shown that mucosal administration of antigens has a greater efficacy of inducing protective immune responses at mucosal surfaces (e.g., mucosal immunity), the route of entry of many pathogens. In addition, mucosal vaccination, such as intranasal vaccination, may induce mucosal immunity not only in the nasal mucosa, but also in distant mucosal sites such as the genital mucosa (See, e.g., Mestecky, Journal of Clinical Immunology, 7:265-276, 1987). More advantageously, in addition to inducing mucosal immune responses, mucosal vaccination may also induce systemic immunity. Non-parenteral administration (e.g., muscosal administration of vaccines) may provide an efficient and convenient way to boost systemic immunity (e.g., induced by parenteral or mucosal vaccination (e.g., in cases where multiple boosts are used to sustain a vigorous systemic immunity)).

A composition as defined in the claims may be used to protect or treat a subject susceptible to, or suffering from, disease by means of administering such a composition via a mucosal route (e.g., an oral/alimentary or nasal route). Alternative mucosal routes include intravaginal and intra-rectal routes. A nasal route of administration may be used, termed "intranasal administration" or "intranasal vaccination" herein. Methods of intranasal vaccination are well known in the art, including the administration of a droplet or spray form of the vaccine into the nasopharynx of a sujbect to be immunized. A nebulized or aerosolized composition comprising a NE and immunogen may be provided. Enteric formulations such as gastro resistant capsules for oral administration, suppositories for rectal or vaginal administration are also an option. Said compositions may also be administered via the oral route. Under these circumstances, a composition comprising a NE and an immunogen may comprise a pharmaceutically acceptable excipient and/or include alkaline buffers, or enteric capsules. Formulations for nasal delivery may include those with dextran or cyclodextran and saponin as an adjuvant.

Compositions as defined in the claims may also be administered via a vaginal route. In such cases, said composition comprising a NE and an immunogen may comprise pharmaceutically acceptable excipients and/or emulsifiers, polymers (e.g., CARBOPOL), and other known stabilizers of vaginal creams and suppositories. Said compositions may be administered via a rectal route. In such cases, said composition comprising a NE and an immunogen may comprise excipients and/or waxes and polymers known in the art for forming rectal suppositories.

The same route of administration (e.g., mucosal administration) may be chosen for both a priming and boosting vaccination. Multiple routes of administration may be utilized (e.g., at the same time, or, alternatively, sequentially) in order to stimulate an immune response (e.g., using a composition as defined in the claims).

For example, a composition as defined in the claimsmay be administered to a mucosal surface of a subject in either a priming or boosting vaccination regime. Alternatively, said composition may be administered systemically in either a priming or boosting vaccination regime. A composition as defined in the claimsmay be administered to a subject in a priming vaccination regimen via mucosal administration and a boosting regimen via systemic administration. A composition as defined in the claimsmay be administered to a subject in a priming vaccination regimen via systemic administration and a boosting regimen via mucosal administration. Examples of systemic routes of administration include, but are not limited to, a parenteral, intramuscular, intradermal, transdermal, subcutaneous, intraperitoneal or intravenous administration. A composition as defined in the claims may be used for both prophylactic and therapeutic purposes.

Compositions as defined in the claims may be administered by pulmonary delivery. For example, a composition can be delivered to the lungs of a subject (e.g., a human) via inhalation (e.g., thereby traversing across the lung epithelial lining to the blood stream (See, e.g., Adjei, et al. Pharmaceutical Research 1990; 7:565-569; Adjei, et al. Int. J. Pharmaceutics 1990; 63:135-144; Braquet, et al. J. Cardiovascular Pharmacology 1989 143-146; Hubbard, et al. (1989) Annals of Internal Medicine, Vol. III, pp. 206-212; Smith, et al. J. Clin. Invest. 1989;84:1145-1146; Oswein, et al. "Aerosolization of Proteins", 1990; Proceedings of Symposium on Respiratory Drug Delivery II Keystone, Colorado; Debs, et al. J. Immunol. 1988; 140:3482-3488; and U.S. Pat. No. 5,284,656 to Platz, et al). A method and composition for pulmonary delivery of drugs for systemic effect is described in U.S. Pat. No. 5,451,569 to Wong; See also U.S. Pat. No. 6,651,655 to Licalsi et al.)).

Further contemplated for use in the practice are a wide range of mechanical devices designed for pulmonary and/or nasal mucosal delivery of pharmaceutical agents including, but not limited to, nebulizers, metered dose inhalers, and powder inhalers, all of which are familiar to those skilled in the art. Some specific examples of commercially available devices suitable for the practice of this invention are the Ultravent nebulizer (Mallinckrodt Inc., St. Louis, Mo.); the Acorn II nebulizer (Marquest Medical Products, Englewood, Colo.); the Ventolin metered dose inhaler (Glaxo Inc., Research Triangle Park, N.C.); and the Spinhaler powder inhaler (Fisons Corp., Bedford, Mass.). All such devices require the use of formulations suitable for dispensing of the therapeutic agent. Typically, each formulation is specific to the type of device employed and may involve the use of an appropriate propellant material, in addition to the usual diluents, adjuvants, surfactants, carriers and/or other agents useful in therapy. Also, the use of liposomes, microcapsules or microspheres, inclusion complexes, or other types of carriers is contemplated.

Thus, a composition comprising a NE and an immunogen of the present invention may be used to protect and/or treat a subject susceptible to, or suffering from, a disease by means of administering said compositions comprising a NE and an immunogen by mucosal, intramuscular, intraperitoneal, intradermal, transdermal, pulmonary, intravenous, subcutaneous or other route of administration described herein. Methods of systemic administration of the vaccine preparations may include conventional syringes and needles, or devices designed for ballistic delivery of solid vaccines (See, e.g., WO 99/27961), or needleless pressure liquid jet device (See, e.g., U.S. Pat. No. 4,596,556; U.S. Pat. No. 5,993,412), or transdermal patches (See, e.g., WO 97/48440; WO 98/28037). The immunogenicity of antigens may be enhanced by application to the skin (transdermal or transcutaneous delivery, See, e.g., WO 98/20734 ; WO 98/28037). Thus,a delivery device for systemic administration, pre-filled with the vaccine composition can be used.

A wide variety of subjects are contemplated to be benefited from administration of a composition as defined in the claims. The subject may be a human. The human subjects may be of any age (e.g., adults, children, infants, etc.) that have been or are likely to become exposed to RSV). The human subjects may be subjects that are more likely to receive a direct exposure to RSV or that are more likely to display signs and symptoms of RSV disease after exposure to RSV (e.g., immune suppressed subjects). The general public may be administered (e.g., vaccinated with) a composition of the present invention (e.g., to prevent the occurrence or spread of disease). For example, compositions as defined in the claims are utilized to vaccinate a group of people (e.g., a population of a region, city, state and/or country) for their own health (e.g., to prevent or treat disease). The subjects may be non-human mammals (e.g., pigs, cattle, goats, horses, sheep, or other livestock; or mice, rats, rabbits or other animal). A composition of the present invention as defined in the claims may be formulated for administration by any route, such as mucosal, oral, topical, parenteral or other route described herein. The compositions may be in any one or more different forms including, but not limited to, tablets, capsules, powders, granules, lozenges, foams, creams or liquid preparations.

Topical formulations may be presented as, for instance, ointments, creams or lotions, foams, and aerosols, and may contain appropriate conventional additives such as preservatives, solvents (e.g., to assist penetration), and emollients in ointments and creams.

Topical formulations may also include agents that enhance penetration of the active ingredients through the skin. Exemplary agents include a binary combination of N-(hydroxyethyl) pyrrolidone and a cell-envelope disordering compound, a sugar ester in combination with a sulfoxide or phosphine oxide, and sucrose monooleate, decyl methyl sulfoxide, and alcohol.

Other exemplary materials that increase skin penetration include surfactants or wetting agents including, but not limited to, polyoxyethylene sorbitan mono-oleoate (Polysorbate 80); sorbitan mono-oleate (Span 80); p-isooctyl polyoxyethylene-phenol polymer (Triton WR-1330); polyoxyethylene sorbitan tri-oleate (Tween 85); dioctyl sodium sulfosuccinate; and sodium sarcosinate (Sarcosyl NL-97); and other pharmaceutically acceptable surfactants.

The compositions may further comprise one or more alcohols, zinc-containing compounds, emollients, humectants, thickening and/or gelling agents, neutralizing agents, and surfactants. Water used in the formulations is preferably deionized water having a neutral pH. Additional additives in the topical formulations include, but are not limited to, silicone fluids, dyes, fragrances, pH adjusters, and vitamins.

Topical formulations may also contain compatible conventional carriers, such as cream or ointment bases and ethanol or oleyl alcohol for lotions. Such carriers may be present as from about 1% up to about 98% of the formulation. The ointment base can comprise one or more of petrolatum, mineral oil, ceresin, lanolin alcohol, panthenol, glycerin, bisabolol, cocoa butter and the like.

Pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product.

The compositions of the present invention as defined in the claims may additionally contain other adjunct components conventionally found in pharmaceutical compositions. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present invention, such as dyes, flavoring agents, preservatives, antioxidants, opacifiers, thickening agents and stabilizers. However, such materials, when added, preferably do not unduly interfere with the biological activities of the components of the compositions of the present invention. The formulations can be sterilized and, if desired, mixed with auxiliary agents (e.g., lubricants, preservatives, stabilizers, wetting agents, emulsifiers, salts for influencing osmotic pressure, buffers, colorings, flavorings and/or aromatic substances and the like) that do not deleteriously interact with the NE and immunogen of the formulation. Immunostimulatory compositions may be administered in the form of a pharmaceutically acceptable salt. When used the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include, but are not limited to, acetic acid and a salt (1-2% w/v); citric acid and a salt (1-3% w/v); boric acid and a salt (0.5-2.5% w/v); and phosphoric acid and a salt (0.8-2% w/v). Suitable preservatives may include benzalkonium chloride (0.003-0.03% w/v); chlorobutanol (0.3-0.9% w/v); parabens (0.01-0.25% w/v) and thimerosal (0.004-0.02% w/v).

A composition comprising NE and an immunogen may be co-administered with one or more antibiotics. For example, one or more antibiotics may be administered with, before and/or after administration of a composition comprising a NE and an immunogen. A variety of antibiotics may be co-administered including, but not limited to, β -lactam antibiotics, penicillins (such as natural penicillins, aminopenicillins, penicillinase-resistant penicillins, carboxy penicillins, ureido penicillins), cephalosporins (first generation, second generation, and third generation cephalosporins), and other β-lactams (such as imipenem, monobactams,), β -lactamase inhibitors, vancomycin, aminoglycosides and spectinomycin, tetracyclines, chloramphenicol, erythromycin, lincomycin, clindamycin, rifampin, metronidazole, polymyxins, doxycycline, quinolones (e.g., ciprofloxacin), sulfonamides, trimethoprim, and quinolines.

There are an enormous amount of antimicrobial agents currently available for use in treating bacterial, fungal and viral infections. For a comprehensive treatise on the general classes of such drugs and their mechanisms of action, the skilled artisan is referred to Goodman & Gilman's "The Pharmacological Basis of Therapeutics" Eds. Hardman et al., 9th Edition, Pub. McGraw Hill, chapters 43 through 50, 1996,. Generally, these agents include agents that inhibit cell wall synthesis (*e.g.,* penicillins, cephalosporins, cycloserine, vancomycin, bacitracin); and the imidazole antifungal agents (*e.g.,* miconazole, ketoconazole and clotrimazole); agents that act directly to disrupt the cell membrane of the microorganism (*e.g.,* detergents such as polmyxin and colistimethate and the antifungals nystatin and amphotericin B); agents that affect the ribosomal subunits to inhibit protein synthesis (*e.g.,* chloramphenicol, the tetracyclines, erthromycin and clindamycin); agents that alter protein synthesis and lead to cell death (*e.g.,* aminoglycosides); agents that affect nucleic acid metabolism (*e.g.,* the rifamycins and the quinolones); the antimetabolites (*e.g.,* trimethoprim and sulfonamides); and the nucleic acid analogues such as zidovudine, gangcyclovir, vidarabine, and acyclovir which act to inhibit viral enzymes essential for DNA synthesis. Various combinations of antimicrobials may be employed.

Co-administration of a composition comprising a NE and an immunogen as defined in the claims with one or more additional active and/or immunostimulatory agents (e.g., a composition comprising a NE and a different immnogen, an antibiotic, anti-oxidant, etc.) also appears to be an option. Prior art immunostimulatory methods (e.g., immunization methods) and/or pharmaceutical compositions may be enhanced by co-administering a composition of the present invention. In co-administration procedures, the agents may be administered concurrently or sequentially. The compositions described herein may be administered prior to the other active agent(s). The pharmaceutical formulations and modes of administration may be any of those described herein. In addition, the two or more co-administered agents may each be administered using different modes (e.g., routes) or different formulations. The additional agents to be co-administered (e.g., antibiotics, adjuvants, etc.) can be any of the well-known agents in the art, including, but not limited to, those that are currently in clinical use.

A composition comprising a NE and immunogen as defined in the claims may be administered to a subject via more than one route. For example, a subject that would benefit from having a protective immune response (e.g., immunity) towards a pathogenic microorganism may benefit from receiving mucosal administration (e.g., nasal administration or other mucosal routes described herein) and, additionally, receiving one or more other routes of administration (e.g., parenteral or pulmonary administration (e.g., via a nebulizer, inhaler, or other methods described herein). Administration via mucosal route may be sufficient to induce both mucosal as well as systemic immunity towards an immunogen or organism from which the immunogen is derived. Administration via multiple routes may serve to provide both mucosal and systemic immunity. It is contemplated that a subject administered a composition via multiple routes of administration (e.g., immunization (e.g., mucosal as well as airway or parenteral administration) may have a stronger immune response to an immunogen than a subject administered a composition via just one route.

Other delivery systems can include time-release, delayed release or sustained release delivery systems. Such systems can avoid repeated administrations of the compositions, increasing convenience to the subject and a physician. Many types of release delivery systems are available and known to those of ordinary skill in the art. They include polymer based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di-and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Pat. Nos. 4,452,775, 4,675,189, and 5,736,152, and (b) diffusional systems in which an active component permeates at a controlled rate from a polymer such as described in U.S. Pat. Nos. 3,854,480, 5,133,974 and 5,407,686. In addition, pump-based hardware delivery systems can be used, some of which are adapted for implantation.

A composition comprising NE and an immunogen of the present invention may comprise a suitable amount of said immunogen to induce an immune response in a subject when administered to the subject. The immune response may be sufficient to provide the subject protection (e.g., immune protection) against a subsequent exposure to the immunogen or the microorganism (RSV) from which the immunogen was derived. The amount of immunogen may be selected as that amount which induces an immunoprotective response without significant, adverse side effects. The amount will vary depending upon which specific immunogen or combination thereof is/are employed, and can vary from subject to subject, depending on a number of factors including, but not limited to, the species, age and general condition (e.g., health) of the subject, and the mode of administration. Procedures for determining the appropriate amount of immunogen administered to a subject to elicit an immune response (e.g., a protective immune response (e.g., protective immunity)) in a subject are well known to those skilled in the art.

It is expected that each dose comprises 0.05-5000 µg of each immunogen (e.g., recombinant and/or purified protein), e.g. each dose will comprise 1-500 µg, i350-75µg, 50-200µg, or 25-75)µg of immunogen. Each dose may comprise an amount of the immunogen sufficient to generate an immune response. An effective amount of the immunogen in a dose need not be quantified, as long as the amount of immunogen generates an immune response in a subject when administered to the subject. An optimal amount for a particular administration (e.g., to induce an immune response (e.g., a protective immune response (e.g., protective immunity))) can be ascertained by one of skill in the art using standard studies involving observation of antibody titers and other responses in subjects.

It is expected that each dose is from 0.001 to 15% or more (e.g., 0.001-10%, 0.5-5%, 1-3%, 2%, 6%, 10%, 15% or more) by weight immunogen. An initial or prime administration dose may contain more immunogen than a subsequent boost dose.

When a NE is utilized to inactivate a live microorganism (RSV), it is expected that each dose (e.g., administered to a subject to induce and immune response)) comprises between 10 and 10⁹ pfu of the virus per dose; between 10⁵ and 10⁸ pfu of the virus per dose; between 10³ and 10⁵ pfu of the virus per dose; between 10² and 10⁴ pfu of the virus per dose; 10 pfu of the virus per dose; 10² pfu of the virus per dose; 10⁴ pfu of the virus per dosemore than 10⁹ pfu of the virus per dose or 10³ pfu of the virus per dose.

In order to inactivate live microorganisms (RSV), a 0.1% - 5% NE solution may be used, a 5%-20% NE solution may be used, a 20% NE solution may be used, and a NE solution greater than 20% may be used order to inactivate a pathogenic microorganism. In particular, a 15% NE solution may be used.

The microorganism may be incubated for 1-3 hours in NE, for 3-6 hours in NE, for more than 6 hours in NE,or for 3 hours in NE (e.g., a 10% NE solution). The incubation may be carried out at 37°C, or at a temperature greater than or less than 37°C. The amount of microorganism used for inactivation may depend upon a number of factors including, but not limited to, the total amount of immunogenic composition desired, the concentration of solution desired (e.g., prior to dilution for administration), the microorganism and the NE. The amount of microorganism used in an inactivation procedure may be that amount that produces the desired amount of immunogen as described herein to be administered in a single dose (e.g., diluted from a concentrated stock) to a subject.

A composition comprising a NE and an immunogen of the present invention as defined in the claims may be formulated in a concentrated dose that can be diluted prior to administration to a subject. For example, dilutions of a concentrated composition may be administered to a subject such that the subject receives any one or more of the specific dosages provided herein. Dilution of a concentrated composition may be made such that a subject is administered (e.g., in a single dose) a composition comprising 0.5-50% of the NE and immunogen present in the concentrated composition. A subject may administered in a single dose a composition comprising 1% of the NE and immunogen present in the concentrated composition. Concentrated compositions are contemplated to be useful in a setting in which large numbers of subjects may be administered a composition of the present invention (e.g., an immunization clinic, hospital, school, etc.). A composition comprising a NE and an immunogen of the present invention (e.g., a concentrated composition) may stable at room temperature for more than 1 week, for more than 2 weeks, for more than 3 weeks, for more than 4 weeks, for more than 5 weeks, and for more than 6 weeks.

Generally, the emulsion compositions may comprise at least 0.001% to 100%, preferably 0.01 to 90%, of emulsion per ml of liquid composition. It is envisioned that the formulations may comprise about 0.001%, about 0.0025%, about 0.005%, about 0.0075%, about 0.01%, about 0.025%, about 0.05%, about 0.075%, about 0. 1 %, about 0.25%, about 0.5%, about 1.0%, about 2.5%, about 5%, about 7.5%, about 10%, about 12.5%, about 15%, about 20%, about 25%, about 30%, about 35%, about 40%, about 50%, about 55%, about 60%, about 65%, about 70%, about 75%, about 80%, about 85%, about 90%, about 95% or about 100% of emulsion per ml of liquid composition. It should be understood that a range between any two figures listed above is specifically contemplated to be encompassed within the metes and bounds of the present disclosure. Some variation in dosage will necessarily occur depending on the condition of the specific pathogen and the subject being immunized.

Following an initial administration of a composition of the present invention as defined in the claims (e.g., an initial vaccination), a subject may receive one or more boost administrations (e.g., around 2 weeks, around 3 weeks, around 4 weeks, around 5 weeks, around 6 weeks, around 7 weeks, around 8 weeks, around 10 weeks, around 3 months, around 4 months, around 6 months, around 9 months, around 1 year, around 2 years, around 3 years, around 5 years, around 10 years) subsequent to a first, second, third, fourth, fifth, sixth, seventh, eights, ninth, tenth, and/or more than tenth administration. Reintroduction of an immunogen in a boost dose seems to enable vigorous systemic immunity in a subject. The boost can be with the same formulation given for the primary immune response, or can be with a different formulation that contains the immunogen. The dosage regimen will also, at least in part, be determined by the need of the subject and be dependent on the judgment of a practitioner.

Dosage units may be proportionately increased or decreased based on several factors including, but not limited to, the weight, age, and health status of the subject. In addition, dosage units may be increased or decreased for subsequent administrations (e.g., boost administrations).

It is contemplated that the compositions of the present invention as defined in the claims will find use in various settings, including research settings. For example, compositions of the present invention as defined in the claims also find use in studies of the immune system (e.g., characterization of adaptive immune responses (e.g., protective immune responses (e.g., mucosal or systemic immunity))). Uses of the compositions provided by the present invention as defined in the claims encompass human and non-human subjects and samples from those subjects, and also encompass research applications using these subjects. Compositions of the present invention as defined in the claims are also useful in studying and optimizing nanoemulsions, immunogens, and other components and for screening for new components.

The formulations can be tested *in vivo* in a number of animal models developed for the study of mucosal and other routes of delivery. As is readily apparent, the compositions of the present invention as defined in the claims are useful for preventing and/or treating a wide variety of diseases and infections caused by viruses, bacteria, parasites, and fungi, as well as for eliciting an immune response against a variety of antigens. Not only can the compositions be used prophylactically or therapeutically, as described above, the compositions can also be used in order to prepare antibodies, both polyclonal and monoclonal (e.g., for diagnostic purposes), as well as for immunopurification of an antigen of interest. If polyclonal antibodies are desired, a selected mammal, (e.g., mouse, rabbit, goat, horse, etc.) can be immunized with the compositions of the present invention. The animal is usually boosted 2-6 weeks later with one or more--administrations of the antigen. Polyclonal antisera can then be obtained from the immunized animal and used according to known procedures (See, e.g., Jurgens et al., J. Chrom. 1985, 348:363-370).

### EXAMPLES

In the experimental disclosure which follows, the following abbreviations apply: eq (equivalents); µ (micron); M (Molar); µM (micromolar); mM (millimolar); N (Normal); mol (moles); mmol (millimoles); µmol (micromoles); nmol (nanomoles); g (grams); mg (milligrams); µg (micrograms); ng (nanograms); L (liters); ml (milliliters); µl (microliters); cm (centimeters); mm (millimeters); µm (micrometers); nM (nanomolar);°C (degrees Centigrade); and PBS (phosphate buffered saline).

### EXAMPLE 1

### Compositions comprising nanoemulsion inactivated respiratory syncytial virus and methods of utilizing the same

### Materials and Methods

Mice. Balb/c mice were purchased from Jackson Laboratories. All animal work was performed in accordance with the University of Michigan Committee on Use and Care of Animals policy.

Viral plaque assay. The right lobe of lungs from infected mice were harvested and ground with sand using a mortar and pestle. Samples from lungs were spun 2x or taken after incubation with nanoemulsion and supernatants serially diluted onto an ∼90% confluent monolayer of Vero cells. Samples were incubated at 37° with gentle rotation for 2h, then infected supernatants were removed and replaced with 0.9% methylcellulose. After incubation at 37°C for 5 days, methylcellulose was removed, replaced with methanol, and incubated at -80° C for 1h. After removal of methanol, samples were stored at -80°C until plaque development. Plaques were developed using a modified ELISA protocol. Briefly, cells were blocked for 1h at 37° with 25% Blotto (milk powder diluted in phosphate buffered saline), washed and incubated for 1h at 37° with goat anti-human RSV polyclonal Ab (Chemicon International). Cells were washed again and incubated for 1hr with horse radish peroxidase-conjugated anti goat/sheep IgG (Serotec). Cells were washed and incubated at room temperature with chloronaphthol and plaques enumerated.

Preparation of RSV nanoemulsion. RSV (RSV strain Line 19 (See, e.g., Lukacs et al., Immunopathology and Infection, 169, 977-986 (2006)) (2 X 10⁶ pfu) was incubated with 15% W₈₀5EC nanoemulsion for 60 minutes, a time determined in experiments conducted during development of embodiments of the invention to inactivate the virus fully. The RSV vaccine preparation was made up fresh for each of the immunizations. Each animal received 10 µl of the emulsion or the emulsion + RSV into the left nare on day 0 and day 28. This meant that a total of 1 X 10⁴ pfu was used per vaccination/mouse.

Bronchoalveolar lavage cytokine measure. Bronchoalveolar lavage (BAL) was performed on infected mice using 1ml of sterile PBS. Cell suspensions were centrifuged and supernatants collected for cytokine analysis and measured by Bioplex using kits purchased from R&D systems.

Lung Dispersion and RSV rechallenge in vitro. The lungs were removed after the 1 ml PBS-EDTA lavage and dispersed with collagenase (0.2%, Type IV, Sigma) for 45 minutes in a rotating water bath (37C). The dispersed cells were then counted. The single cell suspension was then plated at a concentration of 2 x 10⁶/ml and incubated with RSV (MOI-0.5). The cell-free supernatants were collected after 36 hrs and assessed by Bioplex for the levels of cytokines that were produced. This assay allowed us to assess the overall response within the lungs of the vaccinated vs. the unvaccinated mouse groups.

### EXAMPLE 2

### Nanoemulsion effectively inactivates RSV

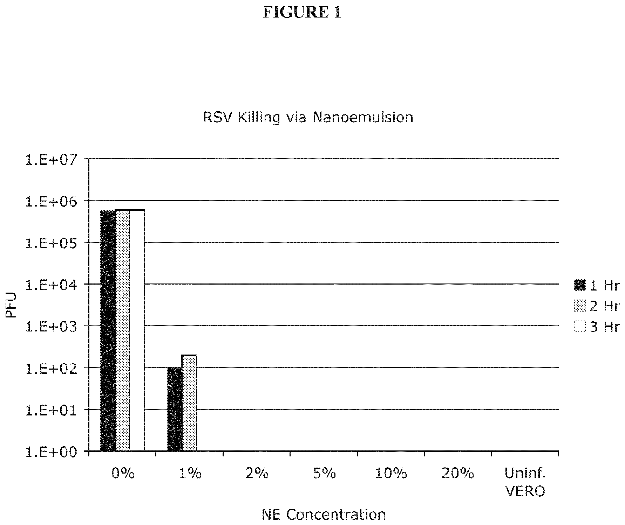

In order to test the ability of emulsion to inactivate RSV, RSV (10⁶ particle forming units (PFU) was incubated with nanoemulsion at varying concentrations (0% - 20%) and varying times (1hr - 3 hrs) (See Figure 1). The number of infectious virus was determined via plaque assay using Vero cells. Nanoemulsion incubated virus was used to infect sub-confluent Vero cells. RSV plaques were visualized using immunohistochemical techniques. At as little as 1% nanoemulsion incubated for 3 hrs, there was no detection of active virus as assessed by standard plaque assay (See Figure 1). Thus, nanoemulsion is effective at completely killing RSV at a concentration of 2% in as little as one hour, or as little as 1% in three hours. According, a nanoemulsion has been provided that is effective at reducing and/or fully inactivating RSV infectivity.

### EXAMPLE 3

### Nanoemulsion immunization enhances immunity upon RSV challenge

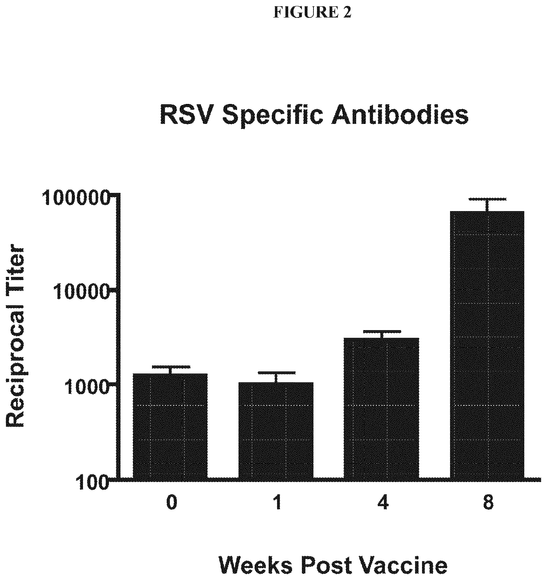

It was next determined whether the nanoemulsion could be used as an immuno-enhancing agent to induce immune responses important for protection against virus infection. To examine this aspect, an immunization protocol was utilized comprising immunizing animals by intranasal sensitization with nanoemulsion inactivated virus (nanoemulsion (15%)-RSV mixture (10 µl total, 5µl /nare)) at day 0 and boosted at day 28 or only nanoemulsion alone with no RSV as a control group. Animals were then challenged with live, infectious RSV at Day 56 (8 weeks) and assessed for evidence of protective immunity. One objective was to monitor RSV-specific antibody production during the immunization protocol. The reciprocal titer of RSV specific antibodies in serum was determined via enzyme-linked immunosorbent assay (ELISA) against RSV protein extract. Blood was harvested and serum collected at specific time points post immunization including Day 0, 1 week, 4 weeks and 8 weeks (at the time of RSV challenge) and the total serum IgG specific for RSV was assessed. As shown in Figure 2, anti-RSV IgG titer was not detectable at 1-week post immunization, increased after 4 weeks prior to the boost and increased significantly by 8 weeks post-initial immunization. Thus, immunization (e.g., intranasal immunization) of a subject with nanoemulsion inactivated RSV induces an anti-RSV immune response in a subject. An anti-RSV immune response is induced in a subject within four weeks after administration of nanoemulsion inactivated RSV to the subject. An anti-RSV immune response is induced in a subject upon a second administration of nanoemulsion inactivated RSV to the subject (e.g., after a "boost" administration). A composition comprising nanoemulsion inactive RSV useful for generating an anti-RSV specific immune response in a subject administered the composition has been provided.

### EXAMPLE 4

### RSV-nanoemulsion immunization induces cytotoxic and Th1 type anti-viral immune responses.

Because of the severe problems associated with other types of vaccines for RSV (e.g., generation of severe exacerbated disease upon infection post administration of formalin inactivated RSV to subjects), it was next determined what type of immune response was generated upon administration of nanoemulsion inactivated RSV to subjects.

Administration of NE-RSV enhanced antiviral cytokines in the BAL fluid from airways of RSV challenged mice. A 1 ml PBS wash of the airway was utilized and the levels of cytokines in the lungs were determined via multiplex analysis of BAL fluid (Bioplex, R&D systems) from lungs at day 8 post-challenge, a time when T cell cytokines peak.

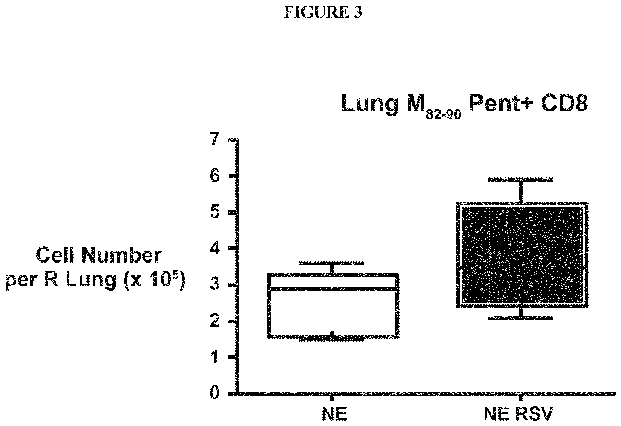

As shown in Figure 3, subjects administered (e.g., nasally) nanoemulsion inactivated RSV displayed increased numbers of M2 peptide specific cytotoxic CD8+ cytotoxic T cells compared to the nanoemulsion control immunization group. The number of RSV M82-90 specific CD8 T cells was determined by flow cytometric analysis of enzymatically-digested lungs at day 4-post challenge using a specific MHC class I tetramer that specifically recognizes the TCR for the immunodominant peptide of M82-90. In addition, the assessment of an anti-viral environment in the airway using BAL fluid indicated an increased production of IFN-γ and IL-17 in the subjects but no increase in the pathogenic Th2 cytokines, IL-4, IL-5 and IL-13, during the viral challenge stage (See Figure 4). As described above, the Th2 type cytokines were identified as having a causative role in previous vaccine trials performed with formalin-inactivated RSV. Moreover, the Th2 cytokine interleukin-13 (IL-13) is a mediator of pulmonary mucus secretion (See Hershey, G. K. 2003. J. Allergy Clin. Immunol. 111:677-690, Walter et al., 2001 J. Immunol. 167:4668-4675; Zhu et al., 1999 J. Clin. Investig. 103:779-788) and IL-13-expressing RSV-specific T cells are found in RSV bronchiolitis (deWaal, 2003 J. Med. Virol. 70:309-318). Thus, immunogenic compositions comprising NE inactive RSV and methods of utilizing the same to generate immune responses to RSV in a subject without enhanced production of mucus, airway constriction, airway hyperresponsiveness, air trapping, hpoxia and/or partial lung collapse (e.g., resulting from enhanced expression of Th2 type cytokines (e.g., IL-13)) have been provided.

To further assess cytokine response in subjects administered NE-RSV, lungs were isolated from animals that were either not infected or from those vaccinated and challenged and compared to animals unvaccinated and challenged with RSV. The lungs were removed and collagenased dispersed into a single cell suspension followed by an in vitro re-challenge with virus. Total lung leukocytes were isolated from the lungs of uninfected control (UC), vaccinated and challenged (Vaccine), or control RSV challenged (Unvaccinated) mice. Cells were cultured at a concentration of 2 x 10⁶/ml for 36 hours in the presence of live RSV (MOI = 0.5). Cytokine concentrations were measured in supernatants via bioplex multiplex assay.

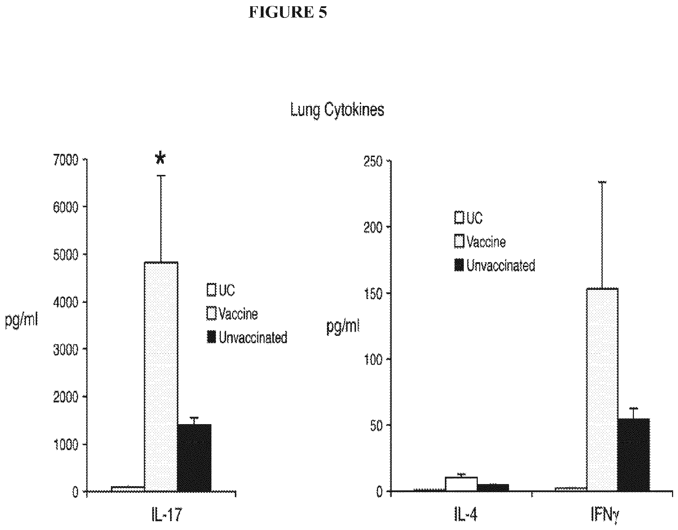

It was observed that while IL-17 production was again significantly upregulated and IFN was increased, the Th2 cytokine IL-4 was not altered (See Figure 5). Thus, vaccination with NE-RSV does not presensitize mice to a more pathogenic response (e.g., in contrast to results obtained with formalin inactivated RSV). An increase in IFN-γ and IL-17 seems to reflect a more anti-viral immune environment induced by nanoemulsion inactivated RSV immunization protocol.

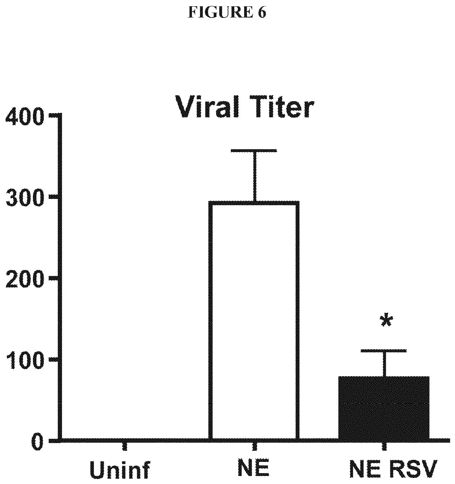

Important to the outcome of any immunization protocol is determining whether there is an increase in viral clearance post immunization and exposure to live virus. Mice were immunized intranasally twice separated by four weeks with 10⁶ PFU RSV in 15% NE (NE-RSV), or 15% NE alone (NE) as a control. Mice were then challenged four weeks after the second vaccination (eight weeks total), and the number of viable virus particles determined via plaque assay of right lungs. When subjects were assessed by plaque assay to determine the counts of live virus present in the lungs of vaccinated and subsequently virally infected subjects, nanoemulsion-RSV immunized subjects demonstrated a significant increased clearance (decreased viral plaques) compared to unimmunized subjects (See Figure 6). Thus, administration of nanoemulsion inactivated RSV (NE-RSV) establishes a protective response in subjects.

### EXAMPLE 5

### RSV-nanoemulsion immunization and allergic asthma

Epidemiologic studies have suggested a link between early severe RSV infection and the subsequent development of allergic asthma. Thus, in order to determine whether vaccination with NE-RSV followed by live viral challenge would affect the subsequent response to a model of allergic asthma, mice were vaccinated twice with NE-RSV, challenged intranaslly with 10⁵ PFU RSV, and sensitized to cockroach allergen. In this model, mice receive an intraperioneal/subcutaneous administration of clinical skin-test grade cockroach allergen (100µg) emulsified in incomplete Freund's adjuvant at day 21 post-RSV challenge. Mice then received one intranasal challenge fourteen days later (15µg) and two intratracheal challenges (40µg) five and seven days subsequent to intranasal challenge. Mice were assessed for allergic disease 24 hours after the last intratracheal challenge.

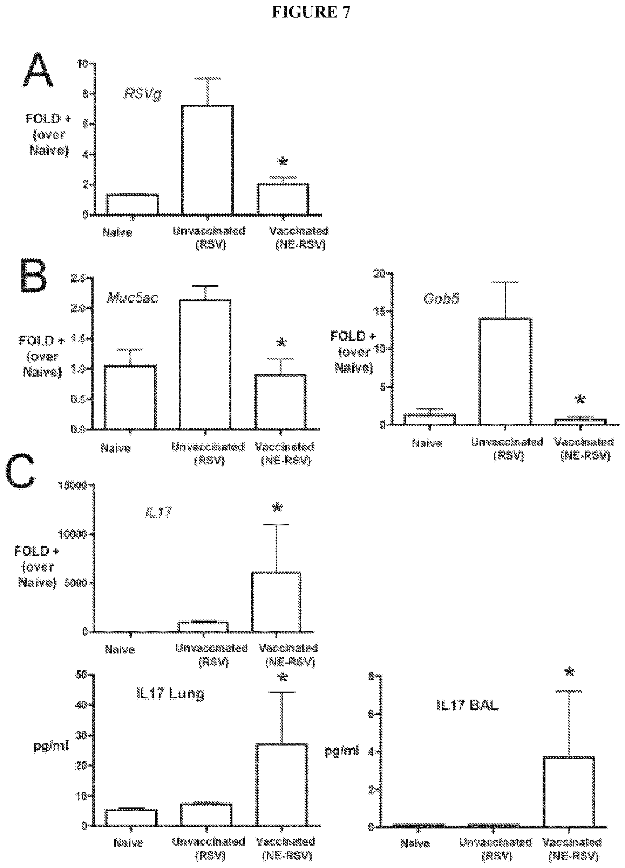

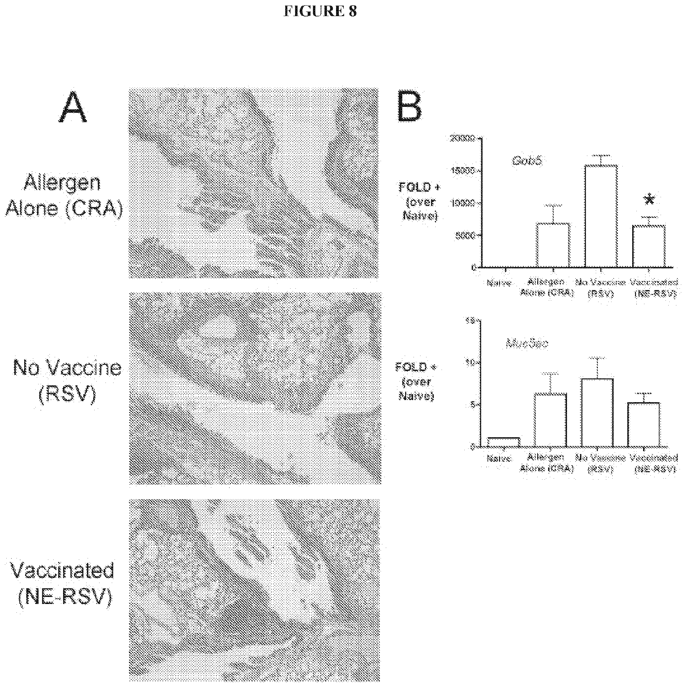

One of the hallmarks of allergic lung disease is the hypersecretion of mucus. Compared to unvaccinated mice, NE-RSV vaccinated mice exhibited attenuated allergen-induced mucus responses as assessed via periodic acid schiff's (PAS) staining of lung histologic sections (See Figure 8), as well as reduced expression of the mucus gene *Gob5* in total lung RNA (See Figures 7 and 8). Similar to viral challenge alone, allergen challenged NE-RSV vaccinated animals had significantly higher induction of IL-17 in the lungs, as assessed via QPCR (See Figure 9A). Th2 cytokines are important for promoting allergic lung disease. NE-RSV vaccinated mice exhibited attenuated production of Th2 cytokines, including IL-4 (homogenized lungs and BAL) and IL-5 (lungs) (See Figure 9B). A trend toward decreased IL-13 mRNA was noted, as well. There was no enhancement of allergic disease in NE-RSV vaccinated animals. Additionally, vaccinated mice had significantly lower expression of the alternatively activated macrophage marker Fizz-1. Alternatively activated macrophages are associated with Th2 responses, as well as fibrotic disease. The decrease in Fizz-1 seems to reflect a consequence of decreased Th2 cytokines in vaccinated mice, and also provides a mechanism by which NE-RSV vaccination protects against the subsequent development of allergic lung disease.

### EXAMPLE 6

### RSV-nanoemulsion immunization induces RSV-specific antibody production

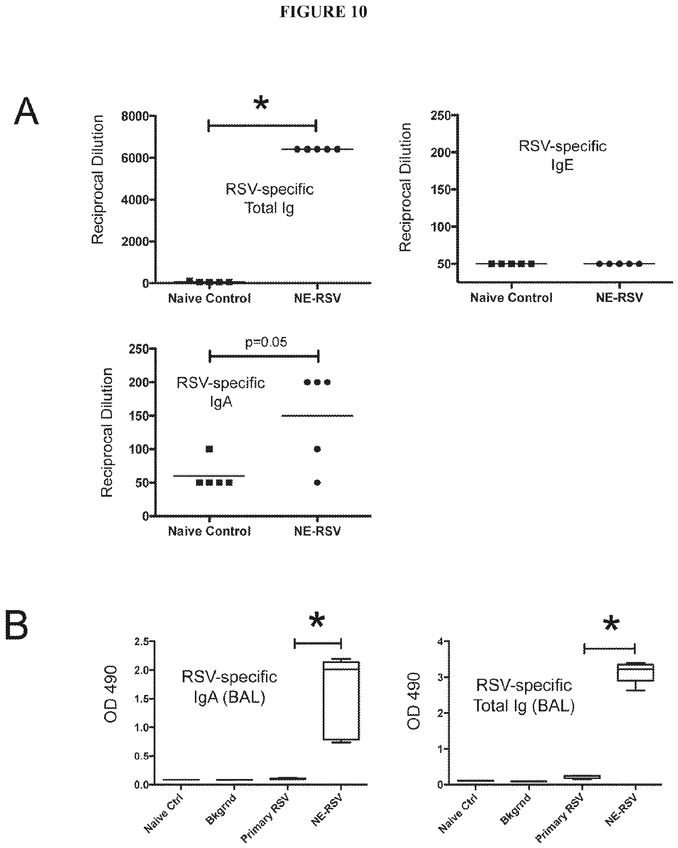

Intranasal vaccination of mice with NE/RSV results in RSV-specific antibody production. Experiments were conducted during development of embodiments of the invention in order to determine whether NE-RSV vaccination would promote antibody responses in subjects administered the NE-RSV composition (e.g., involved in protection against virus infection). An immunization protocol was utilized with vaccinated mice receiving two intranasal doses of NE-RSV, separated by 28 days. Mice were immunized with NE/RSV containing 10⁵ virus particles Line 19 at Day 0 and Day 28. The levels of total RSV specific antibodies in serum were determined at day 55 via ELISA using purified RSV protein extract. As shown in Figure 10, significant RSV-specific responses were generated systemically following vaccination with NE-RSV (See, e.g., Figure 10A). These included dramatic induction of total RSV-specific Ig, with no enhancement in RSV-specific IgE titer (See, e.g., Figure 10A).

Experiments were also conducted during development of emobidments of the invention in order to determine whether vaccination could promote the induction of RSV-specific antibodies locally in the lungs. The presence of RSV-specific total Ig and IgA was assessed by ELISAs of bronchalveolar lavage samples (BAL) at day 2 post-intratracheal challenge with live RSV (10⁵). Specifically, the levels of total RSV specific antibodies in serum were determined at day 55 via ELISA using purified RSV protein extract. Total Ig measured from serum was assessed on samples diluted 1:1600, other samples were assessed at 1:50. (B)

As shown in Figure 10B, compared to control unvaccinated mice (Naive Ctrl) and to mice receiving a primary challenge with NE-RSV (Primary RSV), vaccinated mice receiving two intranasal doses of NE-RSV separated by 28 days (NE-RSV) displayed an increased RSV-specific IgA and RSV-specific total Ig in the bronchoalveolar lavage fluid at day 2 post-challenge with live virus (See, e.g., Figure 10B). These data demonstrate that NE-RSV vaccination induces significant RSV-specific antibodies, and enhances the local induction of RSV-specific antibodies upon live viral challenge.

## Claims

1. An immunogenic composition comprising:
a) a nanoemulsion comprising a solvent, an oil phase, and aqueous phase; in combination with
b) a respiratory syncytial virus (RSV) immunogen;
for use in stimulating an immune response to RSV in a subject.

2. The immunogenic composition for use according to Claim 1, wherein said solvent is ethanol.

3. The immunogenic composition fur use of Claim 1, wherein said nanoemulsion further comprises a cationic halogen containing compound.

4. The immunogenic composition for use according to Claim 3, wherein the cationic halogen containing compound is selected from the group consisting of cetylpyridinium halides, cetyltrimethylammonium halides, cetyldimethyle-thylammonium halides, cetyldimethylbenzylammonium halides, cetyltributylphosphonium halides, dodecyltrimethylammonium halides, and tetradecyltrimethylammonium halides.

5. The immunogenic composition for use according to Claim 1, wherein the RSV immunogen is selected from the group consisting of one or more purified, isolated or synthetic RSV proteins or derivatives, variants, or analogues thereof; or, inactivated RSV from one or more serotypes of RSV.

6. The immunogenic composition for use according to Claim 5, wherein the one or more serotypes of RSV are selected from the group consisting of RSV strain A2, RSV strain B, RSV strain 9320, RSV strain 18537, RSV strain Long, and RSV strain Line 19.

7. The immunogenic composition for use according to Claim 1, wherein said nanoemulsion further comprises a surfactant.

8. The immunogenic composition for use according to claim 7, wherein said surfactant is selected from the group consisting of polyoxyethylene(20)sorbitan monolaurate, polyoxyethylene(20)sorbitan monopalmitate, polyoxyethylene(20)sorbitan monostearate and polyoxyethylene(20)sorbitan monooleate.

9. The immunogenic composition for use according to Claim 1, wherein the nanoemulsion comprises about 5 vol. % TWEEN 80 or TWEEN 20, about 8 vol. % ethanol, about 1 vol. % cetylpyridinium chloride (CPC), about 64 vol. % oil, preferably soybean oil, and about 22 vol. % water.

10. The immunogenic composition for use according to Claim 1, wherein the oil is an animal oil, a vegetable oil, a natural oil, a synthetic oil, a hydrocarbon oil, a silicone oil, or any combination thereof.

11. The immunogenic composition for use according to Claim 10, wherein the oil is soybean oil.

12. A vaccine comprising the immunogenic composition of Claim 1.

13. The vaccine of Claim 12 formulated for administration to a mucosal surface.

14. Use of the immunogenic composition of Claim 1 in the manufacture of a vaccine for treatment or prevention of respiratory syncytial virus infection.

15. An immunogenic composition of Claim 1 for use in the treatment or prevention of respiratory syncytial virus infection.

## Patentansprüche

1. Immunogene Zusammensetzung, umfassend:
a) eine Nanoemulsion, umfassend ein Lösungsmittel, eine Ölphase und eine wässrige Phase; in Kombination mit
b) einem Respiratorischen Synzytial Virus (RSV) Immunogen;
zur Verwendung bei der Stimulierung einer Immunantwort auf RSV in einem Subjekt.

2. Immunogene Zusammensetzung zur Verwendung nach Anspruch 1, wobei besagtes Lösungsmittel Ethanol ist.

3. Immunogene Zusammensetzung zur Verwendung nach Anspruch 1, wobei besagte Nanoemulsion des Weiteren eine kationische halogenhaltige Verbindung umfasst.

4. Immunogene Zusammensetzung zur Verwendung nach Anspruch 3, wobei die kationische halogenhaltige Verbindung ausgewählt ist aus der Gruppe bestehend aus Cetylpyridiniumhalogeniden, Cetyltrimethylammoniumhalogeniden, Cetyldimethylethylammoniumhalogeniden, Cetyldimethylbenzylammoniumhlogeniden, Cetyltributylphosphoniumhalogeniden, Dodecyltrimethylammoniumhalogeniden und Tetradecyltrimethylammoniumhalogeniden.

5. Immunogene Zusammensetzung zur Verwendung nach Anspruch 1, wobei das RSV-Immunogen ausgewählt ist aus der Gruppe bestehend aus einem oder mehreren gereinigten, isolierten oder synthetischen RSV-Proteinen oder Derivaten, Varianten oder Analoga davon, oder inaktivierten RSV von einem oder mehreren RSV-Serotypen.

6. Immunogene Zusammensetzung zur Verwendung nach Anspruch 5, wobei der eine RSV-Serotyp oder die mehreren RSV-Serotypen ausgewählt sind aus der Gruppe bestehend aus dem RSV-Stamm A2, dem RSV-Stamm B, dem RSV-Stamm 9320, dem RSV-Stamm 18537, dem RSV-Stamm Long und dem RSV-Stamm 19.

7. Immunogene Zusammensetzung zur Verwendung nach Anspruch 1, wobei besagte Nanoemulsion des Weiteren ein Tensid umfasst.

8. Immunogene Zusammensetzung zur Verwendung nach Anspruch 7, wobei besagtes Tensid ausgewählt ist aus der Gruppe bestehend aus Polyoxyethylen(20)sorbitanmonolaurat, Polyoxyethylen(20)sorbitanmonopalmitat, Polyoxyethylen(20)sorbitanmo-nostearat und Polyoxyethylen(20)sorbitanmonooleat.

9. Immunogene Zusammensetzung zur Verwendung nach Anspruch 1, wobei die Nanoemulsion etwa 5 Vol.-% TWEEN 80 oder TWEEN 20, etwa 8 Vol.-% Ethanol, etwa 1 Vol.-% Cetylpyridiniumchlorid (CPC), etwa 64 Vol.-% Öl, vorzugsweise Sojabohnenöl, und etwa 22 Vol.-% Wasser umfasst.

10. Immunogene Zusammensetzung zur Verwendung nach Anspruch 1, wobei das Öl ein tierisches Öl, ein Pflanzenöl, ein natürliches Öl, ein synthetisches Öl, ein Kohlenwasserstofföl, ein Silikonöl oder eine Kombination daraus ist.

11. Immunogene Zusammensetzung zur Verwendung nach Anspruch 10, wobei das Öl Sojabohnenöl ist.

12. Vakzin umfassend die immunogene Zusammensetzung nach Anspruch 1.

13. Vakzin nach Anspruch 12 formuliert für die Anwendung auf einer Schleimhautoberfläche.

14. Verwendung der immunogenen Zusammensetzung nach Anspruch 1 bei der Herstellung eines Vakzins zur Behandlung oder zur Vorbeugung gegen eine Infektion mit dem Respiratorischen Synzytial Virus.

15. Immunogene Zusammensetzung nach Anspruch 1 zur Verwendung bei der Behandlung oder zur Vorbeugung gegen eine Infektion mit dem Respiratorischen Synzytial Virus.

## Revendications

1. Composition immunogène comprenant :
a) une nano-émulsion comprenant un solvant, une phase huileuse et une phase aqueuse ; en combinaison avec
b) un agent immunogène de virus respiratoire syncytial (VRS) ;
pour utilisation dans la stimulation d'une réponse immunitaire contre VRS chez un sujet.

2. Composition immunogène pour utilisation selon la revendication 1, dans laquelle ledit solvant est l'éthanol.

3. Composition immunogène pour utilisation de la revendication 1, dans laquelle ladite nano-émulsion comprend en outre un composé cationique contenant un halogène.

4. Composition immunogène pour utilisation selon la revendication 3, dans laquelle le composé cationique contenant un halogène est choisi dans le groupe constitué d'halogénures de cétylpyridinium, halogénures de cétyltriméthylammonium, halogénures de cétyldiméthyléthylammonium, halogénures de cétyldiméthylbenzylammonium, halogénures de cétyltributylphosphonium, halogénures de dodécyltriméthylammonium et halogénures de tétradécyltriméthylammonium.

5. Composition immunogène pour utilisation selon la revendication 1, dans laquelle l'agent immunogène de VRS est choisi dans le groupe constitué d'une ou plusieurs protéines de VRS purifiées, isolées ou synthétiques ou des dérivés, des variants ou des analogues de celles-ci ; ou, VRS inactivé d'un ou plusieurs sérotypes de VRS.

6. Composition immunogène pour utilisation selon la revendication 5, dans laquelle les un ou plusieurs sérotypes de VRS sont choisis dans le groupe constitué de la souche de VRS A2, la souche de VRS B, la souche de VRS 9320, la souche de VRS 18537, la souche de VRS Long, et la souche de VRS Line 19.

7. Composition immunogène pour utilisation selon la revendication 1, dans laquelle ladite nano-émulsion comprend en outre un tensioactif.

8. Composition immunogène pour utilisation selon la revendication 7, dans laquelle ledit tensioactif est choisi dans le groupe constitué des monolaurate de polyoxyéthylène(20)sorbitane, monopalmitate de polyoxyéthylène(20)sorbitane, monostéarate de polyoxyéthylène(20)sorbitane et monooléate de polyoxyéthylène(20)sorbitane.

9. Composition immunogène pour utilisation selon la revendication 1, dans laquelle la nano-émulsion comprend environ 5 % en volume de TWEEN 80 ou TWEEN 20, environ 8 % en volume d'éthanol, environ 1 % en volume de chlorure de cétylpyridinium (CPC), environ 64 % en volume d'huile, de préférence l'huile de soja, et environ 22 % en volume d'eau.

10. Composition immunogène pour utilisation selon la revendication 1, dans laquelle l'huile est une huile animale, une huile végétale, une huile naturelle, une huile synthétique, une huile d'hydrocarbure, une huile silicone, ou une combinaison quelconque de celles-ci.

11. Composition immunogène pour utilisation selon la revendication 10, dans laquelle l'huile est l'huile de soja.

12. Vaccin comprenant la composition immunogène de la revendication 1.

13. Vaccin de la revendication 12 formulé pour administration à une surface muqueuse.

14. Utilisation de la composition immunogène de la revendication 1 dans la fabrication d'un vaccin pour le traitement ou la prévention d'une infection par le virus respiratoire syncytial.

15. Composition immunogène de la revendication 1 pour utilisation dans le traitement ou la prévention d'une infection par le virus respiratoire syncytial.
